(19) Europäisches Patentamt
European Patent Office
Office européen des brevets

(11) EP 4 534 537 A1

(12) **EUROPEAN PATENT APPLICATION**
published in accordance with Art. 153(4) EPC

(43) Date of publication:
**09.04.2025 Bulletin 2025/15**

(21) Application number: **23811122.3**

(22) Date of filing: **25.05.2023**

(51) International Patent Classification (IPC):
*C07D 471/04* (2006.01) *C07D 401/12* (2006.01)
*C07D 401/14* (2006.01) *C07D 519/00* (2006.01)
*A61K 31/498* (2006.01) *A61K 31/4709* (2006.01)
*A61P 35/00* (2006.01) *A61K 31/496* (2006.01)

(52) Cooperative Patent Classification (CPC):
**A61K 31/4709; A61K 31/496; A61K 31/498; A61P 35/00; C07D 401/12; C07D 401/14; C07D 471/04; C07D 519/00**

(86) International application number:
**PCT/CN2023/096215**

(87) International publication number:
**WO 2023/227052 (30.11.2023 Gazette 2023/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:
**25.05.2022 CN 202210577843**
**01.06.2022 CN 202210621147**
**11.07.2022 CN 202210811952**
**05.09.2022 CN 202211080233**
**03.11.2022 CN 202211374066**
**16.03.2023 CN 202310252343**

(71) Applicant: **Xizang Haisco Pharmaceutical Co., Ltd.**
**Lhoka, Tibet 856099 (CN)**

(72) Inventors:
- **LI, Yao**
  **Lhoka, Tibet 856099 (CN)**
- **CHEN, Lei**
  **Lhoka, Tibet 856099 (CN)**
- **ZHANG, Haoliang**
  **Lhoka, Tibet 856099 (CN)**
- **KANG, Peng**
  **Lhoka, Tibet 856099 (CN)**
- **FU, Chao**
  **Lhoka, Tibet 856099 (CN)**
- **GUI, Naicheng**
  **Lhoka, Tibet 856099 (CN)**
- **TANG, Pingming**
  **Lhoka, Tibet 856099 (CN)**
- **ZHANG, Chen**
  **Lhoka, Tibet 856099 (CN)**
- **YAN, Pangke**
  **Lhoka, Tibet 856099 (CN)**

(74) Representative: **IP TRUST SERVICES**
**Europole - Le Grenat**
**3, avenue Doyen Louis Weil**
**38000 Grenoble (FR)**

(54) **BICYCLIC DERIVATIVE PARP INHIBITOR AND USE THEREOF**

(57) Disclosed herein are a compound represented by formula (II) or a stereoisomer, a pharmaceutically acceptable salt, a solvate, a co-crystal or a deuterated form thereof, or a pharmaceutical composition comprising same, and use thereof as a PARP-1 inhibitor in preparing a medicament for treating a related disease. Groups in formula (II) are defined in the description.

EP 4 534 537 A1

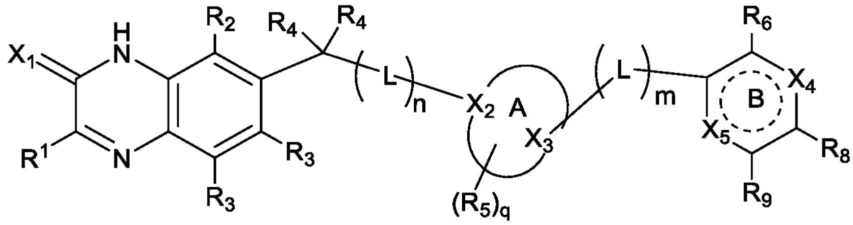

(II)

## Description

### Technical Field

The present invention belongs to the field of drugs, and in particular relates to a small molecule compound having a PARP-1 inhibitory activity, or a stereoisomer, pharmaceutically acceptable salt, solvate, co-crystal or deuterated form thereof, and the use thereof in the treatment of a related disease.

### Background Art

Approximately 5% of breast cancer patients are associated with germline mutations in the BRCA1/2 genes (3% in the BRCA1 gene and 2% in the BRCA2 gene). Most breast cancers caused by BRCA1 mutations are triple-negative breast cancers (70%), while BRCA2 mutations are more likely to cause oestrogen receptor-positive breast cancers (70%). The BRCA1/2 genes are tumour suppressor genes and play an important role in DNA damage repair, normal cell growth, etc. Mutations in the genes can inhibit the normal repair ability after DNA damage and cause homologous recombination deficiency (HRD), that is, loss of BRCA function or mutation or loss of function in other homologous recombination-related genes, making repair of DNA double-strand breaks impossible through homologous recombination repair (HRR), eventually leading to cancer.

Poly(ADP-ribose) polymerase (PARP) is a DNA repair enzyme that plays a key role in the DNA repair pathway. PARP is activated by DNA damage and breakage. As a molecular sensor of DNA damage, it has the function of identifying and binding to the DNA break location, thereby activating and catalysing the polyADP ribosylation of the receptor protein and participating in the DNA repair process. PARP plays a key role in the process of DNA single-strand base excision and repair. In HRD tumour cells, DNA double-strand breaks cannot be repaired, and PARP inhibitors block the single-strand repair, resulting in a "synthetic lethal" effect and leading to tumour cell death.

PARP inhibitors have a "trapping" effect on the PARP protein, causing the PARP protein that binds to damaged DNA to be trapped on the DNA, directly causing other DNA repair proteins to be unable to bind, eventually leading to cell death. At present, several PARP inhibitors have been successfully developed, such as olaparib, rucaparib and niraparib. However, adverse reactions limit their ability to be used in combination with chemotherapy drugs. This may be related to the lack of selectivity of marketed PARP inhibitors against the PARP family. The side effects include intestinal toxicity caused by tankyrase inhibition and haematological toxicity caused by PARP-2 inhibition. Therefore, it is of great clinical significance to develop highly selective PARP-1 inhibitors and reduce the toxic and side effects associated with non-selective PARP inhibitors.

### Summary of the Invention

The objective of the present invention is to provide a compound that inhibits PARP-1, or a stereoisomer, solvate, deuterated form or pharmaceutically acceptable salt thereof, and the medical application thereof. The compound of the present invention has the advantages of good activity, low toxic and side effects, high safety, strong selectivity, good pharmacokinetics and high bioavailability.

The present invention provides a compound represented by formula (I), (III), (IV) or (V) as shown below, a stereoisomer, solvate, deuterated form or pharmaceutically acceptable salt thereof:

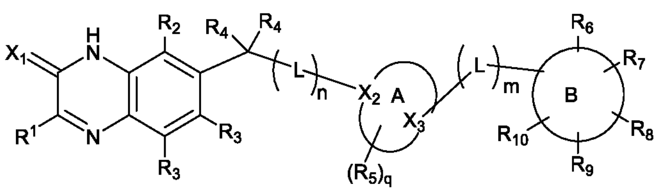

(I)

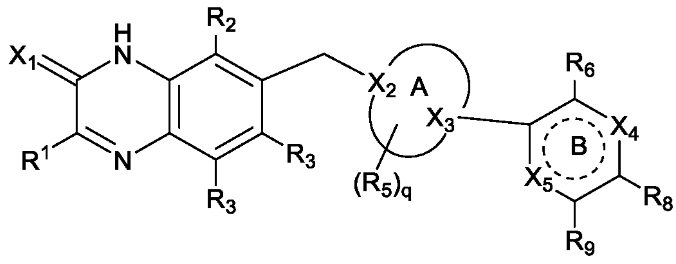
(III)

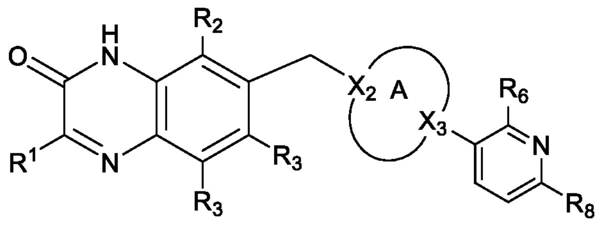
(IV)

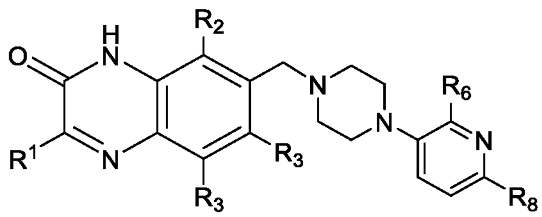
(V)

wherein $X_1$ is O, S or N-R, R is selected from CN and $C_{1-4}$ alkyl; in some embodiments, $X_1$ is O or S;

$X_2$ is N, C or $CR_{5'}$; in some embodiments, $X_2$ is N or $CR_{5'}$;

$X_3$ is N, C or $CR_{5''}$;

ring A is 4- to 12-membered heterocycloalkyl containing 1-3 N atoms and 0-2 heteroatoms selected from O and S; in some embodiments, ring A is unsubstituted piperazinyl; in some embodiments, ring A is optionally substituted 4- to 12-membered heterocycloalkyl containing 1-3 N atoms and 0-2 heteroatoms selected from O and S, except unsubstituted piperazinyl; in some embodiments, ring A is 4-to 9-membered monocyclic heterocycloalkyl, 6- to 12-membered spiroheterocycloalkyl, 6- to 12-membered fused heterocycloalkyl or 6- to 12-membered bridged heterocycloalkyl containing 1-3 N atoms and 0-2 heteroatoms selected from O and S; in some embodiments, the 4- to 9-membered monocyclic heterocycloalkyl does not comprise unsubstituted piperazinyl; in some embodiments, ring A is substituted or unsubstituted 6- to 10-membered fused heterocycloalkyl, substituted or unsubstituted 6- to 11-membered spiroheterocycloalkyl, substituted or unsubstituted 6- to 10-membered bridged heterocycloalkyl, substituted or unsubstituted 4- to 5-membered monocyclic heterocycloalkyl, substituted or unsubstituted 7-membered monocyclic heterocycloalkyl, substituted or unsubstituted

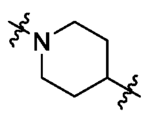
,

or substituted or unsubstituted

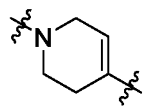
;

in some embodiments, ring A is selected from

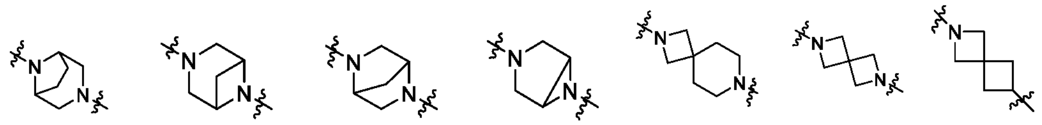

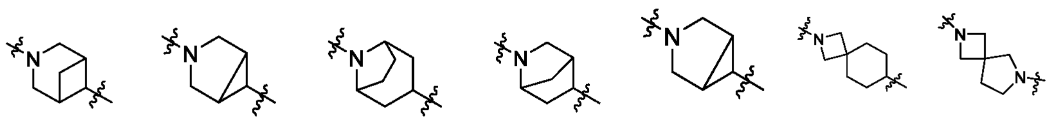

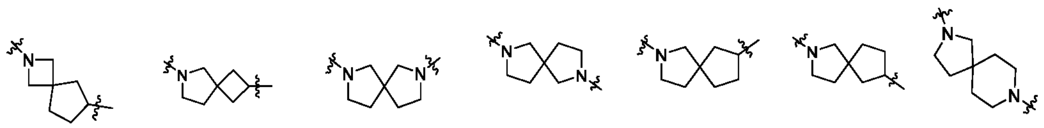

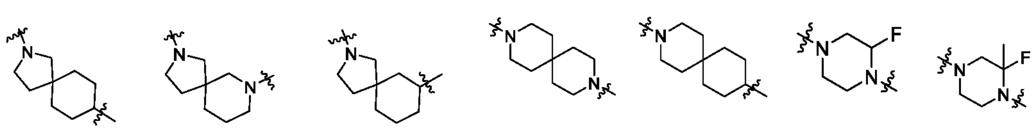

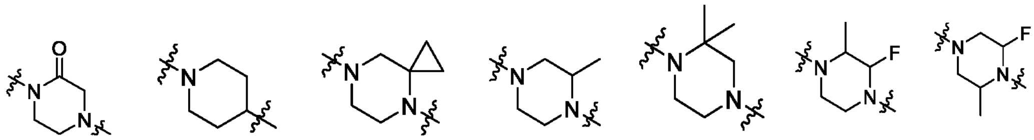

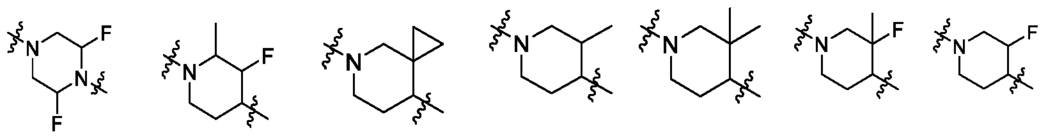

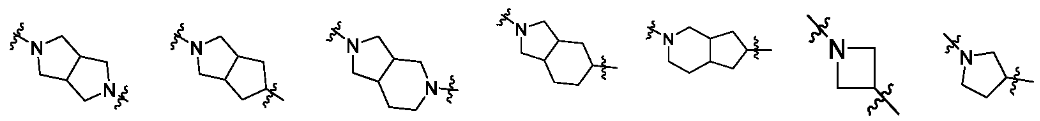

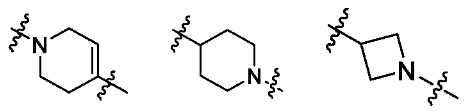

;

$R_1$, $R_2$ and $R_3$ are independently H, D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, $-O-C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, and the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;

in some embodiments, $R_1$ is H, D, halogen, CN, $C_{1-4}$ alkyl, $C_{2-4}$ alkenyl, $C_{2-4}$ alkynyl, $C_{1-4}$ alkoxy, $C_{3-6}$ cycloalkyl, $-O-C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, wherein the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, $C_{3-6}$ cycloalkyl and $C_{1-4}$ haloalkyl; in some embodiments, $R_1$ is halogen, CN, $C_{2-4}$ alkenyl, $C_{2-4}$ alkynyl, $C_{1-4}$ alkoxy, $C_{1-4}$ alkyl and $C_{3-6}$ cycloalkyl, wherein the alkyl is further substituted with D, halogen, $C_{1-4}$ alkoxy, $C_{3-6}$ cycloalkyl and $C_{1-4}$ haloalkyl, and the alkoxy, haloalkyl and cycloalkyl are optionally substituted with the aforementioned groups; in some embodiments, $R_1$ is $C_{1-4}$ alkyl, $C_{1-4}$ haloalkyl and $C_{3-6}$ cycloalkyl, and is optionally substituted with 1-5 groups selected from D, halogen, $C_{1-4}$ alkyl, $C_{1-4}$ alkoxy, OH, CN, $NH_2$ and $C_{1-4}$ haloalkyl; in some embodiments, $R_1$ is $C_{1-2}$ alkyl, wherein the alkyl is optionally substituted with 1-3 groups selected from D, F and Cl;

in some embodiments, $R_2$ is H, D, halogen or $C_{1-4}$ alkyl, wherein the alkyl is optionally substituted with 1-5 groups selected from D, halogen, OH, CN and $NH_2$; in some embodiments, $R_2$ is H, D, F, Cl, Br, I or $C_{1-4}$ alkyl, wherein the alkyl is optionally substituted with 1-5 groups selected from D, halogen, OH, CN and $NH_2$; in some embodiments, $R_2$ is H, D, Cl, Br, I, or $C_{1-4}$ alkyl, wherein the alkyl is optionally substituted with 1-5 groups selected from D, halogen, OH, CN and $NH_2$; in some embodiments, $R_2$ is F, Cl or $C_{1-2}$ alkyl, wherein the alkyl is optionally further substituted with 1-3 groups selected from D, F and Cl;

$R_3$ is independently H, D, halogen or $C_{1-4}$ alkyl, wherein the alkyl is optionally substituted with 1-5 groups selected from D, halogen, OH, CN and $NH_2$; in some embodiments, $R_3$ is H, D, F, Cl, Br or I; in some embodiments, at least one $R_3$ is D, halogen or $C_{1-4}$ alkyl, wherein the alkyl is optionally substituted with 1-5 groups selected from D, halogen, OH, CN and $NH_2$; in some embodiments, $R_3$ is H;

each $R_4$ is independently selected from H, D, halogen, $C_{1-4}$ alkyl, $C_{3-6}$ cycloalkyl, OH, CN, $NH_2$, $C_{1-4}$ alkoxy, $C_{2-6}$ alkenyl or $C_{2-6}$ alkynyl, wherein the alkyl, alkoxy, cycloalkyl, alkenyl and alkynyl are optionally substituted with 1-5 groups selected from D, halogen, $C_{1-4}$ alkyl, $C_{1-4}$ alkoxy, OH, CN, $NH_2$ and $C_{1-4}$ haloalkyl; in some embodiments, at least one $R_4$ is selected from D, halogen, $C_{1-4}$ alkyl, $C_{3-6}$ cycloalkyl, OH, CN, $NH_2$, $C_{1-4}$ alkoxy, $C_{2-6}$ alkenyl or $C_{2-6}$ alkynyl, wherein the alkyl, alkoxy, cycloalkyl, alkenyl and alkynyl are optionally substituted with 1-5 groups selected from D, halogen, $C_{1-4}$ alkyl, $C_{1-4}$ alkoxy, OH, CN, $NH_2$ and $C_{1-4}$ haloalkyl; in some embodiments, each $R_4$ is independently selected from H, D, halogen, $C_{1-4}$ alkyl, $C_{3-6}$ cycloalkyl, OH, CN or $NH_2$, wherein the alkyl and cycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl; in some embodiments, at least one $R_4$ is selected from D, halogen, $C_{1-4}$ alkyl, $C_{3-6}$ cycloalkyl, OH, CN or $NH_2$, wherein the alkyl and cycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl; in some embodiments, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, two $R_4$ are not both H, or two $R_4$ together with the carbon atom to which they are attached form substituted or unsubstituted $C_{3-6}$ cycloalkyl or substituted or unsubstituted 4- to 7-membered heterocycloalkyl containing 1-3 heteroatoms selected from N, S and O, wherein substituents are as described above;

each L is independently -NH-, $-CH_2-$, -O-, -S-, -S(=O)-, $-S(=O)_2-$ or -C(=O)-; in some embodiments, each L is independently -NH-, $-CH_2-$, -O- or -C(=O)-; in some embodiments, at least one L is present, i.e., n and m are not both 0;

n and m are independently 0, 1, 2 or 3; in some embodiments, n and m are independently 0, 1 or 2; in some embodiments, n and m are independently 0 or 1; in some embodiments, n and m are not both 0; in some embodiments, n and m are both 0;

$R_5$, $R_{5'}$ and $R_{5''}$ are each independently H, D, halogen, =O, $C_{1-4}$ alkyl, $C_{1-4}$ alkoxy, CN or $C_{3-6}$ cycloalkyl, wherein the alkyl, alkoxy and cycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_5$, $R_{5'}$ and $R_{5''}$ are each independently H, D, halogen, $C_{1-4}$ alkyl or $C_{1-4}$ alkoxy, wherein the alkyl and alkoxy are optionally substituted with 1-3 groups selected from D, halogen, OH, CN and $NH_2$;

in some embodiments, $R_5$ is H, halogen, $C_{1-4}$ alkyl or =O, wherein the alkyl is optionally substituted with 1-3 groups selected from D, halogen, OH, CN and $NH_2$;

in some embodiments, $R_{5'}$ and $R_{5''}$ are independently H, halogen or $C_{1-4}$ alkyl, wherein the alkyl is optionally substituted with 1-3 groups selected from D, halogen, OH, CN and $NH_2$;

in some embodiments, $R_5$, $R_{5'}$ and $R_{5''}$ are not all H;

in some embodiments, one $R_4$ forms a bond with $R_{5'}$, so that when n is 0, a double bond is formed between the C connected to $R_4$ and ring A, and when n is not 0, a spiro ring is formed;

q is 0, 1, 2, 3 or 4, and in some embodiments, q is 0, 1, 2 or 3; in some embodiments, q is 0, 1 or 2; in some embodiments, q is 0 or 1; in some embodiments, q is 0;

ring B is phenyl, pyridyl, pyrazinyl, pyrimidyl or pyridazinyl; in some embodiments, ring B is not

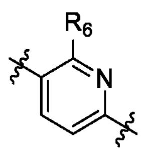
;

$R_6$, $R_7$, $R_9$ and $R_{10}$ are independently H, D, CN, OH, halogen, $C_{1-4}$ alkyl, $C_{1-4}$ alkoxy, $C_{3-6}$ cycloalkyl or 4- to 7-membered heterocycloalkyl containing 1-3 heteroatoms selected from N, S and O, wherein the alkyl, alkoxy, cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_6$, $R_7$, $R_9$ and $R_{10}$ are independently H, D, CN,

OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_6$, $R_7$, $R_9$ and $R_{10}$ are independently H, D, CN, OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_6$ is D, F, Cl and $C_{1-2}$ alkyl, wherein the alkyl is optionally substituted with 1-3 groups selected from D, F and Cl;

$R_8$ is $C_{1-4}$ alkyl, $C_{1-4}$ alkoxy, $-NHC_{1-4}$ alkyl, $-CONHC_{1-4}$ alkyl, $-CONHC_{1-4}$ alkyleneO$C_{1-4}$ alkyl, $-NHCOC_{1-4}$ alkyl, $-NHCOOC_{1-4}$ alkyl, $-OCONHC_{1-4}$ alkyl, $C_{2-6}$ alkenyl, $C_{2-6}$ alkynyl, $R_{11}$, $-NHR_{11}$, $-(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, $-O(CH_2)_rR_{11}$, $-CONHR_{11}$, $-NHCOR_{11}$, $-NHCONHR_{11}$, $-CONH(CH_2)_rR_{11}$, $-CONH(CH_2)_rOR_{11}$, $-(CH_2)_rCONHR_{11}$, $-OCONHR_{11}$, $-COOR_{11}$, or $-CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_8$ is $C_{1-4}$ alkyl, $C_{1-4}$ alkoxy, $-NHC_{1-4}$ alkyl, $-CONHC_{1-4}$ alkyleneO$C_{1-4}$ alkyl, $-NHCOC_{1-4}$ alkyl, - $NHCOOC_{1-4}$ alkyl, $-OCONHC_{1-4}$ alkyl, $C_{2-6}$ alkenyl, $C_{2-6}$ alkynyl, $R_{11}$, $-NHR_{11}$, - $(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, - $O(CH_2)_rR_{11}$, $-CONHR_{11}$, $-NHCOR_{11}$, $-NHCONHR_{11}$, $-CONH(CH_2)_rR_{11}$, - $CONH(CH_2)_rOR_{11}$, $-(CH_2)_rCONHR_{11}$, $-COOR_{11}$, $-OCONHR_{11}$, or - $CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_8$ is $C_{1-4}$ alkyl, $C_{1-4}$ alkoxy, $-NHC_{1-4}$ alkyl, $-NHCOC_{1-4}$ alkyl, $-NHCOOC_{1-4}$ alkyl, $-OCONHC_{1-4}$ alkyl, $-CONHC_{1-4}$ alkyleneO$C_{1-4}$ alkyl, $C_{2-6}$ alkenyl, $C_{2-6}$ alkynyl, $R_{11}$, $-NHR_{11}$, - $(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, - $O(CH_2)_rR_{11}$, $-CONHR_{11}$, $-NHCOR_{11}$, $-NHCONHR_{11}$, $-CONH(CH_2)_rR_{11}$, - $CONH(CH_2)_rOR_{11}$, $-(CH_2)_rCONHR_{11}$, $-COOR_{11}$, $-OCONHR_{11}$, or - $CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl; in some embodiments, $R_8$ is $-CONHR_{11}$ or $-NHCOR_{11}$;

each $R_{11}$ is independently $C_{3-6}$ cycloalkyl, 4- to 7-membered heterocycloalkyl containing 1-3 heteroatoms selected from N, S and O, 5- to 6-membered heteroaryl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, $-NHC_{1-4}$ alkyl, - $CONHC_{1-4}$ alkyl, $-NHCOC_{1-4}$ alkyl, OH, CN, $NH_2$ and $C_{1-4}$ haloalkyl; in some embodiments, $R_{11}$ is cyclopropyl, cyclobutyl, cyclopentyl, cyclohexyl, azacyclopropyl, azetidinyl, tetrahydropyrrolyl, piperidyl, piperazinyl, tetrahydrofuryl, tetrahydropyranyl, morpholinyl, thiazolyl, thienyl, thiadiazolyl, pyrazolyl, imidazolyl, pyrrolyl, pyranyl, furyl, oxadiazolyl, pyridyl, pyrazinyl, pyridazinyl, pyrimidyl, etc.; in some embodiments, $R_{11}$ is $C_{3-6}$ cycloalkyl or 5- to 6-membered heteroaryl containing 1-3 heteroatoms selected from N, S and O, wherein the cycloalkyl and heteroaryl are optionally substituted with 1-3 groups selected from D, F, Cl, $C_{1-2}$ alkyl and $C_{1-2}$ haloalkyl; in some embodiments, $R_{11}$ is cyclopropyl or pyrazolyl, wherein the cyclopropyl and pyrazolyl are optionally substituted with 1-3 groups selected from D, F, Cl, $C_{1-2}$ alkyl and $C_{1-2}$ haloalkyl; in some embodiments, $R_{11}$ is cyclopropyl or pyrazolyl, wherein the cyclopropyl and pyrazolyl are optionally substituted with 1-3 groups selected from $C_{1-2}$ alkyl; each r is independently 1, 2 or 3; in some embodiments, r is 1 or 2; in some embodiments, r is 1;

in some embodiments,

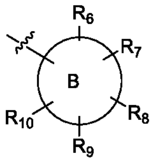 is 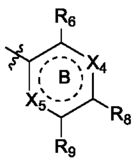,

that is, the compound as described previously has a structure of formula (II):

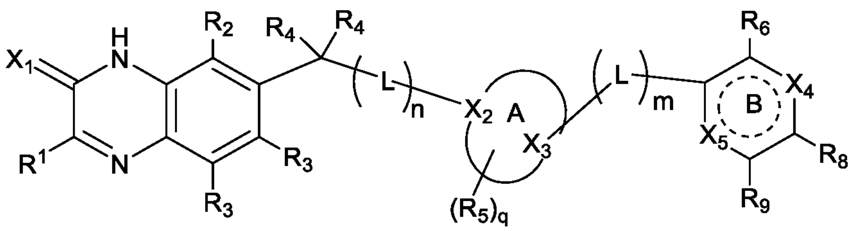

(II)

ring B is six-membered heteroaryl, and the remaining groups are as described above.

In some embodiments, provided is the compound, or the stereoisomer, solvate, deuterated form or pharmaceutically acceptable salt thereof of the present invention, wherein the compound at least satisfies one of the following conditions:

(1) $R_1$ is D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, -O-$C_{3-6}$ cycloalkyl, $-(CH_2)_r$-$C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, the alkyl is substituted with 1-5 groups selected from D, Cl, Br, I, $C_{1-4}$ alkoxy, OH, CN and $NH_2$, the alkoxy or cycloalkyl is substituted with 1-5 groups selected from D, halogen, $C_{1-4}$ alkyl, $C_{1-4}$ alkoxy, OH, CN, $NH_2$ and $C_{1-4}$ haloalkyl, the alkenyl, alkynyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
(2) $R_2$ is H, D, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, -O-$C_{3-6}$ cycloalkyl, $-(CH_2)_r$-$C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, the alkyl is substituted with 1-5 groups selected from D, halogen, $C_{1-4}$ alkoxy, OH, CN, $NH_2$ and $C_{1-4}$ haloalkyl, the alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
(3) at least one $R_3$ is D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, -O-$C_{3-6}$ cycloalkyl, $-(CH_2)_r$-$C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
(4) two $R_4$ are not both H, or two $R_4$ together with the carbon atom to which they are attached form substituted or unsubstituted $C_{3-6}$ cycloalkyl or substituted or unsubstituted 4- to 7-membered heterocycloalkyl containing 1-3 heteroatoms selected from N, S and O;
(5) m and n are not both 0;
(6)

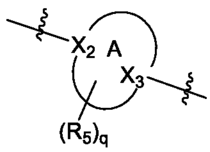

is not

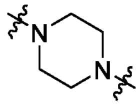

;

(7) ring B is not

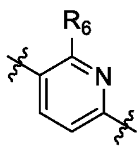

,

$R_6$ is halogen or $C_{1-4}$ alkyl;
(8) $R_8$ is $C_{1-4}$ alkyl, $C_{1-4}$ alkoxy, -NH$C_{1-4}$ alkyl, -NHCO$C_{1-4}$ alkyl, - CONH$C_{1-4}$ alkyleneO$C_{1-4}$ alkyl, -NHCOO$C_{1-4}$ alkyl, -OCONH$C_{1-4}$ alkyl, $C_{2-6}$ alkenyl, $C_{2-6}$ alkynyl, $R_{11}$, $-NHR_{11}$, $-(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, $-O(CH_2)_rR_{11}$, $-CONHR_{11}$, $-NHCOR_{11}$, $-NHCONHR_{11}$, $-CONH(CH_2)_rR_{11}$, $CONH(CH_2)_rOR_{11}$, $-(CH_2)_rCONHR_{11}$, $-COOR_{11}$, $-OCONHR_{11}$, or $-CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl.

In some embodiments, provided is the compound, or the stereoisomer, solvate, deuterated form or pharmaceutically acceptable salt thereof as described previously in the present invention, wherein the compound at least satisfies one of the above conditions (1) to (5).

In some embodiments, provided is the compound, or the stereoisomer, solvate, deuterated form or pharmaceutically acceptable salt thereof as described previously in the present invention, wherein the compound at least satisfies one of the above conditions (6) to (8).

In some embodiments, provided is the compound, or the stereoisomer, solvate, deuterated form or pharmaceutically acceptable salt thereof as described previously in the present invention, wherein the compound at least satisfies one of the above conditions (4) to (8).

In some embodiments, provided is the compound, or the stereoisomer, solvate, deuterated form or pharmaceutically acceptable salt thereof as described previously in the present invention, wherein the compound has a structure of formula (III) as shown below:

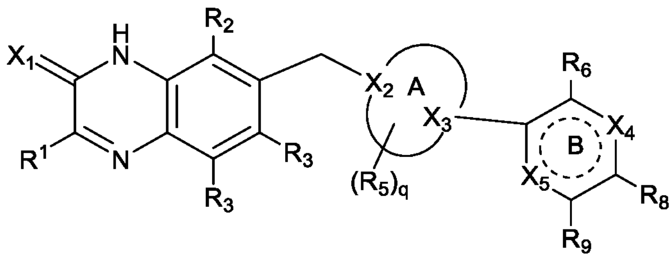

(III)

wherein each group is as described in the previous embodiments and at least satisfies one of the above conditions (1) to (3) and (6) to (8).

In some embodiments, provided is the compound represented by formula (III) as described previously, wherein each group is as described in the previous embodiments and at least satisfies one of the above conditions (6) to (8).

The present invention provides a specific solution 1, i.e., a compound represented by formula (I), (II), or (III), or a stereoisomer, solvate, deuterated form or pharmaceutically acceptable salt thereof,

wherein $X_1$ is O or S;
$X_2$ is N, C or $CR_{5'}$;
$X_3$ is N, C or $CR_{5''}$;
ring A is 4- to 12-membered heterocycloalkyl containing 1-3 N atoms and 0-2 heteroatoms selected from O and S;
$R_1$, $R_2$ and $R_3$ are independently H, D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, $-O-C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, and the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
each $R_4$ is independently selected from H, D, halogen, $C_{1-4}$ alkyl, $C_{3-6}$ cycloalkyl, OH, CN, $NH_2$, $C_{1-4}$ alkoxy, $C_{2-6}$ alkenyl or $C_{2-6}$ alkynyl, wherein the alkyl, alkoxy, cycloalkyl, alkenyl and alkynyl are optionally substituted with 1-5 groups selected from D, halogen, $C_{1-4}$ alkyl, $C_{1-4}$ alkoxy, OH, CN, $NH_2$ and $C_{1-4}$ haloalkyl;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each L is independently -NH-, $-CH_2-$, -O-, -S-, -S(=O)-, $-S(=O)_2-$ or -C(=O)-;
n and m are independently 0, 1, 2 or 3;
$R_5$, $R_{5'}$ and $R_{5''}$ are each independently H, D, =O, halogen, $C_{1-4}$ alkyl, $C_{1-4}$ alkoxy, CN or $C_{3-6}$ cycloalkyl, wherein the alkyl, alkoxy and cycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
optionally, one $R_4$ forms a bond with $R_{5'}$;
q is 0, 1, 2, 3 or 4;
$X_4$ is N, $NR_7$ or $CR_7$ (formula II or III);
$X_5$ is N, $NR_{10}$ or $CR_{10}$ (formula II or III);
ring B is six-membered heteroaryl (ring B in formula I can also be phenyl);
$R_6$, $R_7$, $R_9$ and $R_{10}$ are independently H, D, CN, OH, halogen, $C_{1-4}$ alkyl, $C_{1-4}$ alkoxy, $C_{3-6}$ cycloalkyl or 4- to 7-membered heterocycloalkyl containing 1-3 heteroatoms selected from N, S and O, wherein the alkyl, alkoxy, cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;

$R_8$ is $C_{1-4}$ alkyl, $C_{1-4}$ alkoxy, -$NHC_{1-4}$ alkyl, -$CONHC_{1-4}$ alkyl, -$CONHC_{1-4}$ alkyleneO$C_{1-4}$ alkyl, -$NHCOC_{1-4}$ alkyl, -$NHCOOC_{1-4}$ alkyl, -$OCONHC_{1-4}$ alkyl, $C_{2-6}$ alkenyl, $C_{2-6}$ alkynyl, $R_{11}$, -$NHR_{11}$, -$(CH_2)_rR_{11}$, -$OR_{11}$, -$COR_{11}$, -$(CH_2)_rNHR_{11}$, -$NH(CH_2)_rR_{11}$, -$(CH_2)_rOR_{11}$, -$O(CH_2)_rR_{11}$, -$CONHR_{11}$, -$NHCOR_{11}$, -$NHCONHR_{11}$, -$CONH(CH_2)_rR_{11}$, -$CONH(CH_2)_rOR_{11}$, -$(CH_2)_rCONHR_{11}$, -$OCONHR_{11}$, -$COOR_{11}$, or -$CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl;

each $R_{11}$ is independently $C_{3-6}$ cycloalkyl, 4- to 7-membered heterocycloalkyl containing 1-3 heteroatoms selected from N, S and O, 5- to 6-membered heteroaryl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, -$NHC_{1-4}$ alkyl, - $CONHC_{1-4}$ alkyl, -$NHCOC_{1-4}$ alkyl, OH, CN, $NH_2$ and $C_{1-4}$ haloalkyl;

each r is independently 1, 2 or 3.

In solution 2 provided in the present invention, the compound represented by formula (I), (II) or (III) also satisfies that when

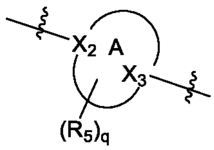

is

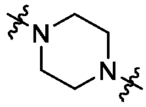,

n and m are 0,

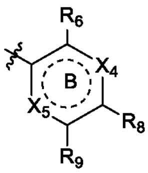

is

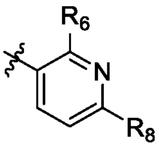,

and $R_6$ is H, halogen, $C_{1-4}$ alkyl or halo$C_{1-4}$ alkyl, $R_8$ is not -$CONHC_{1-4}$ alkyl; the rest is as described in solution 1.

In solution 3 provided in the present invention, the compound represented by formula (I) or formula (II) at least satisfies one of the above conditions (1) to (8), and the compound represented by formula (III) at least satisfies one of the above conditions (1) to (3) and (6) to (8); and the remaining groups are as described in solution 1.

In solution 4 provided in the present invention, the compound represented by formula (III) at least satisfies one of the above conditions (6) to (8); and the remaining groups are as described in solution 1.

The present invention provides solution 5, i.e., a compound represented by formula (II), or a stereoisomer, solvate, deuterated form or pharmaceutically acceptable salt thereof, wherein $X_1$ is O or S;

$X_2$ is N, C or $CR_{5'}$;

$X_3$ is N, C or $CR_{5''}$;
ring A is 4- to 12-membered heterocycloalkyl containing 1-3 N atoms and 0-2 heteroatoms selected from O and S;
$R_1$, $R_2$ and $R_3$ are independently H, D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, $-O-C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, and the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
each $R_4$ is independently selected from H, D, halogen, $C_{1-4}$ alkyl, $C_{3-6}$ cycloalkyl, OH, CN, $NH_2$, $C_{1-4}$ alkoxy, $C_{2-6}$ alkenyl or $C_{2-6}$ alkynyl, wherein the alkyl, alkoxy, cycloalkyl, alkenyl and alkynyl are optionally substituted with 1-5 groups selected from D, halogen, $C_{1-4}$ alkyl, $C_{1-4}$ alkoxy, OH, CN, $NH_2$ and $C_{1-4}$ haloalkyl;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each L is independently -NH-, $-CH_2-$, -O-, -S-, -S(=O)-, $-S(=O)_2-$ or -C(=O)-;
n and m are independently 0, 1, 2 or 3;
$R_5$, $R_{5'}$ and $R_{5''}$ are each independently H, D, =O, halogen, $C_{1-4}$ alkyl, $C_{1-4}$ alkoxy, CN or $C_{3-6}$ cycloalkyl, wherein the alkyl, alkoxy and cycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
optionally, one $R_4$ forms a bond with $R_{5'}$;
q is 0, 1, 2, 3 or 4;
$X_4$ is N, $NR_7$ or $CR_7$;
$X_5$ is N, $NR_{10}$ or $CR_{10}$;
ring B is six-membered heteroaryl;
$R_6$, $R_7$, $R_9$ and $R_{10}$ are independently H, D, CN, OH, halogen, $C_{1-4}$ alkyl, $C_{1-4}$ alkoxy, $C_{3-6}$ cycloalkyl or 4- to 7-membered heterocycloalkyl containing 1-3 heteroatoms selected from N, S and O, wherein the alkyl, alkoxy, cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
$R_8$ is $C_{1-4}$ alkyl, $C_{1-4}$ alkoxy, $-NHC_{1-4}$ alkyl, $-CONHC_{1-4}$ alkyl, $-CONHC_{1-4}$ alkylene$OC_{1-4}$ alkyl, $-NHCOC_{1-4}$ alkyl, $-NHCOOC_{1-4}$ alkyl, $-OCONHC_{1-4}$ alkyl, $C_{2-6}$ alkenyl, $C_{2-6}$ alkynyl, $R_{11}$, $-NHR_{11}$, $-(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, $-O(CH_2)_rR_{11}$, $-CONHR_{11}$, $-NHCOR_{11}$, $-NHCONHR_{11}$, $-CONH(CH_2)_rR_{11}$, $-CONH(CH_2)_rOR_{11}$, $-(CH_2)_rCONHR_{11}$, $-OCONHR_{11}$, $-COOR_{11}$, or $-CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each $R_{11}$ is independently $C_{3-6}$ cycloalkyl, 4- to 7-membered heterocycloalkyl containing 1-3 heteroatoms selected from N, S and O, 5- to 6-membered heteroaryl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, $-NHC_{1-4}$ alkyl, - $CONHC_{1-4}$ alkyl, $-NHCOC_{1-4}$ alkyl, OH, CN, $NH_2$ and $C_{1-4}$ haloalkyl;
each r is independently 1, 2 or 3;
provided that when

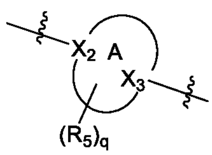

is

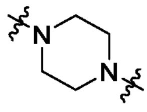,

n and m are 0,

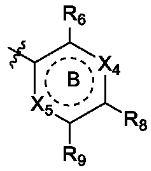

is

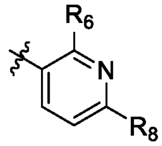

and $R_6$ is H, halogen, $C_{1-4}$ alkyl or halo$C_{1-4}$ alkyl, $R_8$ is not - $CONHC_{1-4}$ alkyl.

The present invention provides solution 6, i.e., the compound, or the stereoisomer, solvate, deuterated form or pharmaceutically acceptable salt thereof as described in solution 5, wherein $X_1$ is O;

$X_2$ is N or $CR_{5'}$;
$X_3$ is N, C or $CR_{5''}$;
ring A is 4- to 9-membered monocyclic heterocycloalkyl, 6- to 12-membered spiroheterocycloalkyl, 6- to 12-membered fused heterocycloalkyl or 6- to 12-membered bridged heterocycloalkyl containing 1-3 N atoms and 0-2 heteroatoms selected from O and S;
$R_1$ is H, D, halogen, CN, $C_{1-4}$ alkyl, $C_{2-4}$ alkenyl, $C_{2-4}$ alkynyl, $C_{1-4}$ alkoxy, $C_{3-6}$ cycloalkyl, -O-$C_{3-6}$ cycloalkyl, -$(CH_2)_r$-$C_{3-6}$ cycloalkyl, heterocycloalkyl, -O-heterocycloalkyl or -$(CH_2)_r$-heterocycloalkyl, wherein the heterocycloalkyl is 4- to 7-membered heterocycle containing 1-3 heteroatoms selected from N, S and O, wherein the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy and $C_{1-4}$ haloalkyl;
$R_2$ and $R_3$ are independently H, D, halogen or $C_{1-4}$ alkyl, wherein the alkyl is optionally substituted with 1-5 groups selected from D, halogen, OH, CN and $NH_2$;
each $R_4$ is independently selected from H, D, halogen, $C_{1-4}$ alkyl, $C_{3-6}$ cycloalkyl, OH, CN or $NH_2$, wherein the alkyl and cycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each L is independently -NH-, -$CH_2$-, -O- or -C(=O)-;
n and m are independently 0, 1, 2 or 3;
$R_5$, $R_{5'}$ and $R_{5''}$ are each independently H, D, =O, halogen, $C_{1-4}$ alkyl or $C_{1-4}$ alkoxy, wherein the alkyl and alkoxy are optionally substituted with 1-3 groups selected from D, halogen, OH, CN and $NH_2$;
optionally, one $R_4$ forms a bond with $R_{5'}$;
q is 0, 1 or 2;
$R_6$, $R_7$, $R_9$ and $R_{10}$ are independently H, D, CN, OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl.

The present invention provides solution 7, i.e., the compound, or the stereoisomer, solvate, deuterated form or pharmaceutically acceptable salt thereof as described in solution 6, wherein

ring A is substituted or unsubstituted 6- to 10-membered fused heterocycloalkyl, substituted or unsubstituted 6- to 11-membered spiroheterocycloalkyl, substituted or unsubstituted 6- to 10-membered bridged heterocycloalkyl, substituted or unsubstituted 4- to 5-membered monocyclic heterocycloalkyl, substituted or unsubstituted 7- to 8-membered monocyclic heterocycloalkyl, substituted or unsubstituted

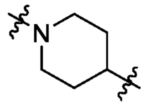,

or substituted or unsubstituted

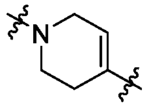;

or

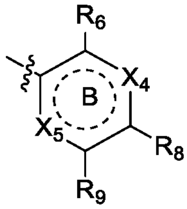

is selected from substituted or unsubstituted

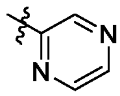 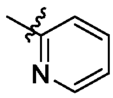;

or

$R_8$ is $C_{1-4}$ alkyl, $C_{1-4}$ alkoxy, $-NHC_{1-4}$ alkyl, $-NHCOC_{1-4}$ alkyl, $-NHCOOC_{1-4}$ alkyl, $-OCONHC_{1-4}$ alkyl, $-CONHC_{1-4}$ alkyleneO$C_{1-4}$ alkyl, $C_{2-6}$ alkenyl, $C_{2-6}$ alkynyl, $R_{11}$, $-NHR_{11}$, $-(CH_2)_rR_{11}$, $-OR_{11}$, $-COR_{11}$, $-(CH_2)_rNHR_{11}$, $-NH(CH_2)_rR_{11}$, - $(CH_2)_rOR_{11}$, $-O(CH_2)_rR_{11}$, $-CONHR_{11}$, $-NHCOR_{11}$, $-NHCONHR_{11}$, - $CONH(CH_2)_rR_{11}$, $-CONH(CH_2)_rOR_{11}$, $-COOR_{11}$, $-(CH_2)_rCONHR_{11}$, $-OCONHR_{11}$, or $-CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl.

The present invention provides solution 8, i.e., the compound, or the stereoisomer, solvate, deuterated form or pharmaceutically acceptable salt thereof as described in solution 6,
wherein
ring A is selected from

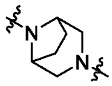 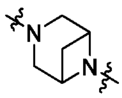 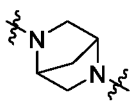 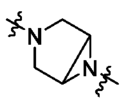 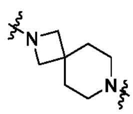 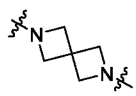 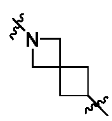

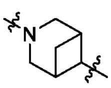 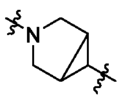 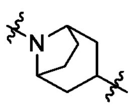 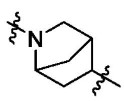 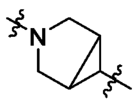 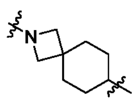 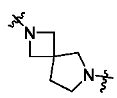

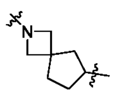 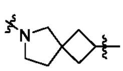 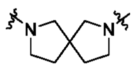 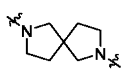 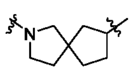 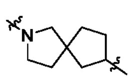 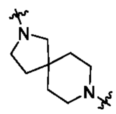

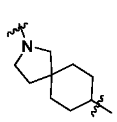 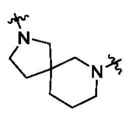 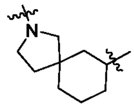 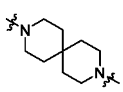 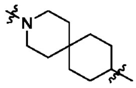 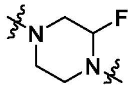 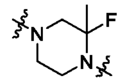

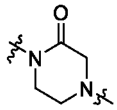 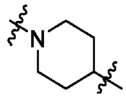 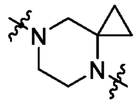 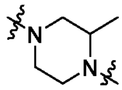 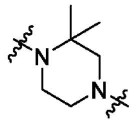 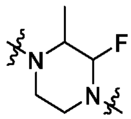 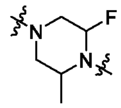

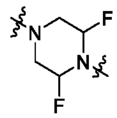 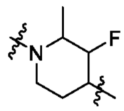 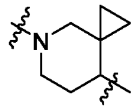 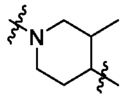 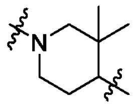 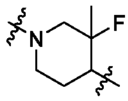 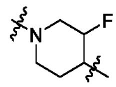

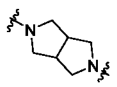 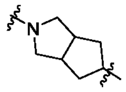 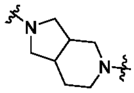 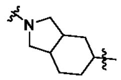 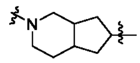 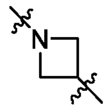 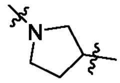

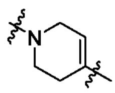 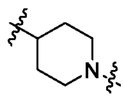 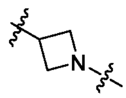 .

The present invention provides solution 9, i.e., the compound, or the stereoisomer, solvate, deuterated form or pharmaceutically acceptable salt thereof as described in solution 7 or 8,

wherein $R_1$ is H, D, F, Cl, Br, I, $C_{1-4}$ alkyl or $C_{3-6}$ cycloalkyl, wherein the alkyl and cycloalkyl are optionally substituted with 1-3 groups selected from D, halogen, $C_{1-4}$ alkyl, $C_{1-4}$ alkoxy and $C_{1-4}$ haloalkyl;
$R_2$ is H, D, F, Cl, Br, I or $C_{1-4}$ alkyl, wherein the alkyl is optionally substituted with 1-5 groups selected from D, halogen, OH, CN and $NH_2$;
$R_3$ is H, D, F, Cl, Br or I;
each $R_4$ is independently selected from H, D, F, Cl, Br or I;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
n and m are 0;
$R_5$, $R_{5'}$ and $R_{5''}$ are each independently H, D, F, Cl, Br, I, $C_{1-4}$ alkyl or $C_{1-4}$ alkoxy, wherein the alkyl and alkoxy are optionally substituted with 1-3 groups selected from D, halogen, OH, CN and $NH_2$;
optionally, one $R_4$ forms a bond with $R_{5'}$;
q is 0, 1 or 2;
ring B is six-membered heteroaryl containing 1-2 N atoms;
$R_6$, $R_7$, $R_9$ and $R_{10}$ are independently H, D, CN, OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl.

The present invention provides solution 10, i.e., a compound represented by formula (IV) or (V), or a stereoisomer, solvate, deuterated form or pharmaceutically acceptable salt thereof,

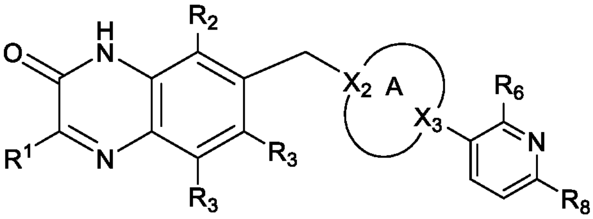 (IV)

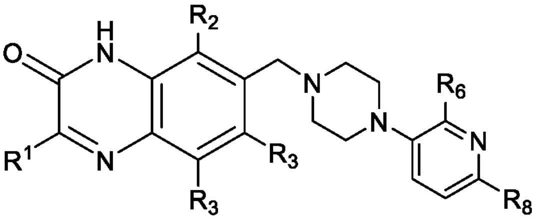 (V)

wherein

ring A is selected from

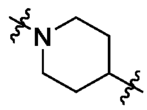, 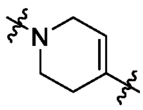, 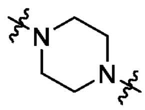;

$R_1$ is $C_{1-4}$ alkyl and $C_{3-6}$ cycloalkyl, wherein the alkyl and cycloalkyl are optionally substituted with 1-3 groups selected from D, halogen, OH, CN and $NH_2$; in some embodiments, $R_1$ is $C_{1-2}$ alkyl or $C_{3-4}$ cycloalkyl, wherein the alkyl and cycloalkyl are optionally substituted with 1, 2 and 3 groups selected from D, F, Cl, OH, CN and $NH_2$; in some embodiments, $R_1$ is $-CH_3$, $-CH_2CH_3$, $-CH_2F$, $-CH_2Cl$, - $CHF_2$, $-CHCl_2$, $-CF_3$, $-CCl_3$, $-CHFCH_3$, $-CF_2CH_3$, $-CH_2CH_2F$, $-CH_2CHF_2$, cyclopropyl, cyclobutyl,

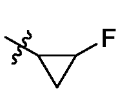, 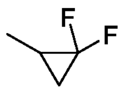, 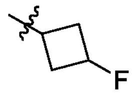 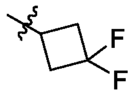;

$R_3$ is H or D;

each $R_2$ is independently F, Cl, CN, $NH_2$, $C_{1-2}$ alkyl, $C_{2-4}$ alkenyl or $C_{2-4}$ alkynyl, wherein the alkyl is optionally further substituted with 1-3 groups selected from D, F, Cl, OH, CN and $NH_2$; in some embodiments, $R_2$ is F, Cl, CN, $NH_2$, - $CH_3$, $-CD_3$, $-CH_2F$, $-CHF_2$, $-CH_2Cl$, $-CH_2CN$, $-CH_2NH_2$, $-CH_2CH_3$, $-CH_2CD_3$, - $CH_2CH_2F$, $-CH_2CH_2Cl$, $-CH_2CH_2OH$, $-CH_2CH_2CN$, $-CH_2CH_2NH_2$, ethenyl, propenyl, ethynyl or propynyl;

$R_6$ is D, CN, OH, F, Cl, $C_{1-2}$ alkyl, $C_{1-2}$ alkoxy, $C_{3-4}$ cycloalkyl or 4- to 5-membered heterocycloalkyl containing 1-3 heteroatoms selected from N, S and O, wherein the alkyl, alkoxy, cycloalkyl and heterocycloalkyl are optionally substituted with 1-3 groups selected from D, F, Cl, OH, CN and $NH_2$; in some embodiments, $R_6$ is D, CN, OH, F, Cl, $C_{1-2}$ alkyl, $C_{1-2}$ alkoxy or $C_{3-4}$ cycloalkyl, wherein the alkyl, alkoxy, cycloalkyl and heterocycloalkyl are optionally substituted with 1-3 groups selected from D, F, Cl, OH, CN and $NH_2$; in some embodiments, $R_6$ is D, CN, OH, F, Cl, $-CH_3$, $-CH_2D$, $-CD_3$, $-CH_2F$, $-CHF_2$, $-CH_2Cl$, $-CH_2CN$, $-CH_2NH_2$, $-CH_2CH_3$, $-CH_2CH_2F$, $-CH_2CH_2Cl$, $-CH_2CH_2CN$, - $CH_2CH_2NH_2$, $-OCH_3$, $-OCD_3$, $-OCH_2F$, $-OCHF_2$, $-OCH_2Cl$, $-OCH_2OH$, $-OCH_2CH_3$, $-OCH_2CH_2F$, $-OCH_2CH_2Cl$, $-OCH_2CH_2OH$, $-OCH_2CH_2NH_2$, $-OCH_2CH_2CN$, cyclopropyl, cyclobutyl, cyclopropyl, cyclobutyl,

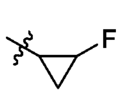, 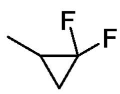, 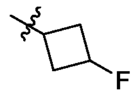

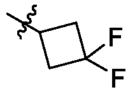;

$R_8$ is -CONHR$_{11}$ or -NHCOR$_{11}$;

each $R_{11}$ is independently $C_{3-6}$ cycloalkyl, 4- to 7-membered heterocycloalkyl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, -NHC$_{1-4}$ alkyl, - CONHC$_{1-4}$ alkyl, -NHCOC$_{1-4}$ alkyl, OH, CN, $NH_2$ and $C_{1-4}$ haloalkyl; in some embodiments, $R_{11}$ is $C_{3-6}$ cycloalkyl, 4- to 6-membered heterocycloalkyl containing 1-3 heteroatoms selected from N, S and O, or 5- to 6-membered heteroaryl containing 1-3 heteroatoms selected from N, S and O, wherein the cycloalkyl and heteroaryl are optionally substituted with 1, 2 and 3 groups selected from D, =O, F, Cl, $C_{1-2}$ alkyl, $C_{1-4}$ alkoxy, -NHC$_{1-4}$ alkyl, OH, CN, $NH_2$ and $C_{1-4}$ haloalkyl; in some embodiments, $R_{11}$ is cyclopropyl, cyclobutyl, cyclopentyl, cyclohexyl, azacyclopropyl, azetidinyl, tetrahydropyrrolyl, piperidyl, piperazinyl, tetrahydrofuryl, tetrahydropyranyl, morpholinyl, thiazolyl, thienyl, thiadiazolyl, pyrazolyl, imidazolyl, pyrrolyl, pyranyl, furyl, oxadiazolyl, pyridyl, pyrazinyl, pyridazinyl, pyrimidyl, etc., wherein the groups are optionally substituted with 1, 2 and 3 groups selected from D, =O, F, Cl, methyl, ethyl, $-OCH_3$, $-OCH_2CH_3$, - $NHCH_3$, $-NHCH_2CH_3$, OH, CN, $NH_2$ and $C_{1-4}$ haloalkyl; in some embodiments, $R_{11}$ is cyclopropyl,

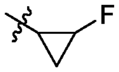, 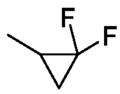,

cyclobutyl,

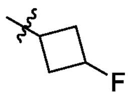 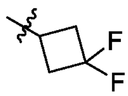,

pyrazolyl,

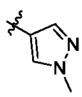, 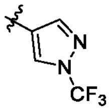, 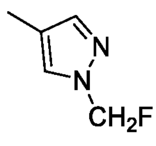, 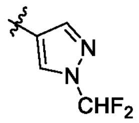.

The present invention provides solution 11, i.e., the compound represented by formula (IV) or (V), or the stereoisomer, solvate, deuterated form or pharmaceutically acceptable salt thereof as described in solution 10, wherein

$R_1$ is $C_{1-2}$ alkyl, wherein the alkyl is optionally substituted with 1-3 groups selected from D, F and Cl;
$R_3$ is H;
$R_2$ is F, Cl or $C_{1-2}$ alkyl, wherein the alkyl is optionally further substituted with 1-3 groups selected from D, F and Cl;
$R_6$ is D, F, Cl and $C_{1-2}$ alkyl, wherein the alkyl is optionally substituted with 1-3 groups selected from D, F and Cl;
$R_8$ is -CONHR$_{11}$ or -NHCOR$_{11}$;
$R_{11}$ is $C_{3-6}$ cycloalkyl or 5- to 6-membered heteroaryl containing 1-3 heteroatoms selected from N, S and O, wherein the cycloalkyl and heteroaryl are optionally substituted with 1-3 groups selected from D, F, Cl, $C_{1-2}$ alkyl and $C_{1-2}$ haloalkyl.

The present invention provides solution 12, i.e., a compound selected from one of the following structures, or a stereoisomer, solvate, deuterated form or pharmaceutically acceptable salt thereof:

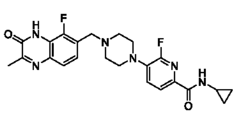 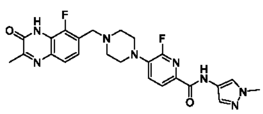 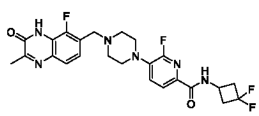 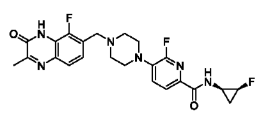

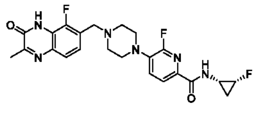
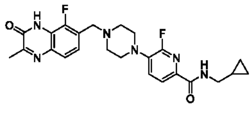
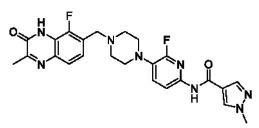
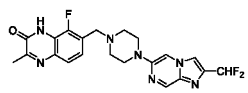

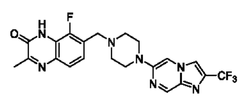
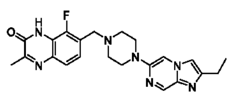
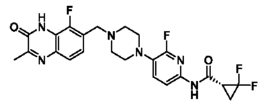
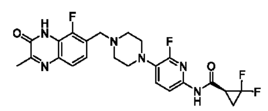

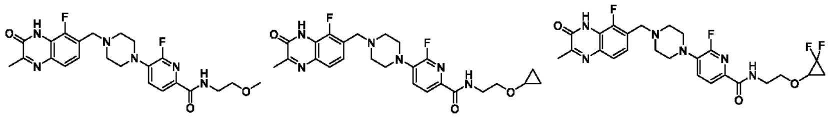

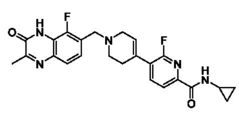
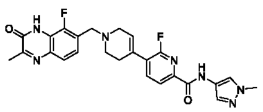
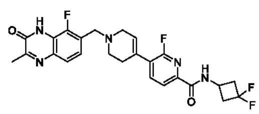
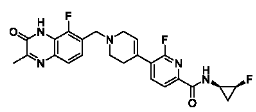

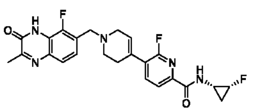
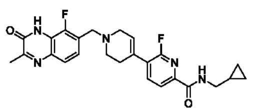
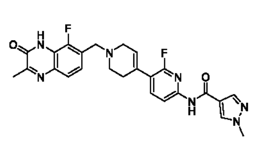
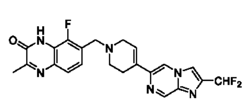

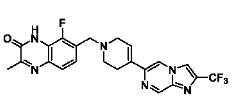
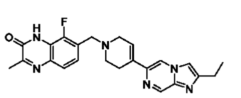
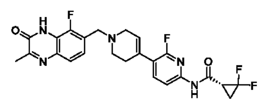
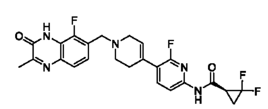

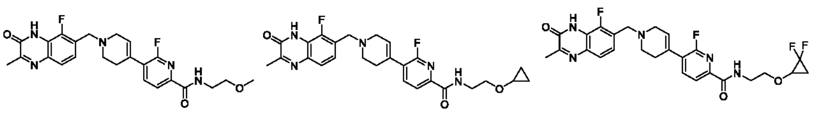

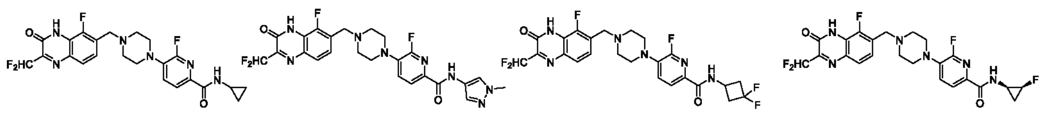

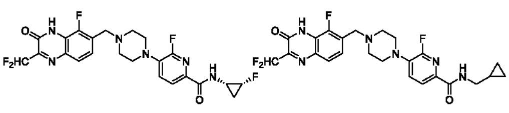
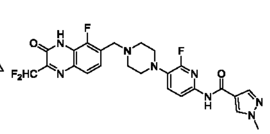
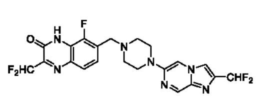

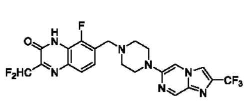
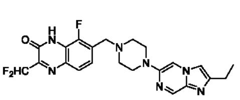
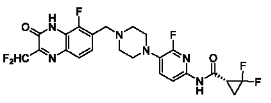
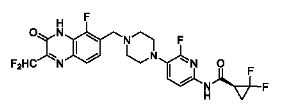

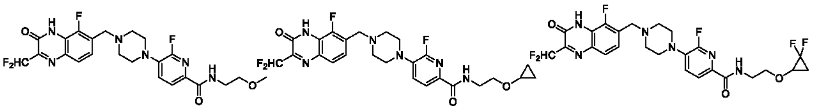

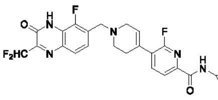
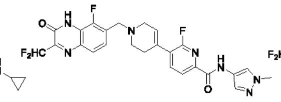
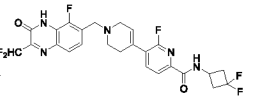
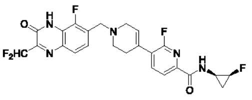

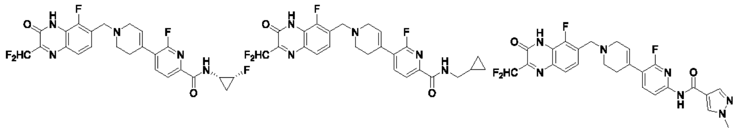
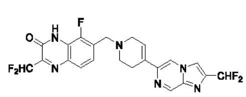

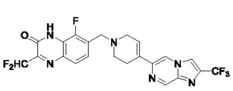
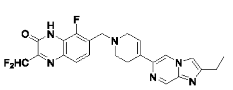
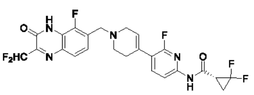
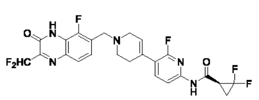

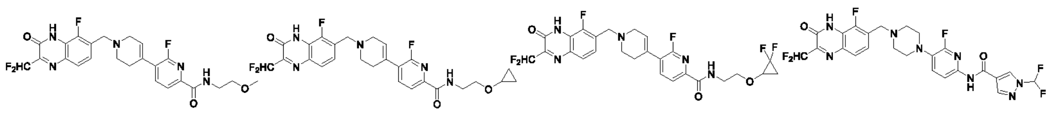

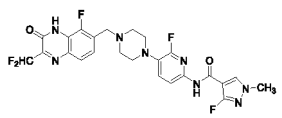

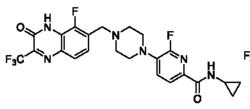
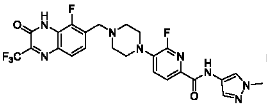
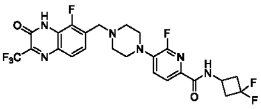
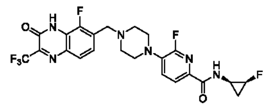

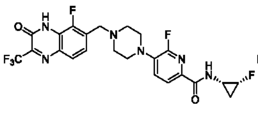
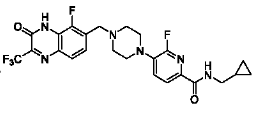
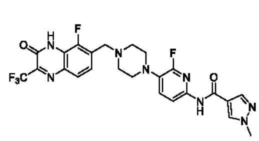
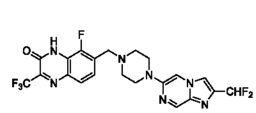

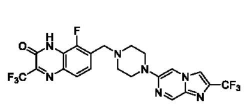
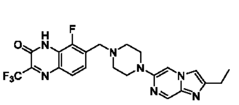
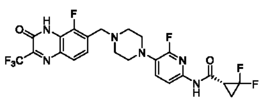
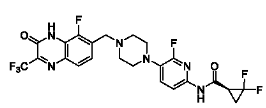

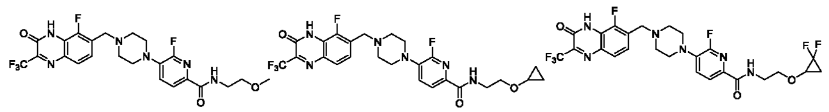

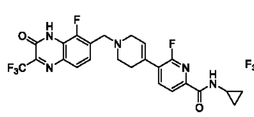
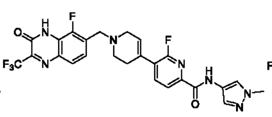
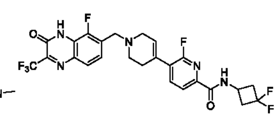
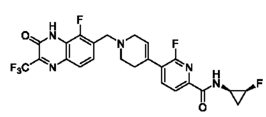

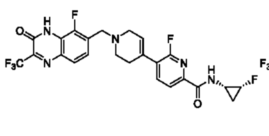
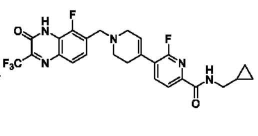
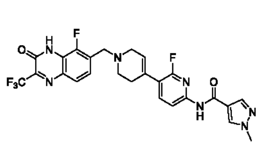
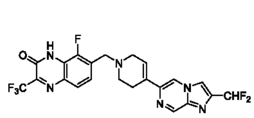

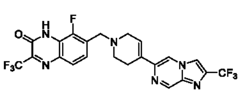
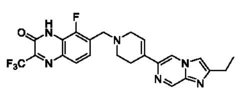
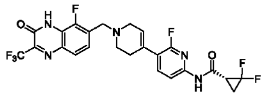
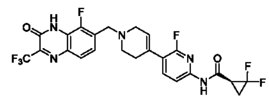

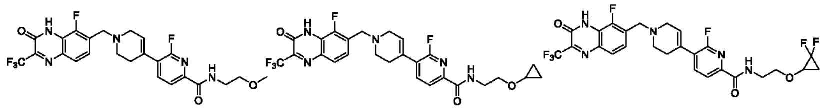

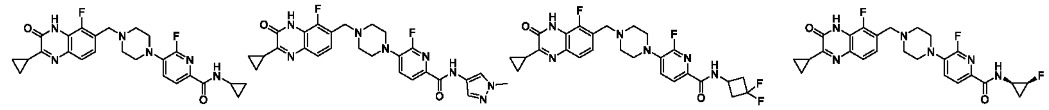

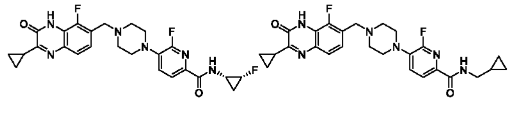
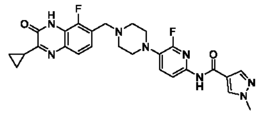
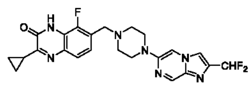

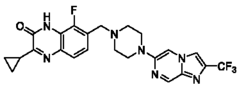
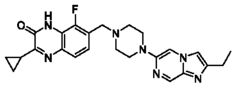
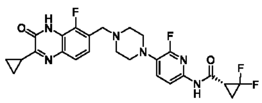
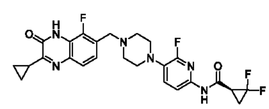

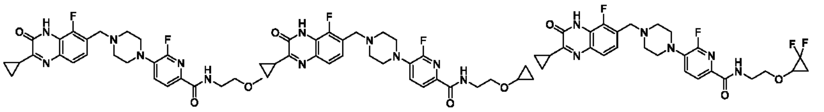

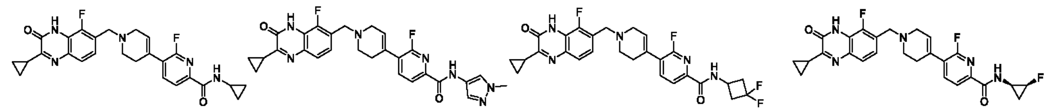

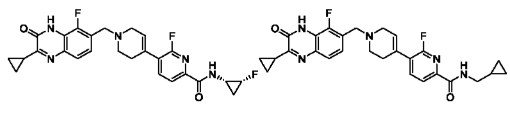
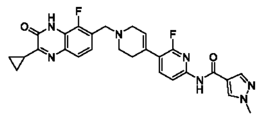
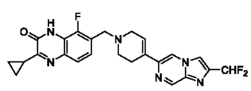

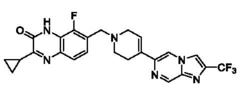
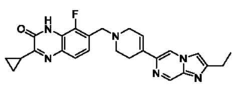
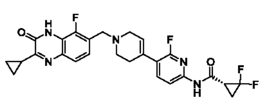
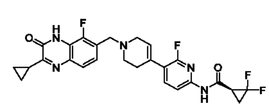

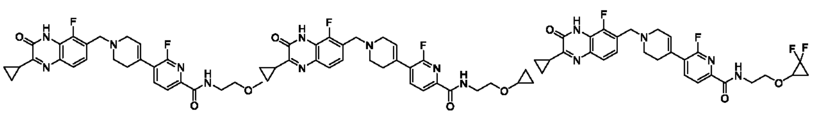

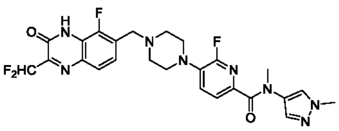
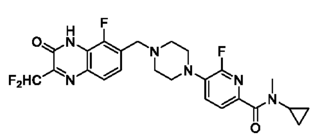
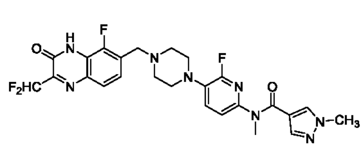.

The present invention further provides a pharmaceutical composition, comprising the compound, or the stereoisomer, solvate, deuterated form or pharmaceutically acceptable salt thereof according to any of the preceding

technical solutions, and a pharmaceutically acceptable carrier and/or excipient. The present invention further provides the use, i.e., the use of the compound, or the stereoisomer, solvate, deuterated form or pharmaceutically acceptable salt thereof, or the pharmaceutical composition according to any of the preceding technical solutions in the preparation of a drug for treating/preventing a PARP1-mediated disease.

The PARP1-mediated disease of the present invention is selected from breast cancer, uterine cancer, cervical cancer, ovarian cancer and prostate cancer.

The present invention further provides a method for treating a disease in a mammal, comprising administering to a subject a therapeutically effective amount of the compound, or the stereoisomer, solvate, deuterated form or pharmaceutically acceptable salt thereof according to any one of the preceding solutions 1 to 11, and a pharmaceutically acceptable excipient and/or carrier, wherein the therapeutically effective amount is preferably 1-1440 mg, and the disease is preferably breast cancer, uterine cancer, cervical cancer, ovarian cancer and prostate cancer.

The present invention further provides a method for treating a disease in a mammal, comprising administering to the mammal a therapeutically effective amount of the compound, or the stereoisomer, solvate, deuterated form or pharmaceutically acceptable salt thereof, or the pharmaceutical composition according to the present invention. In some embodiments, the mammal mentioned in the present invention includes human.

The "effective amount" or "therapeutically effective amount" as described in the present application refers to administration of a sufficient amount of the compound disclosed in the present application that will alleviate to some extent one or more symptoms of the diseases or conditions being treated. In some embodiments, the outcome is the reduction and/or remission of signs, symptoms or causes of the disease, or any other desired change in the biological system. For example, an "effective amount" in terms of the therapeutic use is an amount comprising the compound disclosed in the present application that is required to provide clinically significant reduction of the symptoms of the disease. Examples of the therapeutically effective amount include, but are not limited to 1-1440 mg, 1-1400 mg, 1-1300 mg, 1-1200 mg, 1-1000 mg, 1-900 mg, 1-800 mg, 1-700 mg, 1-600 mg, 1-500 mg, 1-400 mg, 1-300 mg, 1-250 mg, 1-200 mg, 1-150 mg, 1-125 mg, 1-100 mg, 1-80 mg, 1-60 mg, 1-50 mg, 1-40 mg, 1-25 mg, 1-20 mg, 5-1000 mg, 5-900 mg, 5-800 mg, 5-700 mg, 5-600 mg, 5-500 mg, 5-400 mg, 5-300 mg, 5-250 mg, 5-200 mg, 5-150 mg, 5-125 mg, 5-100 mg, 5-90 mg, 5-70 mg, 5-80 mg, 5-60 mg, 5-50 mg, 5-40 mg, 5-30 mg, 5-25 mg, 5-20 mg, 10-1000 mg, 10-900 mg, 10-800 mg, 10-700 mg, 10-600 mg, 10-500 mg, 10-450 mg, 10-400 mg, 10-300 mg, 10-250 mg, 10-200 mg, 10-150 mg, 10-125 mg, 10-100 mg, 10-90 mg, 10-80 mg, 10-70 mg, 10-60 mg, 10-50 mg, 10-40 mg, 10-30 mg, 10-20 mg; 20-1000 mg, 20-900 mg, 20-800 mg, 20-700 mg, 20-600 mg, 20-500 mg, 20-400 mg, 20-350 mg, 20-300 mg, 20-250 mg, 20-200 mg, 20-150 mg, 20-125 mg, 20-100 mg, 20-90 mg, 20-80 mg, 20-70 mg, 20-60 mg, 20-50 mg, 20-40 mg, 20-30 mg; 50-1000 mg, 50-900 mg, 50-800 mg, 50-700 mg, 50-600 mg, 50-500 mg, 50-400 mg, 50-300 mg, 50-250 mg, 50-200 mg, 50-150 mg, 50-125 mg, 50-100 mg; 100-1000 mg, 100-900 mg, 100-800 mg, 100-700 mg, 100-600 mg, 100-500 mg, 100-400 mg, 100-300 mg, 100-250 mg, or 100-200 mg.

The present invention relates to a pharmaceutical composition or pharmaceutical preparation, comprising a therapeutically effective amount of the compound, or the stereoisomer, solvate, deuterated form or pharmaceutically acceptable salt thereof according to the present invention, and an excipient and/or carrier. The pharmaceutical composition can be in a unit preparation form (the amount of the active drug in the unit preparation is also referred to as the "preparation specification"). In some embodiments, the pharmaceutical composition comprises the compound, or the stereoisomer, solvate, deuterated form or pharmaceutically acceptable salt thereof according to the present invention in an amount including but not limited to 1-1440 mg, 5-1000 mg, 10-800 mg, 20-600 mg, 25-500 mg, 40-200 mg, 50-100 mg, 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 110 mg, 120 mg, 125 mg, 130 mg, 140 mg, 150 mg, 160 mg, 170 mg, 180 mg, 190 mg, 200 mg, 210 mg, 220 mg, 230 mg, 240 mg, 250 mg, 275 mg, 300 mg, 325 mg, 350 mg, 375 mg, 400 mg, 425 mg, 450 mg, 475 mg, 500 mg, 525 mg, 550 mg, 575 mg, 600 mg, 625 mg, 650 mg, 675 mg, 700 mg, 725 mg, 750 mg, 775 mg, 800 mg, 850 mg, 900 mg, 950 mg, 1000 mg, 1100 mg, 1200 mg, 1300 mg, 1400 mg, or 1440 mg.

Provided is a method for treating a disease in a mammal, comprising administering to a subject a therapeutically effective amount of the compound, or the stereoisomer, solvate, deuterated form or pharmaceutically acceptable salt thereof according to the present invention, and a pharmaceutically acceptable excipient and/or carrier, wherein the therapeutically effective amount is preferably 1-1440 mg, and the disease is preferably breast cancer, uterine cancer, cervical cancer, ovarian cancer and prostate cancer.

Provided is a method for treating a disease in a mammal, comprising administering to a subject a drug, i.e., the compound, or the stereoisomer, solvate, deuterated form or pharmaceutically acceptable salt thereof according to the present invention, and a pharmaceutically acceptable excipient and/or carrier in a daily dose of 1-1440 mg/day, wherein the daily dose can be a single dose or divided doses; in some embodiments, the daily dose includes, but is not limited to 10-1440 mg/day, 20-1440 mg/day, 25-1440 mg/day, 50-1440 mg/day, 75-1440 mg/day, 100-1440 mg/day, 200-1440 mg/day, 10-1000 mg/day, 20-1000 mg/day, 25-1000 mg/day, 50-1000 mg/day, 75-1000 mg/day, 100-1000 mg/day, 200-1000 mg/day, 25-800 mg/day, 50-800 mg/day, 100-800 mg/day, 200-800 mg/day, 25-400 mg/day, 50-400 mg/day, 100-400 mg/day, or 200-400 mg/day; in some embodiments, the daily dose includes, but is not limited to 1 mg/day, 5 mg/day, 10 mg/day, 20 mg/day, 25 mg/day, 50 mg/day, 75 mg/day, 100 mg/day, 125 mg/day, 150 mg/day, 200 mg/day, 400

mg/day, 600 mg/day, 800 mg/day, 1000 mg/day, 1200 mg/day, 1400 mg/day, or 1440 mg/day.

The present invention relates to a kit, which may include a composition in single-dose or multiple-dose form, wherein the kit comprises the compound, or the stereoisomer, solvate, deuterated form or pharmaceutically acceptable salt thereof according to the present invention, and the amount of the compound, or the stereoisomer, solvate, deuterated form or pharmaceutically acceptable salt thereof according to the present invention is the same as that in the above-mentioned pharmaceutical composition.

In the present invention, the amount of the compound, or the stereoisomer, solvate, deuterated form or pharmaceutically acceptable salt thereof according to the present invention is calculated in the form of a free base in each case.

The term "preparation specification" refers to the weight of the active drug contained in each vial, tablet or other unit preparation.

**Synthetic route**

Patent documents such as WO 2021013735 A1 introduce a method for preparing a PARP-1 inhibitor, and those skilled in the art would have been able to prepare the compounds of the present invention by means of combining the document and known organic synthesis techniques, wherein the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. Specific and similar reactants can be selectively identified by the indexes of known chemicals prepared by the Chemical Abstracts Service of the American Chemical Society, wherein the indexes are available in most public libraries and university libraries and online. Chemicals that are known but not commercially available in the catalogue are optionally prepared by custom chemical synthesis plants, wherein many of standard chemical supply plants (such as those listed above) provide custom synthesis services.

**Term**

Unless otherwise specified, the terms of the present invention have the following meanings.

The carbon, hydrogen, oxygen, sulphur, nitrogen or halogen involved in the groups and compounds of the present invention all include isotopes thereof, and are optionally further replaced by one or more of the corresponding isotopes thereof, wherein the isotopes of carbon include $^{12}C$, $^{13}C$ and $^{14}C$; the isotopes of hydrogen include protium (H), deuterium (D, also known as heavy hydrogen) and tritium (T, also known as superheavy hydrogen); the isotopes of oxygen include $^{16}O$, $^{17}O$ and $^{18}O$; the isotopes of sulphur include $^{32}S$, $^{33}S$, $^{34}S$ and $^{36}S$; the isotopes of nitrogen include $^{14}N$ and $^{15}N$; the isotope of fluorine includes $^{19}F$; the isotopes of chlorine include $^{35}Cl$ and $^{37}Cl$; and the isotopes of bromine include $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H), which is synonymous with "D".

The term "deuterated" or "deuterated form" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms. "$C_0$" usually indicates the bond.

The term "alkyl" refers to a monovalent straight or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl,

sec-butyl, neobutyl, tert-butyl, n-pentyl, isopentyl, neopentyl, n-hexyl, etc., and alkyl may be further substituted with a substituent.

The term "alkylene" refers to a divalent straight or branched saturated alkyl group. Examples of alkylene include, but are not limited to methylene, ethylidene, *etc.*

The term "haloalkyl" refers to an alkyl group in which one or more hydrogens are replaced by one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. Specific examples include, but are not limited to $-CF_3$, $-CH_2Cl$, $-CH_2CF_3$, $-CCl_2$, $CF_3$, etc.

The term "alkoxy" or "alkyloxy" refers to -O-alkyl, such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, sec-butoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl, such as -O-halo $C_{1-8}$ alkyl, -O-halo $C_{1-6}$ alkyl, -O-halo $C_{1-4}$ alkyl or -O-halo $C_{1-2}$ alkyl; the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Examples of alkenyl include, but are not limited to ethenyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkenylene" refers to a linear or branched divalent unsaturated hydrocarbon group comprising at least one carbon-carbon double bond (C=C) and generally comprises 2 to 18 carbon atoms, such as 2 to 8 carbon atoms, further such as 2 to 6 carbon atoms, and still further such as 2 to 4 carbon atoms. Non-limiting examples of alkenylene include ethynylene and the alkenylene may be optionally substituted with a substituent.

The term "alkynyl" refers to a straight or branched hydrocarbon group comprising at least one carbon-carbon triple bond (C≡C) and generally comprises 2 to 18 carbon atoms, further comprises 2 to 8 carbon atoms, further comprises 2 to 6 carbon atoms, and still further comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "alkynylene" refers to a linear or branched divalent unsaturated hydrocarbon group containing a carbon-carbon triple bond (C≡C) and generally comprises 2 to 18 carbon atoms, further comprises 2 to 8 carbon atoms, further comprises 2 to 6 carbon atoms, and further comprises 2 to 4 carbon atoms. Non-limiting examples of alkynylene include ethynylene, propynylene and butynylene; and the alkynylene may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic; the bicyclic or polycyclic cycloalkyl may be in the form of a fused ring, a spiro ring, a bridged ring or a combination thereof, and may comprise one or more aromatic rings, but the ring system is non-aromatic as a whole; and the connection site is on a non-aromatic ring. Generally, the cycloalkyl contains 3 to 20 carbon atoms, further contains 3-8 carbon atoms, and still further contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms. Non-limiting examples of cycloalkyl include cyclopropyl, cyclobutyl, cyclopentyl, cyclohexyl, cycloheptyl, butenyl, cyclopentenyl, cyclohexenyl,

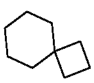, 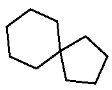, 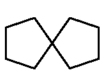, 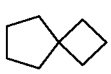, 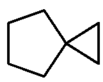, 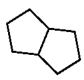,

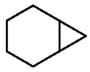, 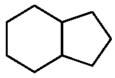, 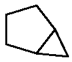, 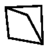, 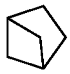, 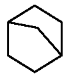, 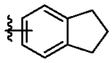,

etc., and the cycloalkyl may be optionally substituted with a substituent.

The term "cycloalkylene" refers to a divalent group of cycloalkyl.

The term "aryl" refers to an aromatic carbocycle that does not contain heteroatoms, including monocyclic aryl and fused aryl. Generally, the aryl contains 6 to 14 carbon atoms, and further contains 6 to 10 carbon atoms. Non-limiting examples of aryl include phenyl, naphthyl, anthryl, phenanthryl, and the aryl may be optionally substituted with a substituent.

"Carbocycle" or "carbocyclyl" refers to a saturated, partially unsaturated, or aromatic carbocycle, and its meaning includes aryl and cycloalkyl. The carbocycle may be monocyclic, bicyclic or polycyclic, and the bicyclic or polycyclic carbocycle may be in the form of a bridged ring, a fused ring, a spiro ring and a combination thereof. Generally, the carbocycle contains 3-12 carbon atoms, or 3-10 carbon atoms, or 3-6 carbon atoms. Non-limiting examples of the monocyclic carbocycle include cyclopropyl, cyclobutyl, cyclopentyl, cyclohexyl, cycloheptyl, phenyl, *etc.* A bicyclic bridged ring includes

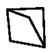, 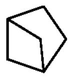, 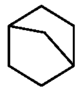,

*etc.,* a **bicyclic** fused ring includes

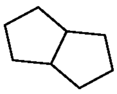, 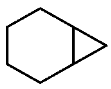, 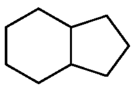, 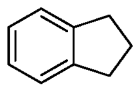, 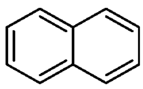, 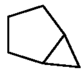,

*etc.,* and a bicyclic spiro ring includes

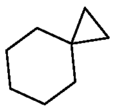, 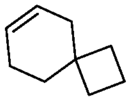, 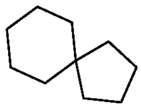, 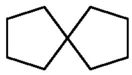,

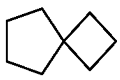, 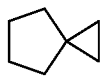,

*etc.* The carbocycle may be optionally substituted with a substituent.

"Heterocycloalkyl" refers to a saturated or partially unsaturated non-aromatic carbocycle containing 1, 2, 3, or 4 heteroatoms selected from N, S or O. The heterocycloalkyl may be monocyclic, bicyclic or polycyclic; the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole; and the connecting site is on a non-aromatic ring. Generally, the heterocycloalkyl is a 3- to 20-membered ring. When the heterocycloalkyl is monocyclic heterocycloalkyl, the heterocycloalkyl is usually a 3- to 15-membered ring, or a 3- to 10-membered ring, or a 3- to 8-membered ring, or a 3- to 6-membered ring; when the heterocycloalkyl is bicyclic or polycyclic heterocycloalkyl, the heterocycloalkyl is usually a 5- to 12-membered ring, or a 5- to 11-membered ring, or a 6- to 9-membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and the heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which may be monocyclic, bicyclic or polycyclic, wherein the bicyclic or polycyclic heteroaromatic ring or heteroaryl may be in the form of a bridged ring, a fused ring, a spiro ring and a combination thereof. The bicyclic or polycyclic heteroaromatic ring or heteroaryl can be formed by fusion of heteroaryl to aryl, or of heteroaryl to heteroaryl, wherein the heteroaryl or aryl may be the attachment site. Non-limiting examples of heteroaromatic ring or heteroaryl include furyl, thienyl, pyrrolyl, oxazolyl, thiazolyl, imidazolyl, pyrazolyl, pyridyl, pyrimidyl,

pyridazinyl, pyrazinyl, indolyl, purinyl,

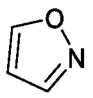, 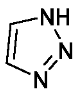, 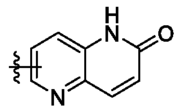,

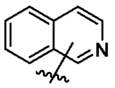, 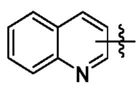, 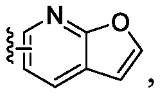, 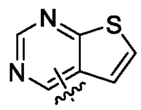, 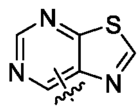, 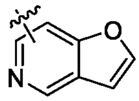,

etc. The heteroaryl may be optionally substituted with a substituent.

The term "heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. The heterocycle may be in the form of a monocyclic heterocycle, a bicyclic bridged heterocycle, a bicyclic fused heterocycle, a bicyclic spiro heterocycle or a combination thereof. The heterocycle is usually a 3- to 12-membered heterocycle, or a 5- to 12-membered heterocycle, or a 5- to 7-membered heterocycle. Heterocyclyl can be connected to a heteroatom or a carbon atom. Non-limiting examples of heterocyclyl include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furyl, thienyl, pyranyl, N-alkylpyrrolyl, pyrimidyl, pyrazinyl, pyrazolyl, pyridazinyl, imidazolyl, piperidyl, piperadinyl, morpholinyl, thiomorpholinyl, 1,3-dithianyl, dihydrofuryl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzoimidazolyl, benzopyridyl, pyrrolopyridyl, benzodihydrofuryl, azabicyclo[3.2.1]octanyl, azabicyclo[5.2.0]nonanyl, oxatricyclo[5.3.1.1]dodecyl, azaadamantyl and oxaspiro[3.3]heptanyl,

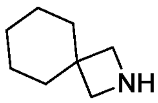, 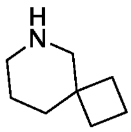, 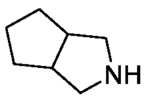,

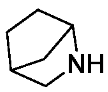, 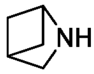, 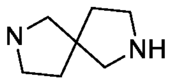, 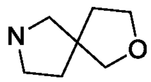, 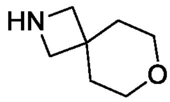, 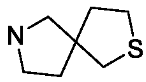,

etc., and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

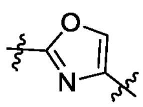, 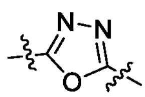, 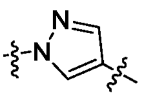, 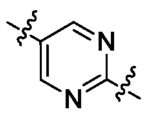,

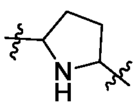,

*etc.*

The term "spiro ring" refers to a polycyclic group sharing one carbon atom (referred to as a spiro atom) between rings, which may contain 0 or at least 1 double or triple bond, and may contain 0 to 5 heteroatoms selected from N, O, S, P, Si and their oxidation states. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of the spiro ring include:

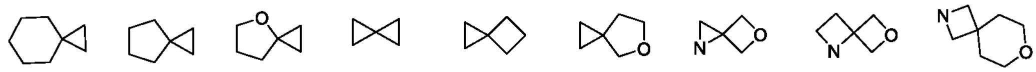

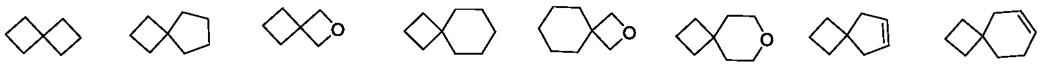

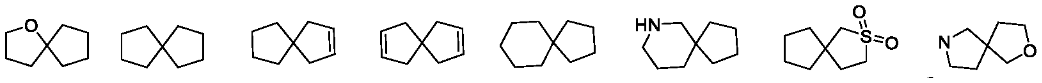

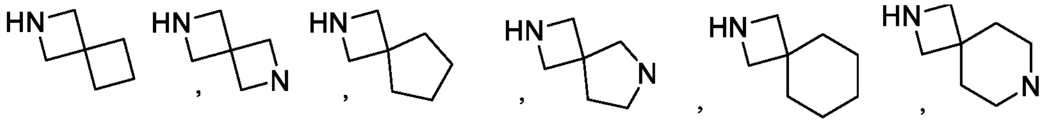

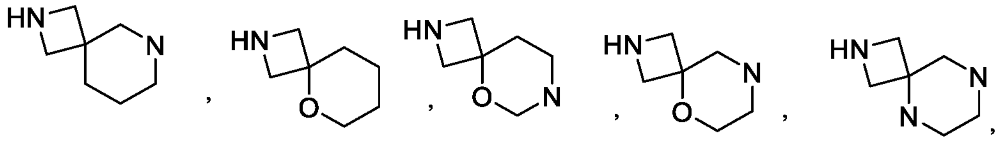

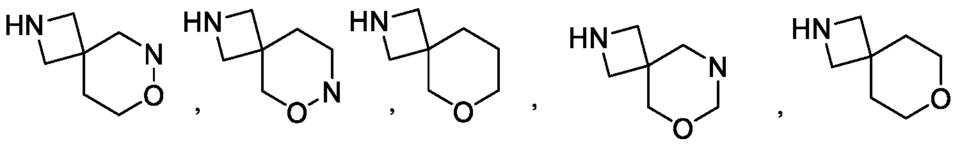

,

and the spiro ring may be optionally substituted with a substituent.

The term "fused ring" or "fused" refers to a polycyclic group in which the rings share two adjacent ring atoms and one chemical bond, may contain one or more double or triple bonds, and may contain 0 to 5 heteroatoms selected from N, S, O, P, Si and their oxidation states. Generally, a fused ring is a 5- to 20-membered ring, or a 5- to 14-membered ring, or a 5- to 12-membered ring or a 5-to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring. Non-limiting examples of fused ring include purine, quinoline, isoquinoline, benzopyran, benzofuran, benzothiophene, and

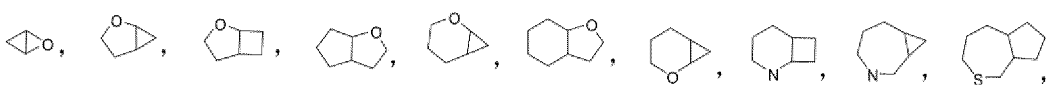

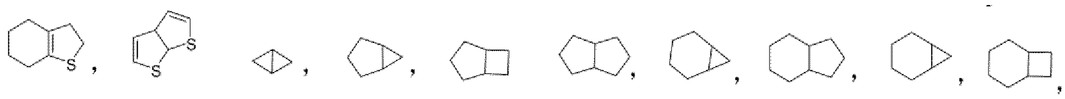

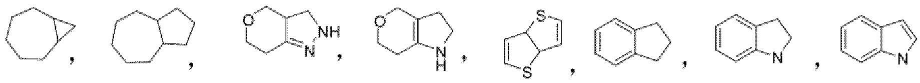

;

and the fused ring may be aromatic or non-aromatic and is optionally substituted with a substituent.

The term "bridged ring" refers to a ring system in which two non-adjacent ring atoms are shared between two

rings, which may contain one or more double or triple bonds. The bridged ring may contain 0 to 5 heteroatoms selected from N, S, O, P, Si and their oxidation states. Generally, the bridged ring has 5 to 20, or 5 to 14, or 5 to 12, or 5 to 10 ring atoms. Non-limiting examples of the bridged ring include adamantane,

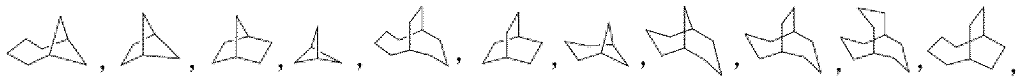

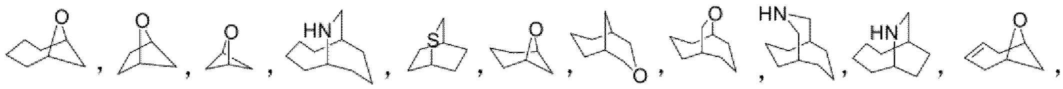

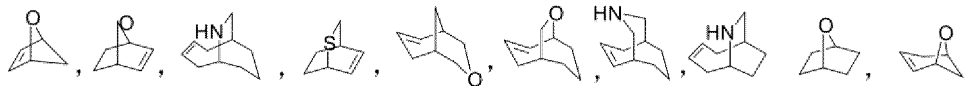

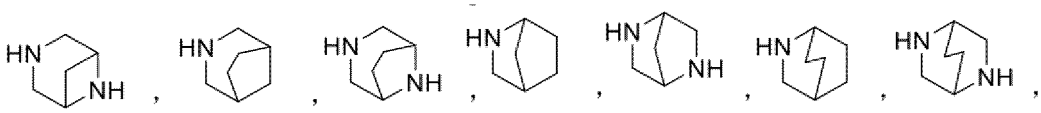

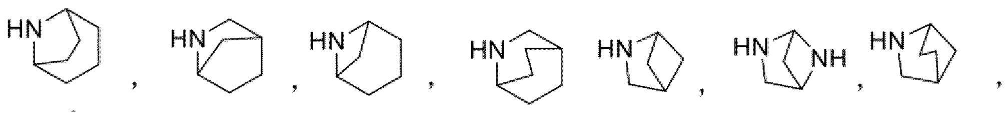

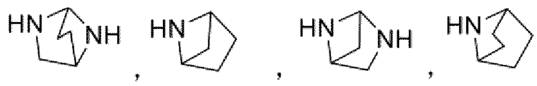

Unless otherwise specified, the term "substitution" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}$ alkyl), $-OC(=O)(C_{1-6}$ alkyl), $-OCO_2(C_{1-6}$ alkyl), $-C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)C(=O)($C_{1-6}$ alkyl), $-NHCO_2(C_{1-6}$ alkyl), $-NHC(=O)N(C_{1-6}$ alkyl$)_2$, $-HC(=O)NH(C_{1-6}$ alkyl), $-NHC(=O)NH_2$, $-NHSO_2(C_{1-6}$ alkyl), $-SO_2N(C_{1-6}$ alkyl$)_2$, $-SO_2NH(C_{1-6}$ alkyl), $-SO_2NH_2$, $-SO_2C_{1-6}$ alkyl, etc.

The term "optional" or "optionally" refers to that the events or circumstances subsequently described may but not necessarily occur, and the description includes the occasions where the events or circumstances occur or do not occur. For example, "alkyl optionally substituted with F" means that the alkyl may but not necessarily be substituted with F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" represents a mixture of one or more compounds described herein or the stereoisomers, solvates, pharmaceutically acceptable salts or co-crystals thereof and other components comprising physiologically/pharmaceutically acceptable carriers and/or excipients.

The term "carrier" refers to: a system that does not cause significant irritation to the organism and does not eliminate the biological activity and characteristics of the administered compound and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating

agent, an absorbent, a preservative, a surfactant, a colorant, a flavouring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatine; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerine, sorbitol, mannitol and polyethylene glycol; (12) esters, such as ethyl oleate and ethyl laurate; (13) agar; (14) buffers, such as magnesium hydroxide and aluminium hydroxide; (15) alginic acid; (16) pyrogen-free water; (17) isotonic saline; (18) Ringer's solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

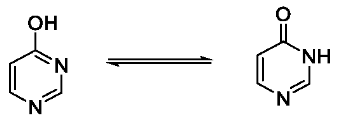

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

Detailed Description of Embodiments

The technical solutions of the present invention will be described in detail below in conjunction with examples, but the protection scope of the present invention includes but is not limited thereto.

**Detection method**

The structure of the compound is determined by nuclear magnetic resonance (NMR) or (and) mass spectrometry (MS). The NMR shift (δ) is given in the unit of 10-6 (ppm). NMR is determined with (Bruker Avance III 400 and Bruker Avance 300) nuclear magnetic resonance instrument; the solvents for determination are deuterated dimethyl sulfoxide (DMSO-$d_6$), deuterated chloroform ($CDCl_3$) and deuterated methanol ($CD_3OD$); and the internal standard is tetramethylsilane (TMS).

MS is determined with Agilent 6120B (ESI) and Agilent 6120B (APCI); and

HPLC is determined with Agilent 1260DAD high pressure liquid chromatograph (Zorbax SB-C18 100 × 4.6 mm, 3.5 μM);

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm;

and for the column chromatography, Yantai Huanghai silica gel of 200-300 mesh silica gel is generally used as a carrier.

**Example 1:**

N-cyclopropyl-6-fluoro-5-(4-((5-fluoro-2-methyl-3-oxo-3,4-dihydroquinoxalin-6-yl)methyl)piperazin-1-yl)picolinamide (compound 1)

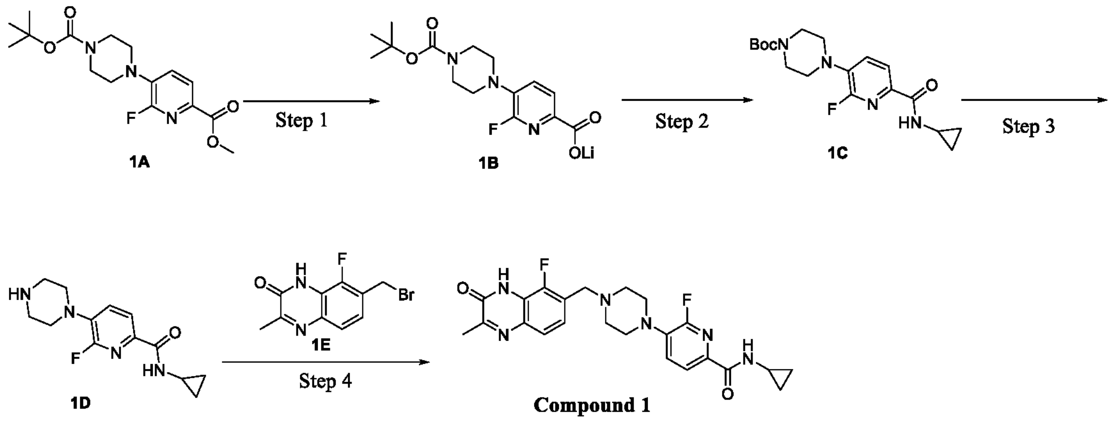


Step 1: Compound 1A (synthesized according to patent US 2022/0009901 A1) (3.39 g, 10 mmol) was dissolved in tetrahydrofuran (100 mL) and water (10 mL), and lithium hydroxide (400 mg, 16.7 mmol) was added. The reaction was stirred at room temperature for 2 h. The solvent was removed by distillation under reduced pressure to obtain 1B (3.38 g, 100%) as a white powdery solid, which was directly used in the next step without purification.

LC-MS (ESI): m/z= 324.2 $[M-H]^+$.

Step 2: Compound 1B (331 mg, 1 mmol) was dissolved in DMF (10 mL), and HATU (565 mg, 1.49 mmol) was added with stirring. The mixture was stirred at room temperature. After the solid was completely dissolved, DIEPA (2 mL) was added, and excess cyclopropylamine was finally added. The reaction was stirred at room temperature for 4 h. After the reaction was completed as monitored by LCMS, ethyl acetate (50 mL) was added to the system. The mixture was washed with water (50 mL × 4). The organic phase was collected, dried over anhydrous sodium sulphate, filtered and concentrated, and then the residue was separated by silica gel column chromatography (PE : EA = 1 : 0-1 : 1) to obtain the title compound 1C (327 mg, 89.8%).

LC-MS (ESI): m/z = 365.2 $[M+H]^+$.

Step 3: 1C (327 mg, 0.90 mmol) was dissolved in methanol (10 mL), and a solution of hydrogen chloride in dioxane (3 mL, 4 M) was added. The mixture was reacted at room temperature for 2 hours and spun to dryness to obtain the title compound 1D (276 mg, crude).

LC-MS (ESI): m/z = 265.1 $[M+H]^+$.

Step 4: 1E (synthesized according to patent US 2022/0009901 A1) (200 mg, 0.74 mmol) and 1D (276 mg) were dissolved in anhydrous acetonitrile (20 mL), and potassium iodide (8 mg, 0.05 mmol) and DIPEA (1 mL) were added. The mixture was subjected to nitrogen replacement, and then reacted at 60°C for 4 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and purified by column chromatography (DCM : MeOH = 1 : 0-0 : 1) to obtain compound 1 (189 mg, 63.5%).

$^1H$ NMR (400 MHz, DMSO-$d_6$) δ 12.18 (s, 1H), 9.20 - 8.02 (m, 1H), 7.96 - 7.72 (m, 1H), 7.75 - 7.44 (m, 2H), 7.36 - 7.19 (m, 1H), 3.69 (s, 2H), 3.23 - 3.06 (m, 4H), 2.90 - 2.79 (m, 1H), 2.66 - 2.53 (m, 4H), 2.42 (s, 3H), 0.80 - 0.48 (m, 4H).

LC-MS (ESI): m/z = 455.2 $[M+H]^+$.

**Example 2**

6-fluoro-5-(4-((5-fluoro-2-methyl-3-oxo-3,4-dihydroquinoxalin-6-yl)methyl)piperazin-1-yl)-N-(1-methyl-1H-pyrazol-4-yl) picolinamide (compound 2)

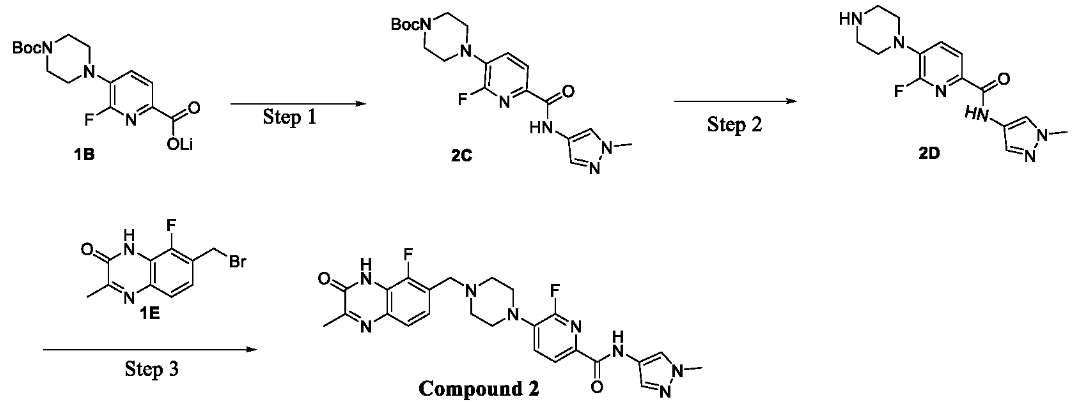


1B 2C 2D 1E Compound 2

Step 1: Compound 1B (331 mg, 1 mmol) was dissolved in DMF (10 mL), and HATU (565 mg, 1.49 mmol) was added with stirring. The mixture was stirred at room temperature. After the solid was completely dissolved, DIEPA (2 mL) was added, and 1-methyl-1H-pyrazol-4-amine hydrochloride (246 mg, 1.84 mmol) was finally added. The reaction was stirred at room temperature for 4 h. After the reaction was completed as monitored by LCMS, ethyl acetate (50 mL) was added to the system. The mixture was washed with water (50 mL × 4). The organic phase was collected, dried over anhydrous sodium sulphate, filtered and concentrated, and then the residue was separated by silica gel column chromatography (PE : EA = 1 : 0-1 : 1) to obtain the title compound 2C (352 mg, 87.03%).

LC-MS (ESI): m/z = 405.2 $[M+H]^+$.

Step 2: 2C (352 mg, 0.88 mmol) was dissolved in methanol (10 mL), and a solution of hydrogen chloride in dioxane (4 mL, 4 M) was added. The mixture was reacted at room temperature for 4 hours and spun to dryness to obtain the title compound 2D (313 mg, crude).

LC-MS (ESI): m/z = 305.2 $[M+H]^+$.

Step 3: 1E (200 mg, 0.74 mmol) and 2D (313 mg) were dissolved in anhydrous acetonitrile (20 mL), and potassium iodide (8 mg, 0.05 mmol) and DIPEA (1 mL, 5.74 mmol) were added. The mixture was subjected to nitrogen replacement, and then reacted overnight at 60°C. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and subjected to column chromatography (DCM : MeOH = 1 : 0-0 : 1) to obtain compound 2 (236 mg, 64.48%).

$^1$H NMR (400 MHz, DMSO-$d_6$) δ 12.40 (s, 1H), 10.48 (s, 1H), 8.03 (s, 1H), 7.95 - 7.91 (m, 1H), 7.70 (s, 1H), 7.63 - 7.56 (m, 1H), 7.54 - 7.49 (m, 1H), 7.37 - 7.26 (m, 1H), 3.81 (s, 3H), 3.71 (s, 2H), 3.24 - 3.16 (m, 4H), 2.64 - 2.57 (m, 4H), 2.42 (s, 3H).

LC-MS (ESI): m/z = 495.2 $[M+H]^+$.

### Example 3

N-(3,3-difluorocyclobutyl)-6-fluoro-5-(4-((5-fluoro-2-methyl-3-oxo-3,4-dihydroquinoxalin-6-yl)methyl)piperazin-1-yl)picolinamide (compound 3)

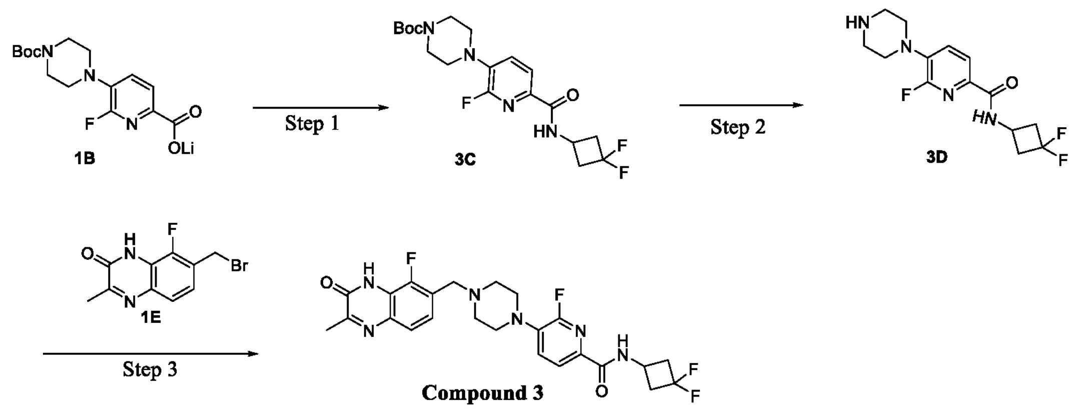


1B 3C 3D 1E Compound 3

Step 1: Compound 1B (331 mg, 1 mmol) was dissolved in DMF (10 mL), and HATU (565 mg, 1.5 mmol) was added with stirring. The mixture was stirred at room temperature. After the solid was completely dissolved, DIEPA (2 mL) was added, and 3,3-difluorocyclobutylamine (214 mg, 2 mmol) was finally added. The reaction was stirred at room temperature for 4 h. After the reaction was completed as monitored by LCMS, ethyl acetate (50 mL) was added to the system. The mixture was washed with water (50 mL × 4). The organic phase was collected, dried over anhydrous sodium sulphate, filtered and concentrated under reduced pressure, and then the residue was separated by silica gel column chromatography (PE : EA = 1 : 0-1 : 1) to obtain the title compound 3C (349 mg, 84.30%).

LC-MS (ESI): m/z = 415.2 $[M+H]^+$.

Step 2: 3C (349 mg, 0.85 mmol) was dissolved in methanol (10 mL), and a solution of hydrogen chloride in dioxane (4 mL, 4 M) was added. The mixture was reacted at room temperature for 4 hours and spun to dryness to obtain the title compound 3D (301 mg, crude).

LC-MS (ESI): m/z = 315.2 $[M+H]^+$.

Step 3: 1E (200 mg, 0.74 mmol) and 3D (301 mg) were dissolved in anhydrous acetonitrile (20 mL), and potassium iodide (8 mg, 0.05 mmol) and DIPEA (1 mL, 25.74 mmol) were added. The mixture was subjected to nitrogen replacement, and then reacted overnight at 60°C. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and purified by column chromatography (DCM : MeOH = 1 : 0-0 : 1) to obtain compound 3 (196 mg, 52.5%).

$^1H$ NMR (400 MHz, DMSO-$d_6$) δ 12.63 (s, 1H), 8.99 - 8.92 (m, 1H), 7.90 - 7.85 (m, 1H), 7.72 - 7.56 (m, 2H), 7.44 - 7.36 (m, 1H), 4.41 (s, 2H), 4.32 - 4.24 (m, 1H), 3.84 - 3.78 (m, 4H), 3.54 - 3.48 (m, 4H), 2.93 - 2.81 (m, 4H), 2.45 (s, 3H).

LC-MS (ESI): m/z = 505.2 $[M+H]^+$.

**Example 4**

6-fluoro-5-(4-((5-fluoro-2-methyl-3-oxo-3,4-dihydroquinoxalin-6-yl)methyl)piperazin-1-yl)-N-((1R,2S)-2-fluorocyclopropyl)picolinamide (compound 4)

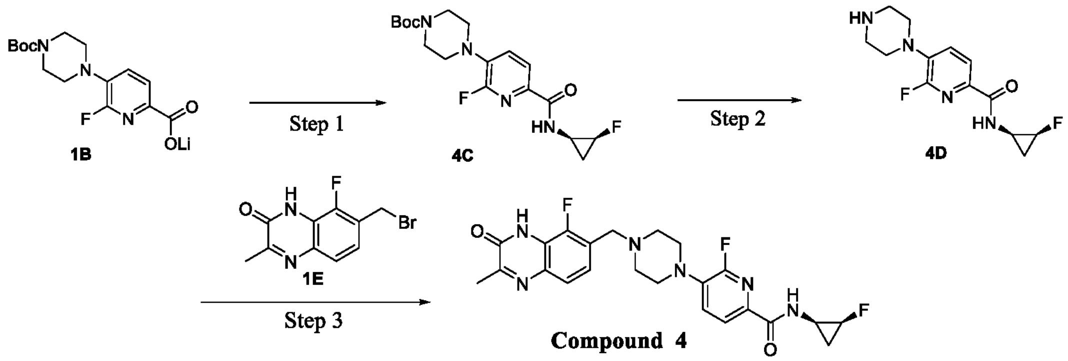


Step 1: Compound 1B (331 mg, 1 mmol) was dissolved in DMF (10 mL), and HATU (565 mg, 1.5 mmol) was added with stirring. The mixture was stirred at room temperature. After the solid was completely dissolved, DIEPA (2 mL) was added, and (1R,2S)-2-fluorocyclopropylamine p-toluenesulfonate (494 mg, 2 mmol) was finally added. The reaction was stirred at room temperature for 4 h. After the reaction was completed as monitored by LCMS, ethyl acetate (50 mL) was added to the system. The mixture was washed with water (50 mL × 4). The organic phase was collected, dried over anhydrous sodium sulphate, filtered and concentrated under reduced pressure, and then the residue was separated by silica gel column chromatography (PE : EA = 1 : 0-1 : 1) to obtain the title compound 4C (294 mg, 76.8%).

LC-MS (ESI): m/z = 383.2 $[M+H]^+$.

Step 2: 4C (294 mg, 0.76 mmol) was dissolved in methanol (10 mL), and a solution of hydrogen chloride in dioxane (4 mL, 4 M) was added. The mixture was reacted at room temperature for 4 hours and spun to dryness to obtain the title compound 4D (241 mg, crude).

LC-MS (ESI): m/z = 283.2 $[M+H]^+$.

Step 3: 1E (200 mg, 0.74 mmol) and 4D (241 mg) were dissolved in anhydrous acetonitrile (20 mL), and potassium iodide (8 mg, 0.05 mmol) and DIPEA (1 mL, 25.74 mmol) were added. The mixture was subjected to nitrogen replacement, and then reacted overnight at 60°C. Upon complete depletion of raw materials monitored by LCMS, the

system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by column chromatography (DCM : MeOH = 1 : 0-0 : 1) to obtain compound 4 (197 mg, 56.3%).

$^1$H NMR (400 MHz, DMSO-$d_6$) δ 12.19 (s, 1H), 8.36 - 8.31 (m, 1H), 7.88 - 7.83 (m, 1H), 7.63 - 7.45 (m, 2H), 7.32 - 7.25 (m, 1H), 4.87 - 4.61 (m, 1H), 3.70 (s, 2H), 3.23 - 3.13 (m, 4H), 2.94 - 2.72 (m, 1H), 2.64 - 2.54 (m, 4H), 2.42 (s, 3H), 1.37 - 1.23 (m, 1H), 1.14 - 1.00 (m, 1H).

LC-MS (ESI): m/z = 473.2 [M+H]$^+$.

## Example 5

6-fluoro-5-(4-((5-fluoro-2-methyl-3-oxo-3,4-dihydroquinoxalin-6-yl)methyl)piperazin-1-yl)-N-((1S,2R)-2-fluorocyclopropyl)picolinamide (compound 5)

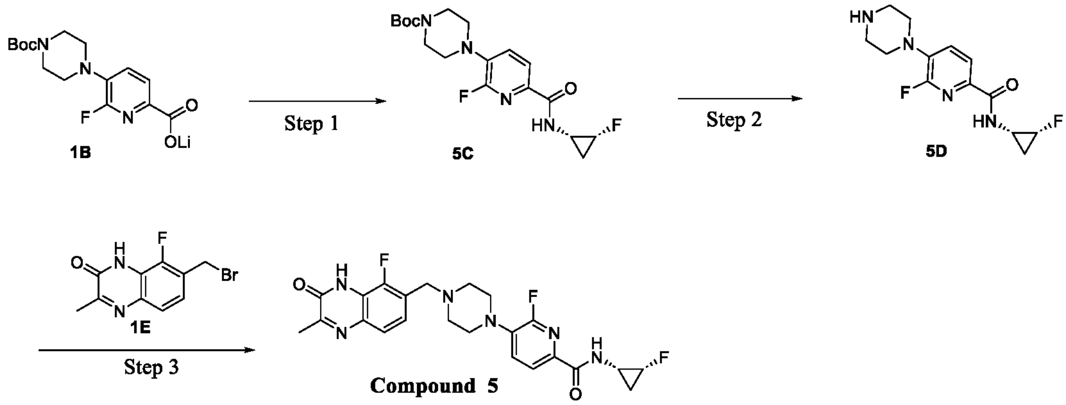


1B 5C 5D 1E Compound 5

Step 1: Compound 1B (331 mg, 1 mmol) was dissolved in DMF (10 mL), and HATU (565 mg, 1.5 mmol) was added with stirring. The mixture was stirred at room temperature. After the solid was completely dissolved, DIEPA (2 mL) was added, and (1S,2R)-2-fluorocyclopropylamine (150 mg, 2 mmol) was finally added. The reaction was stirred at room temperature for 4 h. After the reaction was completed as monitored by LCMS, ethyl acetate (50 mL) was added to the system. The mixture was washed with water (50 mL × 4). The organic phase was collected, dried over anhydrous sodium sulphate, filtered and concentrated under reduced pressure, and then the residue was separated by silica gel column chromatography (PE: EA = 1 : 0-1 : 1) to obtain the title compound 5C (324 mg, 84.6%).

LC-MS (ESI): m/z = 383.2 [M+H]$^+$.

Step 2: 5C (324 mg, 0.84 mmol) was dissolved in methanol (10 mL), and a solution of hydrogen chloride in dioxane (4 mL, 4 M) was added. The mixture was reacted at room temperature for 4 hours and spun to dryness to obtain the title compound 5D (292 mg, crude).

LC-MS (ESI): m/z = 283.2 [M+H]$^+$.

Step 3: 1E (200 mg, 0.74 mmol) and 5D (292 mg) were dissolved in anhydrous acetonitrile (20 mL), and potassium iodide (8 mg, 0.05 mmol) and DIPEA (1 mL, 25.74 mmol) were added. The mixture was subjected to nitrogen replacement, and then reacted overnight at 60°C. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by column chromatography (DCM : MeOH = 1 : 0-0 : 1) to obtain compound 5 (197 mg, 56.3%).

$^1$H NMR (400 MHz, DMSO-$d_6$) δ 12.44 (s, 1H), 8.36 - 8.31 (m, 1H), 7.88 - 7.83 (m, 1H), 7.59 - 7.49 (m, 2H), 7.32 - 7.26 (m, 1H), 4.86 - 4.63 (m, 1H), 3.70 (s, 2H), 3.22 - 3.13 (m, 4H), 2.88 - 2.76 (m, 1H), 2.63 - 2.56 (m, 4H), 2.42 (s, 3H), 1.38 - 1.24 (m, 1H), 1.14 - 1.00 (m, 1H).

LC-MS (ESI): m/z = 473.2 [M+H]$^+$.

**Example 6**

N-(cyclopropylmethyl)-6-fluoro-5-(4-((2-methyl-3-oxo-3,4-dihydroquinoxalin-6-yl)methyl)piperazin-1-yl)picolinamide (compound 6)

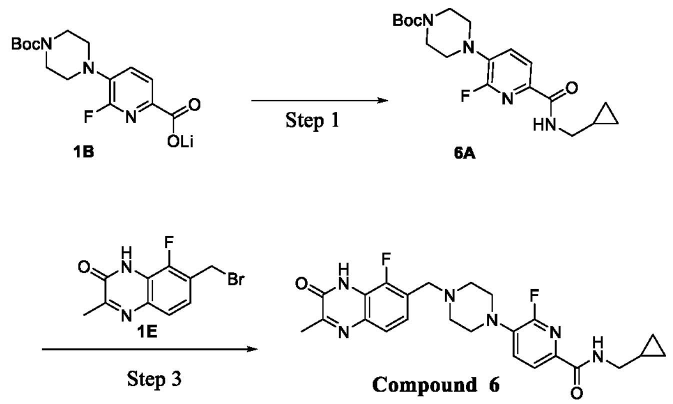


Step 1: 1B (626 mg, 1.89 mmol), cyclopropylmethylamine (0.16 g, 2.25 mmol), DIPEA (3.13 mL, 18.9 mmol) and HATU (1.44 g, 3.78 mmol) were dissolved in DMF (20 mL), and the mixture was reacted overnight at room temperature. Upon depletion of raw materials, the system was poured into water (100 mL), and a large number of solids were precipitated. The solids were collected by suction filtration and dried to obtain compound 6A (630 mg, 88%).

LCMS m/z = 379.1 $[M+1]^+$.

Step 2: 6A was dissolved in a solution of hydrogen chloride in dioxane (10 mL, 4N), and the mixture was reacted overnight at room temperature. The system was concentrated to obtain 6B as a crude hydrochloride, which was directly used in the next step.

LCMS m/z = 279.1 $[M+1]^+$.

Step 3: 6B (crude), 1E (0.36 g, 1.33 mmol), DIPEA (2.2 mL, 12.63 mmol) and potassium iodide (22 mg, 0.13 mmol) were dissolved in a mixed solvent of DMF (2 mL) and acetonitrile (20 mL), and the mixture was warmed to 60°C and reacted overnight. The acetonitrile in the system was removed by concentration, and the residue was poured into water (20 mL). A large number of solids were precipitated, and then subjected to suction filtration. The filter cake was dried and then purified by preparative HPLC (liquid phase preparation conditions: C18 reverse-phase preparative column, mobile phase: deionized water containing 0.1% ammonia water (A) and acetonitrile (B), gradient elution, mobile phase B = 5%-55%, elution time: 16 min, flow rate: 12 mL/min, and column temperature: 30°C) to obtain compound 6 (121 mg, 19%).

LCMS m/z =469.7 $[M+1]^+$.

$^1H$ NMR (400 MHz, CDCl3) δ 9.43 (s, 1H), 8.02 - 7.96 (m, 1H), 7.60 - 7.56 (m, 2H), 7.37 - 7.27 (m, 2H), 3.76 (s, 2H), 3.38 - 3.16 (m, 6H), 2.70 (s, 4H), 2.61 (s, 3H), 1.14 - 0.98 (m, 1H), 0.58 - 0.49 (m, 2H), 0.30 - 0.26 (m, 2H).

**Example 7:**

N-cyclopropyl-2-fluoro-1'-((5-fluoro-2-methyl-3-oxo-3,4-dihydroquinoxalin-6-yl)methyl)-1',2',3',6'-tetrahydro-[3,4'-bipyridine]-6-carboxamide (compound 7)

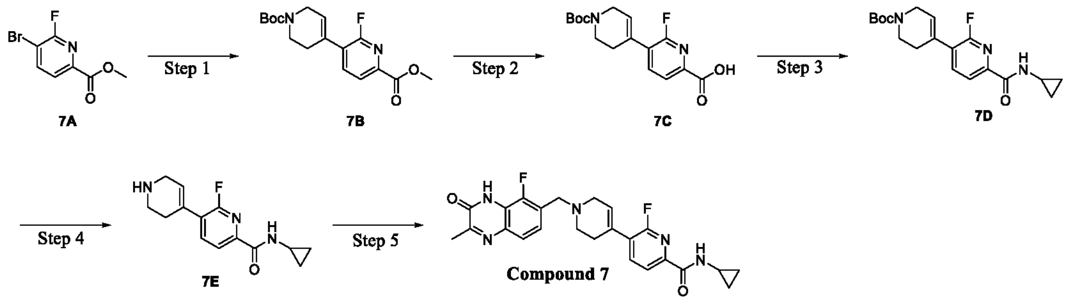

Step 1: 7A (5.20 g, 22.22 mmol), N-Boc-1,2,5,6-tetrahydropyridine-4-boronic acid pinacol ester (8.24 g, 26.66 mmol) and potassium carbonate (6.14 g, 44.44 mmol) were successively added to an N,N-dimethylformamide (100 mL) solution, and the mixture was subjected to nitrogen replacement three times. Then, chloro(2-dicyclohexylphosphino-2',6'-di-isopropoxy-1,1'-biphenyl)(2-amino-1,1'-biphenyl-2-yl)palladium (II) (0.86 g, 1.11 mmol) and water (1.00 g, 55.55 mmol) were added, and the mixture was subjected to nitrogen replacement again three times, warmed to 110°C and reacted for 4 hours. After the reaction was completed, the reaction liquid was cooled to room temperature. Ethyl acetate (200 mL) was added, and the mixture was stirred for 15 minutes and filtered. The filtrate was washed with water (200 mL × 3), the organic phase was dried over anhydrous sodium sulphate and then concentrated to dryness. The residue was purified by silica gel column (dichloromethane : methanol = 50 : 1) to obtain compound 7B (7.20 g, yield: 96.33%).

LC-MS (ESI): m/z = 337.2 $[M+H]^+$.

Step 2: Intermediate 7B (4.00 g, 11.89 mmol) was added to a toluene (20 mL) solution, and a 20% aqueous lithium hydroxide solution (10 mL) and methanol (2 mL) were added. The mixture was stirred overnight at 25°C. After the reaction was completed, ethyl acetate (200 mL) and water (200 mL) were added, and the liquid separation was conducted. The aqueous phase was collected, adjusted to pH 3-4 with 1 mol/L aqueous hydrochloric acid solution and extracted with ethyl acetate (200 mL×3). The organic phases were combined, dried over anhydrous sodium sulphate and then concentrated to dryness to obtain intermediate 7C (3.70 g, yield 96.54%).

LC-MS (ESI): m/z = 323.1 $[M+H]^+$.

Step 3: Intermediate 7C (1.50 g, 4.65 mmol) and N,N,N',N'-tetramethylchlorourea hexafluorophosphate (2.61 g, 9.30 mmol) were added to a dichloromethane (50 mL) solution, and N-methylimidazole (0.8 g, 9.77 mmol) was slowly added. The mixture was stirred for 15 min. Cyclopropylamine (0.32 g, 5.58 mmol) was then added, and the mixture was reacted at 25°C for 3 hours. After the reaction was completed, the reaction liquid was washed with water (50 mL × 3), the organic phase was dried over anhydrous sodium sulphate and concentrated to dryness, and the residue was separated and purified by silica gel column (dichloromethane : methanol = 100%-85%), to obtain intermediate 7D (1.60 g, yield: 95.21%).

LC-MS (ESI): m/z = 362.2 $[M+H]^+$.

Step 4: Intermediate 7D (1.60 g, 4.43 mmol) was added to a solution of hydrogen chloride in dioxane (4.0 mol/L, 20 mL), and the mixture was reacted at room temperature for 5 hours. The reaction liquid was concentrated to dryness, and an isopropanol (5 mL) solution and ethyl acetate (20 mL) solution were added. The mixture was stirred for 1 hour and filtered to obtain intermediate 7E (1.35 g, yield: 91.19%).

LC-MS (ESI): m/z = 262.1 $[M+H]^+$.

Step 5: Compound 7E (180 mg, 0.54 mmol) and 1E (150 mg, 0.49 mmol) were added to a mixed solution of acetonitrile (20 mL) and N,N-dimethylformamide (5 mL), potassium iodide (81 mg, 0.49 mmol) and N,N-diisopropylethylamine (1.00 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to obtain the title compound 7 (50 mg, yield: 22.60%, retention time: about 2.821 min).

LC-MS (ESI): m/z = 452.20 $[M+H]^+$.

$^1H$ NMR (400 MHz, DMSO-$d_6$) δ 12.43 (s, 1H), 8.57 (d, 1H), 8.06 (d, 1H), 7.90 (d, 1H), 7.51 (d, 1H), 7.29 (t, 1H), 6.23 (s, 1H), 3.75 (s, 2H), 3.17 (d, 2H), 2.93 - 2.41 (m, 8H), 0.74 - 0.61 (m, 4H).

**Example 8:**

2-fluoro-1'-((5-fluoro-2-methyl-3-oxo-3,4-dihydroquinoxalin-6-yl)methyl)-N-methyl-1',2',3',6'-tetrahydro-[3,4'-bipyridine]-6-carboxamide (compound 8)

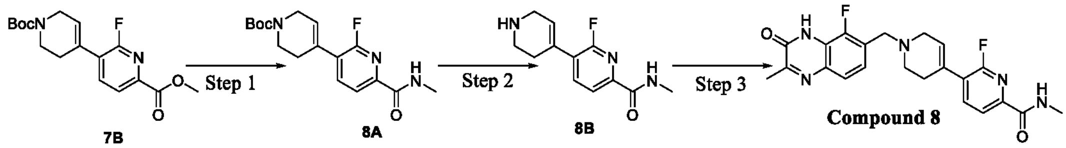


7B 8A 8B Compound 8

Step 1: Intermediate 7B (1.20 g, 3.57 mmol) was added to an acetonitrile (10 mL) solution, and then a 40% methylamine aqueous solution (10 mL) was added. The mixture was stirred at room temperature for 2 hours. After the reaction was completed, the reaction liquid was concentrated to dryness, and the residue was separated and purified by

silica gel column (ethyl acetate:petroleum ether = 0-100%) to obtain the title compound 8A (1.00 g, 83.52%).

LC-MS (ESI): m/z = 336.20 [M+H]$^+$.

Step 2: Intermediate 8A (1.00 g, 2.98 mmol) was added to a solution of hydrogen chloride in dioxane (4.0 mol/L, 20 mL), and the mixture was reacted at room temperature for 5 hours. The reaction liquid was concentrated to dryness, and an isopropanol (5 mL) solution and ethyl acetate (20 mL) solution were added. The mixture was stirred for 1 hour and filtered to obtain intermediate 8B (700 mg, yield: 76.23%).

LC-MS (ESI): m/z = 236.20 [M+H]$^+$.

Step 3: Compound 8B (170 mg, 0.54 mmol) and 1E (150 mg, 0.49 mmol) were added to a mixed solution of acetonitrile (20 mL) and N,N-dimethylformamide (5 mL), potassium iodide (81 mg, 0.49 mmol) and N,N-diisopropylethylamine (1.00 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to obtain the title compound 8 (165 mg, yield: 79.15%, retention time: about 2.592 min).

LC-MS (ESI): m/z = 426.20 [M+H]$^+$.

$^1$H NMR (400 MHz, DMSO-$d_6$) δ 12.43 (s, 1H), 8.60 (d, 1H), 8.09 - 8.04 (m, 1H), 7.91 (s, 1H), 7.53 - 7.50 (m, 1H), 7.33 - 7.29 (m, 1H), 6.25 - 6.23 (m, 1H), 3.81 - 3.70 (m, 2H), 3.17 (q, 2H), 2.79 (d, 3H), 2.70 - 2.42 (m, 7H).

**Example 9:**

2-fluoro-1'-((5-fluoro-2-methyl-3-oxo-3,4-dihydroquinoxalin-6-yl)methyl)-N-(1-methyl-1H-pyrazol-4-yl)-1',2',3',6'-tetrahydro-[3,4'-bipyridine]-6-carboxamide (compound 9)

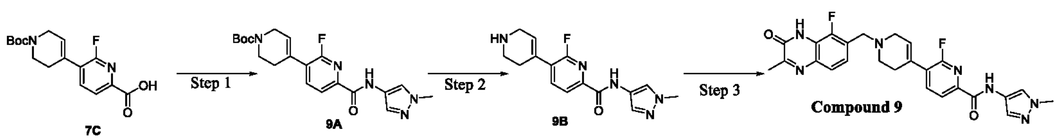


Step 1: Intermediate 7C (1.00 g, 3.10 mmol) and N,N,N',N'-tetramethylchlorourea hexafluorophosphate (1.04 g, 3.72 mmol) were added to a dichloromethane (50 mL) solution, and N-methylimidazole (0.51 g, 6.15 mmol) was slowly added. The mixture was stirred for 15 min. 1-Methyl-1H-pyrazol-4-amine (0.36 g, 3.71 mmol) was then added, and the mixture was reacted at 25°C for 3 hours. After the reaction was completed, the reaction liquid was washed with water (50 mL × 3), the organic phase was dried over anhydrous sodium sulphate and concentrated to dryness, and the residue was separated and purified by silica gel column (dichloromethane:methanol = 100%-85%), to obtain intermediate 9A (1.20 g, yield: 96.43%).

LC-MS (ESI): m/z = 402.30 [M+H]$^+$.

Step 2: Intermediate 9A (1.20 g, 2.99 mmol) was added to a solution of hydrogen chloride in dioxane (4.0 mol/L, 20 mL), and the mixture was reacted at room temperature for 5 hours. The reaction liquid was concentrated to dryness, and an isopropanol (5 mL) solution and ethyl acetate (20 mL) solution were added. The mixture was stirred for 1 hour and filtered to obtain intermediate 9B (0.90 g, yield: 80.43%).

LC-MS (ESI): m/z = 302.40 [M+H]$^+$.

Step 3: Compound 9B (200 mg, 0.54 mmol) and 1E (150 mg, 0.49 mmol) were added to a mixed solution of acetonitrile (20 mL) and N,N-dimethylformamide (5 mL), potassium iodide (81 mg, 0.49 mmol) and N,N-diisopropylethylamine (1.00 mL) were then added, and the mixture was warmed to 80°C and reacted for 2 hours. After the reaction was completed, the reaction liquid was concentrated to dryness, spun to dryness, and then separated and purified by liquid phase preparative column (liquid phase preparative conditions: C18 reverse-phase preparative column, mobile phase: deionized water containing 0.1% ammonia water (A) and acetonitrile (B), gradient elution, mobile phase B = 5%-50%, elution time: 15 min, flow rate: 12 mL/min, and column temperature: 30°C), to obtain the title compound 9 (194 mg, yield: 80.55%, retention time: about 2.752 min).

LC-MS (ESI): m/z = 492.20 [M+H]$^+$.

$^1$H NMR (400 MHz, DMSO-$d_6$) δ 12.45 (s, 1H), 10.73 (s, 1H), 8.15 - 8.04 (m, 2H), 8.00 (s, 1H), 7.72 (s, 1H), 7.52 (d, 1H), 7.33 - 7.29 (m, 1H), 6.28 - 6.26 (m, 1H), 3.79 (d, 5H), 3.19 (d, 2H), 2.71 - 2.42 (m, 7H).

**Example 12**

N-cyclopropyl-5-(4-((2-(difluoromethyl)-5-fluoro-3-oxo-3,4-dihydroquinoxalin-6-yl)methyl)piperazin-1-yl)-6-fluoropicolinamide (compound 12)

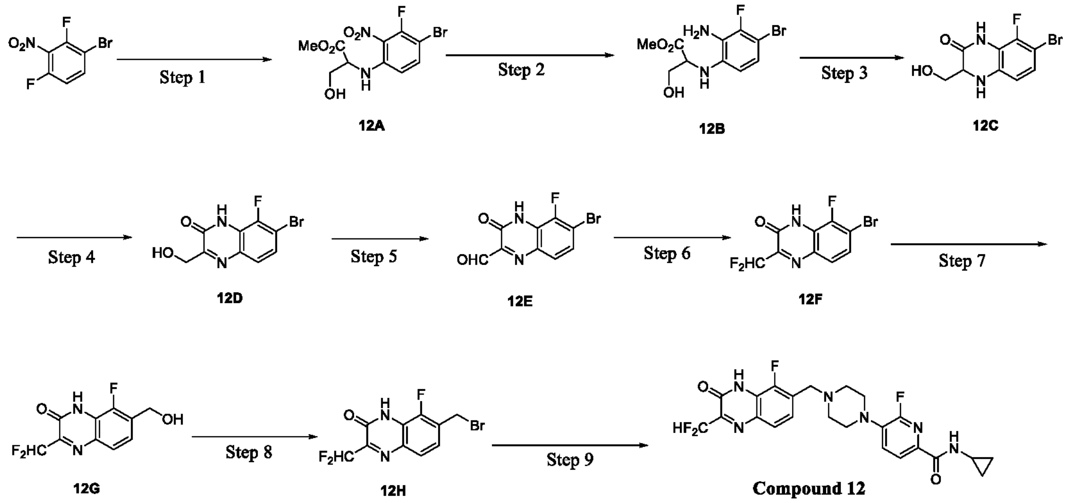


12A 12B 12C 12D 12E 12F 12G 12H Compound 12

Step 1: 1-Bromo-2,4-difluoro-3-nitrobenzene (23.8 g, 0.1 mol) was dissolved in 1,4-dioxane (240 mL), and serine methyl ester hydrochloride (16.5 g, 0.11 mol) and DIPEA (40 mL) were added with stirring. The mixture was reacted overnight at 40°C. After the reaction was completed as monitored by LCMS, the reaction liquid was spun to dryness to remove the solvent and separated by silica gel column chromatography (PE : EA = 5 : 1-1 : 1) to obtain the target product 12A (19.4 g, 57.55%).

LC-MS (ESI): m/z = 337.2, 339.1 $[M+H]^+$.

Step 2: 12A (19.4 g, 57.55 mmol) was dissolved in anhydrous methanol (250 mL) and water (25 mL), and ammonium chloride (32.1 g, 600 mmol) and zinc powder (39 g, 600 mmol) were added. The mixture was reacted at room temperature for 2 h. Upon complete depletion of raw materials monitored by LCMS, the zinc powder was removed by filtration, and the filtrate was concentrated, diluted with ethyl acetate (200 mL), washed with water (200 mL) and extracted again with ethyl acetate (100 mL). The organic phases were combined, washed with saturated brine, dried over anhydrous sodium sulphate and concentrated under reduced pressure to obtain the target compound 12B (14.5 g, 82.06%).

LC-MS (ESI): m/z = 307.2, 309.1 $[M+H]^+$.

Step 3: 12B (14.5 g, 47.2 mmol) was dissolved in anhydrous methanol (100 mL), and a solution of hydrogen chloride in dioxane (4 mL, 4 M) was added. The mixture was reacted at room temperature for 2 hours. Upon complete depletion of raw materials monitored by LCMS, the mixture was concentrated to remove some of the solvents and filtered to obtain the target compound 12C (10.7 g, 82.3%).

LC-MS (ESI): m/z = 275.2, 277.2 $[M+H]^+$.

Step 4: 12C (10.7 g, 38.9 mmol) was dispersed in dichloromethane (200 mL), and DDQ (9.72 g, 42.8 mmol) was added at room temperature. The mixture was reacted at room temperature overnight. Upon complete depletion of raw materials monitored by LCMS, the mixture was spun to remove the solvent. A saturated aqueous sodium bicarbonate solution was added with stirring. When no more bubbles emerged, the mixture was filtered. The filter cake was washed with water and dried to obtain the target compound 12D (8.4 g, 79.09%).

LC-MS (ESI): m/z = 273.2, 275.2 $[M+H]^+$.

Step 5: 12D (8.4 g, 30.76 mmol) was dispersed in dichloromethane (150 mL), and Dess-Martin periodinane (25.8 g, 61 mmol) was added at room temperature. The mixture was reacted at room temperature for 6 h. Upon complete depletion of raw materials monitored by LCMS, the mixture was spun to remove the solvent. A saturated aqueous sodium bicarbonate solution was added with stirring, and the mixture was filtered. The filter cake was washed with water and dried to obtain the target compound 12E (7.1 g, 86.6%).

LC-MS (ESI): m/z = 271.2, 273.2 $[M+H]^+$.

Step 6: 12E (7.1 g, 26.19 mmol) was dispersed in 1,4-dioxane (100 mL), and DAST (21.7 g, 135 mmol) was slowly added at room temperature. The mixture was reacted overnight at room temperature. Upon complete depletion of raw materials monitored by LCMS, water (200 mL) was added with stirring in an ice bath. The mixture was extracted with ethyl acetate (200 mL × 3). The organic phases were combined, washed with saturated brine and dried over anhydrous sodium

sulphate. Then, the residue was separated by silica gel column chromatography (PE : EA = 10 : 1-3 : 1) to obtain the target compound 12F (2.1 g, 27.3%).

LC-MS (ESI): m/z = 291.2, 293.2 $[M-H]^+$.

Step 7: 12F (2.1 g, 7.2 mmol) was dissolved in 1,4-dioxane (40 mL). (Tributylstannyl)methanol (2.54 g, 7.92 mmol) and X-phos Pd G2 (556 mg, 0.72 mmol) were added with stirring, and the mixture was reacted overnight at 80°C. Upon complete depletion of raw materials monitored by LCMS, the residue was separated by silica gel column chromatography (PE : EA = 1 : 1-0 : 1) to obtain the target compound 12G (1.65 g, 94.6%).

LC-MS (ESI): m/z = 245.2 $[M+H]^+$.

Step 8: Compound 12G (488 mg, 2.00 mmol) was dissolved in dichloromethane, and triphenylphosphine (1.57 g, 6.0 mmol) and carbon tetrabromide (1.98 g, 6.0 mmol) were added with stirring at 0°C. The reaction was stirred at room temperature for 2 h. After the reaction was completed as monitored by LCMS, the residue was separated by silica gel column chromatography (PE : EA = 5 : 1-1 : 1) to obtain the target compound 12H (512 mg, 83.4%).

LC-MS (ESI): m/z = 307.2, 309.2 $[M+H]^+$.

Step 9: 1D (264 mg, 1 mmol) and 12H (300 mg, 0.97 mmol) were dissolved in anhydrous acetonitrile (20 mL), and potassium iodide (8 mg, 0.05 mmol) and DIPEA (1 mL) were added. The mixture was subjected to nitrogen replacement, and then reacted at 60°C for 4 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and subjected to column chromatography (DCM : MeOH = 1 : 0-0 : 1) to obtain compound 12 (334 mg, 70.21%).

$^1H$ NMR (400 MHz, DMSO-$d_6$) δ 13.17 (s, 1H), 8.39 - 8.32 (m, 1H), 7.89 - 7.84 (m, 1H), 7.85 - 7.78 (m, 1H), 7.69 - 7.60 (m, 1H), 7.54 - 7.47 (m, 1H), 7.27 - 6.94 (m, 1H), 3.91 (s, 2H), 3.41 - 3.32 (m, 4H), 2.70 - 2.62 (m, 4H), 2.90 - 2.81 (m, 1H), 0.73 - 0.53 (m, 4H).

LC-MS (ESI): m/z= 491.2 $[M+H]^+$.

## Example 13

N-cyclopropyl-6-fluoro-5-(4-((5-fluoro-3-oxo-2-(trifluoromethyl)-3,4-dihydroquinoxalin-6-yl)methyl)piperazin-1-yl)picolinamide (compound 13)

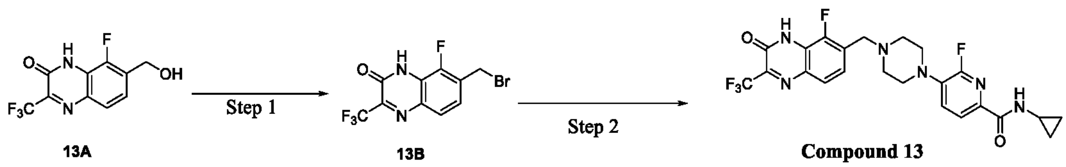


13A 13B Compound 13

Step 1: Compound 13A (synthesized according to patent US 2022/0009901 A1) (524 mg, 2.00 mmol) was dissolved in dichloromethane, and triphenylphosphine (1.57 g, 6.0 mmol) and carbon tetrabromide (1.98 g, 6.0 mmol) were added with stirring at 0°C. The reaction was stirred at room temperature for 2 h. After the reaction was completed as monitored by LCMS, the residue was separated by silica gel column chromatography (PE : EA = 5 : 1-1 : 1) to obtain the target compound 13B (552 mg, 84.9%).

LC-MS (ESI): m/z = 325.2, 327.2 $[M+H]^+$.

Step 2: 1D (264 mg, 1 mmol) and 13B (300 mg, 0.92 mmol) were dissolved in anhydrous acetonitrile (20 mL), and potassium iodide (8 mg, 0.05 mmol) and DIPEA (1 mL) were added. The mixture was subjected to nitrogen replacement, and then reacted at 60°C for 4 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by column chromatography (DCM : MeOH = 1 : 0-0 : 1) to obtain compound 13 (319 mg, 68.3%).

$^1H$ NMR (400 MHz, DMSO-$d_6$) δ 13.17 (s, 1H), 8.34 - 8.28 (m, 1H), 7.86 - 7.81 (m, 1H), 7.74 - 7.69 (m, 1H), 7.59 - 7.52 (m, 1H), 7.44 - 7.37 (m, 1H), 3.75 (s, 2H), 3.21 - 3.13 (m, 4H), 2.89 - 2.81 (m, 1H), 2.65 - 2.61 (m, 4H), 0.69 - 0.59 (m, 4H).

LC-MS (ESI): m/z= 509.2 $[M+H]^+$.

**Example 14**

N-(5-(4-((2-(difluoromethyl)-5-fluoro-3-oxo-3,4-dihydroquinoxalin-6-yl)methyl)piperazin-1-yl)-6-fluoropyridin-2-yl)-1-methyl-1H-pyrazole-4-carboxamide (compound 14)

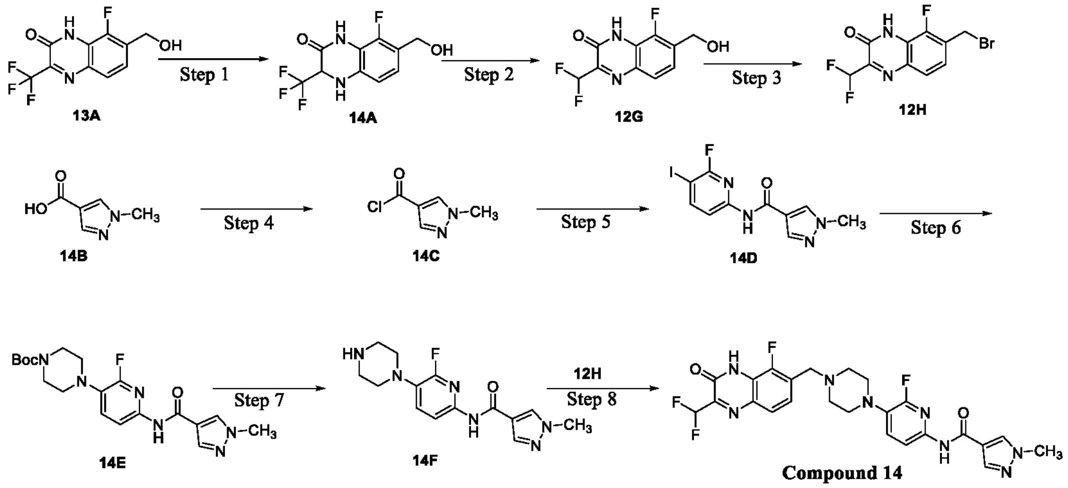


Step 1: Compound 13A (synthesized according to patent US 2022/0009901 A1) (1.3 g, 4.96 mmol) was dissolved in a mixed solution of anhydrous ethanol (26 mL) and water (2 mL). With stirring at room temperature, solid ammonium chloride (3.18 g, 59.47 mmol) and zinc powder (3.24 g, 49.6 mmol) were successively added. After the addition was completed, the mixture was reacted at room temperature for 90 min, and the reaction was monitored by LCMS. After the reaction was completed, a saturated aqueous ammonium chloride solution (30 mL) was added to the reaction liquid, and the mixture was filtered through celite. The filter cake was washed with ethyl acetate (100 mL). Ethyl acetate (100 mL) was added to the filtrate, and the aqueous phase was separated. The organic phase was washed with saturated brine (150 mL), dried over anhydrous sodium sulphate and filtered to obtain the target compound 14A (1.30 g, yield: 99.21%).

LCMS m/z = 265.2 $[M+1]^+$.

$^1$H NMR (400 MHz, DMSO-$d_6$) δ 10.96 (s, 1H), 7.16 (s, 1H), 6.88 - 6.84 (m, 1H), 6.63 - 6.61 (m, 1H), 5.01 - 4.98 (m, 1H), 4.87 - 4.80 (m, 1H), 4.39 - 4.40 (m, 2H).

Step 2: Compound 14A (1.0 g, 3.79 mmol) was dissolved in tetrahydrofuran (20 mL), and then an aqueous sodium hydroxide solution (25% (w/w), 5 mL) was added to the reaction liquid with stirring at room temperature. After the addition was completed, the mixture was heated at 50°C and reacted for 2 h. The reaction was monitored by TLC (ethyl acetate : petroleum ether = 3 : 2). After the reaction was completed, ethyl acetate (40 mL) and water (20 mL) were added to the reaction liquid, and the organic phase was separated. The aqueous phase was adjusted to pH = 6 with hydrochloric acid (6N hydrochloric acid). A large number of solids were precipitated and then filtered. The filter cake was dried to obtain the target compound 12G (0.7 g, yield: 75.64%).

LCMS m/z = 245.2 $[M+1]^+$.

$^1$H NMR (400 MHz, DMSO-$d_6$) δ 12.97 (s, 1H), 7.73 - 7.70 (m, 1H), 7.46 - 7.42 (m, 1H), 7.19 - 6.93 (m, 1H), 5.52 - 5.49 (m, 1H), 4.69 - 4.67 (m, 2H).

$^{19}$FNMR (400 MHz, DMSO-$d_6$) δ -122.41(s), -123.46(s).

Step 3: Compound 12G (488 mg, 2.0 mmol) was dissolved in dichloromethane (20 mL) and cooled to 0°C. Then, triphenylphosphine (1.57 g, 6.0 mmol) and carbon tetrabromide (1.98 g, 6.0 mmol) were successively added. After the addition was completed, the ice bath was removed, and the mixture was naturally warmed to room temperature and stirred at room temperature for 2 h. The reaction was monitored by LCMS. After the reaction was completed, the reaction liquid was directly concentrated and then purified by column chromatography (eluent: PE : EA = 5 : 1-1 : 1) to obtain the target compound 12H (530 mg, yield: 86.32%).

LC-MS (ESI): m/z = 307.2, 309.2 $[M+H]^+$.

Step 4: Compound 14B (2.52 g, 19.98 mmol) was dissolved in dichloromethane (50 mL), and then thionyl chloride (2.5 mL) was added. After the addition was completed, the mixture was heated to reflux and reacted for 3 h. After 3 h of the reaction, the reaction liquid was concentrated to obtain the target compound 14C (2.89 g, crude), which was directly used in the next reaction.

Step 5: 5-iodo-6-fluoro-pyridin-2-amine (4.76 g, 19.99 mmol) was weighed and dissolved in dichloromethane (80

mL), and then pyridine (4.74 g, 59.97 mmol) was added. The reaction liquid was cooled to 0°C. The crude compound 14C (2.89 g, 19.99 mmol) was weighed and dissolved in dichloromethane (20 mL), and then a solution of compound 14C in dichloromethane was slowly added to the above reaction liquid. After the addition was completed, the mixture was warmed to room temperature and stirred for another 1 h. After the reaction was completed, dichloromethane (50 mL) and water (100 mL) were added to the reaction liquid. The mixture was stirred for 2 min, and the aqueous phase was separated. The organic phase was dried over anhydrous sodium sulphate, filtered and concentrated to precipitate solids. Then, n-hexane was added, and the mixture was stirred for crystallization, filtered and dried to obtain the target compound 14D (3.1 g, yield: 44.81%).

LC-MS (ESI): m/z = 347.2 $[M+H]^+$.

$^1H$ NMR (400 MHz, DMSO-$d_6$) δ 10.76 (s, 1H), 8.44 (s, 1H), 8.34 - 8.30 (m, 1H), 8.12 (s, 1H), 7.96 - 7.94 (m, 1H), 3.88 (s, 3H).

Step 6: Under nitrogen protection, compound 14D (1.35 g, 3.90 mmol) and N-Boc piperazine (1.45 g, 7.80 mmol) were successively weighed and added to 1,4-dioxane (30 mL). Then, caesium carbonate (3.81 g, 11.7 mmol), $Pd_2(dba)_3$ (0.71 g, 0.78 mmol) and RuPhos (0.73 g, 1.56 mmol) were added. After the addition was completed, the mixture was heated to 100°C and reacted for 3 h. After the reaction was completed, the reaction liquid was concentrated and purified by column chromatography (ethyl acetate : petroleum ether = 2 : 1) to obtain the target compound 14E (0.55 g, crude, yield: 34.87%), which was directly used in the next reaction.

LC-MS (ESI): m/z = 405.2 $[M+H]^+$.

Step 7: 14E (0.55 g, crude) was dissolved in methanol (10 mL), and a solution of hydrogen chloride in dioxane (4 mL, 4 M) was added. The mixture was reacted at room temperature for 4 hours and spun to dryness to obtain the title compound 14F (460 mg, crude). The crude was directly used in the next reaction.

LC-MS (ESI): m/z = 305.2 $[M+H]^+$.

Step 8: 14F (200 mg, crude) and 12H (100 mg, 0.33 mmol) were dissolved in anhydrous acetonitrile (20 mL), and DIPEA (1 mL, 5.74 mmol) was added. The mixture was subjected to nitrogen replacement, and then reacted at 60°C for 2 h. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 14 (26 mg, yield: 14.86%).

LC-MS (ESI): m/z = 531.2 $[M+H]^+$.

$^1H$ NMR (400 MHz, DMSO-$d_6$) δ 12.96 (s, 1H), 10.41 (s, 1H), 8.38 (s, 1H), 8.08 (s, 1H), 8.01 - 7.96 (m, 1H), 7.72 - 7.66 (m, 1H), 7.60 - 7.52 (m, 1H), 7.42 - 7.34 (m, 1H), 7.07 (t, 1H), 3.87 (s, 3H), 3.74 (s, 2H), 3.09 - 2.98 (m, 4H), 2.65 - 2.56 (m, 4H).

**Example 15**

5-(4-((2-(difluoromethyl)-5-fluoro-3-oxo-3,4-dihydroquinoxalin-6-yl)methyl)piperazin-1-yl)-6-fluoro-N-(1-methyl)-1H-pyrazol-4-yl)picolinamide (compound 15)

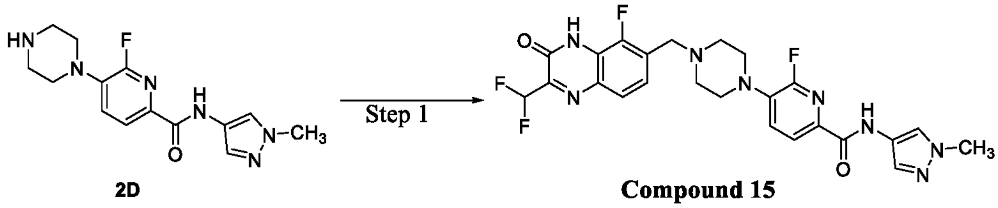


2D

Compound 15

Step 1: 2D (200 mg, crude) and 12H (100 mg, 0.33 mmol) were dissolved in anhydrous acetonitrile (20 mL), and DIPEA (1 mL, 5.74 mmol) was added. The mixture was subjected to nitrogen replacement, and then reacted at 60°C for 2 h. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 15 (37 mg, yield: 21.14%).

$^1H$ NMR (400 MHz, DMSO-$d_6$) δ 12.93 (s, 1H), 10.48 (s, 1H), 8.03 (s, 1H), 7.98 - 7.86 (m, 1H), 7.77 - 7.67 (m, 2H), 7.65 - 7.55 (m, 1H), 7.48 - 7.36 (m, 1H), 7.07 (t, 1H), 3.81 (s, 3H), 3.76 (s, 2H), 3.25 - 3.15 (m, 4H), 2.68 - 2.58 (m, 4H).

LC-MS (ESI): m/z= 531.2 $[M+H]^+$.

### Example 16

N-(6-fluoro-5-(4-((5-fluoro-2-methyl-3-oxo-3,4-dihydroquinoxalin-6-yl)methyl)piperazin-1-yl)pyridin-2-yl)-1-methyl-1H-pyrazole-4-carboxamide (compound 16)

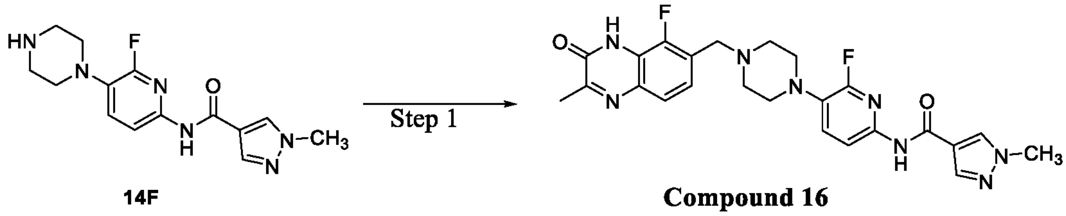


14F

Compound 16

Step 1: 14F (200 mg, crude) and 1E (100 mg, 0.37 mmol) were dissolved in anhydrous acetonitrile (20 mL), and DIPEA (1 mL, 2.45 mmol) was added. The mixture was subjected to nitrogen replacement, and then reacted at 60°C for 2 h. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 16 (37 mg, 21.14%).

$^1H$ NMR (400 MHz, DMSO-$d_6$) δ 12.38 (s, 1H), 10.41 (s, 1H), 8.38 (s, 1H), 8.08 (s, 1H), 8.01 - 7.93 (m, 1H), 7.59 - 7.47 (m, 2H), 7.34 - 7.27 (m, 1H), 3.87 (s, 3H), 3.69 (s, 2H), 3.06 - 2.97(m, 4H), 2.62 - 2.53 (m, 4H), 2.42 (s, 3H).

LC-MS (ESI): m/z = 495.2 $[M+H]^+$.

### Example 17

1-(difluoromethyl)-N-(5-(4-((2-(difluoroethyl(-5-fluoro-3-oxo-3,4-dihydroquinoxalin-6-yl)methyl)piperazin-1-yl)-6-fluoropyridin-2-yl)-1H-pyrazole-4-carboxamide (compound 17)

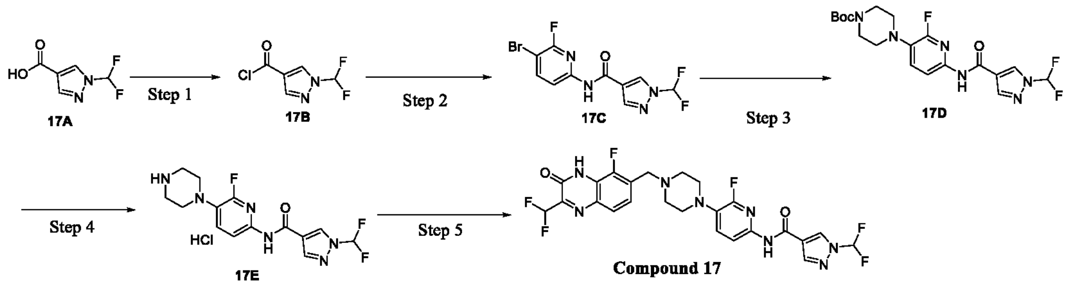


17A 17B 17C 17D 17E Compound 17

Step 1: Compound 17A (synthesized according to patent WO 2016051193 A1) (9.62 g, 59 mmol) was dissolved in DCM (60 mL), and thionyl chloride (10 mL) was added. The mixture was reacted under reflux for 2 h and stopped until the system was completely clarified. The system was concentrated to remove the solvent, so as to obtain the title compound 17B (10.6 g, crude), which was directly used in the next step without further purification.

Step 2: 5-bromo-6-fluoropyridin-2-amine (10.6 g, 55.49 mmol) and pyridine (14.2 g, 180 mmol) were successively added to a 500 mL eggplant-shaped bottle and dissolved in 200 mL of dichloromethane, and the mixture was cooled to 0°C. 100 mL of a solution of compound 17B (10.6 g, crude) in dichloromethane was slowly added dropwise. After the dropwise addition was completed, the mixture was reacted at room temperature for another 1 h. 200 ml of half-saturated brine was added, and the mixture was shaken thoroughly and left to stand for layer separation. The aqueous phase was separated, and some of the organic phases were concentrated until a large number of solids were precipitated. The solids were filtered, and the filter cake was washed with petroleum ether and dried under vacuum to obtain the title compound 17C (7.8 g, 41.95%).

LC-MS (ESI): m/z = 335.2, 337.2 $[M+H]^+$.

Step 3: Under nitrogen protection, compound 17C (7.8 g, 23 mmol) and N-Boc piperazine (6.34 g, 34 mmol) were successively weighed and added to 1,4-dioxane (100 mL). Then, caesium carbonate (15 g, 46 mmol), $Pd_2(dba)_3$ (4.2 g, 4.6 mmol) and Xphos (2.2 g, 4.6 mmol) were added. After the addition was completed, the mixture was heated to 100°C and

reacted overnight. After the reaction was completed, the reaction liquid was concentrated and purified by column chromatography (ethyl acetate : petroleum ether = 1 : 1) to obtain the target compound 17D (0.464 g, yield: 4.8%).

LC-MS (ESI): m/z= 441.2 $[M+H]^+$.

Step 4: 17D (0.464 g, 1.05 mmol) was dissolved in methanol (10 mL), and a solution of hydrogen chloride in dioxane (5 mL, 4 M) was added. The mixture was reacted at room temperature for 2 h and concentrated to obtain the title compound 17E (388 mg, crude).

LC-MS (ESI): m/z = 341.2 $[M+H]^+$.

Step 5: 12H (305 mg, 1 mmol) and 17E (388 mg, crude) were dissolved in anhydrous acetonitrile (20 mL), and DIPEA (0.5 mL) was added. The mixture was subjected to nitrogen replacement, and then reacted at 60°C for 3 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 17 (181 mg, 31.95%).

$^1$H NMR (400 MHz, DMSO-$d_6$) δ 13.00 (s, 1H), 10.76 (s, 1H), 8.95 (s, 1H), 8.37 (s, 1H), 8.02 - 7.96 (m, 1H), 8.04 - 7.73(m, 1H) 7.71 (d, 1H), 8.62 - 7.55 (m, 1H), 7.43 - 7.37 (m, 1H), 7.07 (t, 1H), 3.74 (s, 2H), 3.09 - 2.99 (m, 4H), 2.65 - 2.55 (m, 4H).

LC-MS (ESI): m/z=567.2 $[M+H]^+$.

### Example 18

N-(5-(4-((2-(difluoromethyl)-5-fluoro-3-oxo-3,4-dihydroquinoxalin-6-yl)methyl)piperazin-1-yl)-6-fluoropyridin-2-yl)-3-fluoro-1-methyl-1H-pyrazole-4-carboxamide (compound 18)

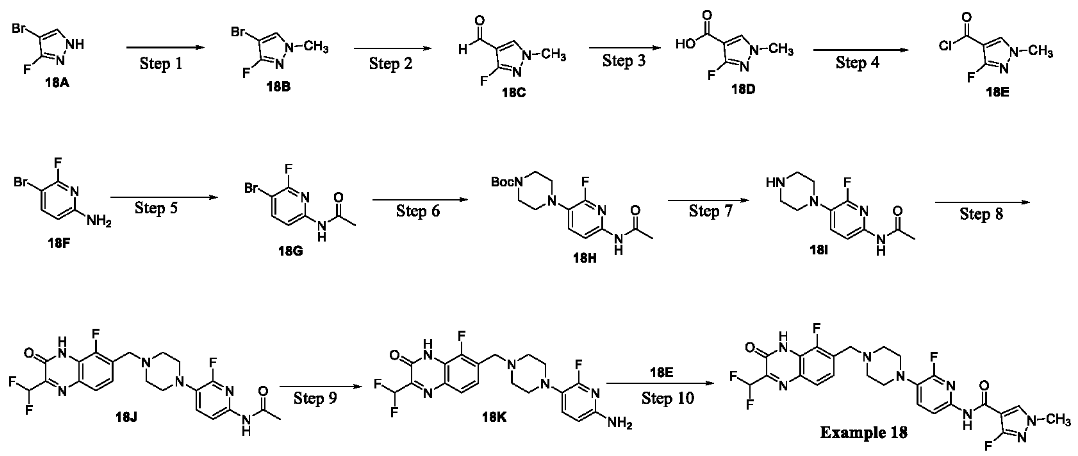


Step 1: Compound 18A (1 g, 6.1 mmol) was dissolved in THF (20 mL), and 500 mg (60%) of sodium hydride was added at 0°C. The mixture was stirred for 30 min. Iodomethane (0.6 mL, 9.6 mmol) was added, and the mixture was slowly warmed to room temperature and reacted for 2 h. The reaction was quenched by adding a saturated aqueous ammonium chloride solution and extracted with ethyl acetate. The organic phase was collected and concentrated, and the residue was separated by column chromatography (PE : EA = 20 : 1 to 5 : 1) to obtain compound 18B (601 mg, 56%).

$^1$H NMR (400 MHz, $CDCl_3$) δ 7.23 - 7.21 (m, 1H), 3.77 - 3.75 (m, 3H).

Step 2: Compound 18B (342 mg, 1.9 mmol) was dissolved in THF (5 mL), and the mixture was cooled to -78°C. n-Butyllithium (1.15 mL, 2.5 M in hexane) was added, and the mixture was stirred at -78°C for 15 min. DMF (0.3 mL, 3.9 mmol) was added, and the mixture was reacted at -78°C for 1 h. The system was quenched by adding ethyl acetate and water, and then extracted with ethyl acetate. The organic phase was collected and concentrated, and the residue was separated by column chromatography (PE : EA = 15 : 1 to 2 : 1) to obtain compound 18C (84 mg, 35%).

LC-MS (ESI): m/z = 129.1 $[M+H]^+$.

Step 3: Compound 18C (40 mg, 0.28 mmol) was dissolved in $KMnO_4$ solution (0.375 M). The mixture was warmed to 75°C, reacted for 1 h and cooled to room temperature. The system was adjusted to a basic pH by adding an aqueous KOH solution (10%), and solids appeared in the system. Suction filtration was performed to obtain a filtrate. The filtrate was adjusted to pH = 2 by adding hydrochloric acid. The system was extracted three times with ethyl acetate/-methanol = 10/1 (50 mL). The resulting organic phase was concentrated to obtain 30 mg of crude compound 18D.

LC-MS (ESI): m/z = 145.1 $[M+H]^+$.

Step 4: 5 mL of dichloromethane and 1 mL of thionyl chloride were added to the crude compound 18D, and under nitrogen atmosphere, the system was warmed to 50°C and reacted for 1 h. The reaction liquid was cooled to room temperature and concentrated until no solvent remained, and then used directly in the next reaction.

Step 5: Compound 18F (4 g, 21 mmol) was dissolved in dichloromethane (50 mL), and pyridine (2.5 mL, 32 mmol) and acetyl chloride (1.7 mL, 23 mmol) were added at room temperature. Upon complete depletion of raw materials monitored by TLC, the system was concentrated and slurried with ethyl acetate/petroleum ether = 1/15 (160 mL) to obtain compound 18G (4 g, 81.74%).

LC-MS (ESI): m/z= 233.1 $[M+H]^+$.

Step 6: 18G (4 g, 17 mmol), N-Boc-piperazine (3.8 g, 21 mmol), $Pd_2dba_3$ (1.6 g, 1.7 mmol), RuPhos (1.4 g, 3.2 mmol) and potassium tert-butoxide (4.8 g, 43 mmol) were added to a round-bottom flask, and 1,4-dioxane (100 mL) was added. The mixture was subjected to nitrogen replacement, heated to reflux, stirred overnight and cooled to room temperature. Silica gel was added. The mixture was concentrated to dryness, and the residue was separated by column chromatography (PE : EA = 5 : 1 to 1 : 3) to obtain compound 18H (1.1 g, 19%).

LC-MS (ESI): m/z = 339.1 $[M+H]^+$.

Step 7: Compound 18H (400 mg, 1.2 mmol) was dissolved in TFA/DCM = 1 : 4 (10 mL), and the mixture was reacted at room temperature until complete depletion of raw materials. Then, the system was directly concentrated to obtain compound 18I as a crude trifluoroacetate salt, which was directly used in the next step.

LC-MS (ESI): m/z = 239.2 $[M+H]^+$.

Step 8: Compound 18I (crude) and 12H (337 mg, 1.1 mmol) were dissolved in acetonitrile (15 mL), and DIPEA (3 mL) was added. The mixture was warmed to 60°C and reacted for 2 h, cooled to room temperature and then concentrated to dryness. The residue was separated by reverse phase column chromatography (water/acetonitrile = 1 : 1) to obtain compound 18J (160 mg, 27%).

LC-MS (ESI): m/z = 465.1 $[M+H]^+$.

Step 9: Compound 18J (60 mg, 0.13 mmol) was dissolved in a mixed solvent of concentrated hydrochloric acid/ethanol = 2 : 1 (6 mL), and the mixture was heated to 70°C and reacted for 1 h, and cooled to room temperature. The system was then directly concentrated to dryness to obtain compound 18K as a crude hydrochloride, which was directly used in the next step.

LC-MS (ESI): m/z = 423.2 $[M+H]^+$.

Step 10: Compound 18K (crude hydrochloride) and compound 18E were dissolved in dichloromethane (10 mL), and pyridine (1.5 mL) was added. The mixture was reacted at room temperature for 30 min. After the reaction was completed as monitored by LCMS, the system was concentrated to obtain a crude, which was purified by preparative TLC (developing agent: EA) to obtain the target compound 18 (8 mg, 11%).

$^1H$ NMR (400 MHz, DMSO-$d_6$) $\delta$ 10.24 (s, 1H), 8.37 (s, 1H), 7.95 - 7.88 (m, 1H), 7.70 - 7.63 (m, 1H), 7.61 - 7.51 (m, 1H), 7.35 (s, 1H), 7.06 (t, 1H), 3.76 (s, 3H), 3.73 (s, 2H), 3.02 (s, 4H), 2.60 (s, 4H).

$^{19}F$ NMR (400 MHz, DMSO-$d_6$) $\delta$ -73.75, -122.05, -125.45, -132.16.

LC-MS (ESI): m/z= 549.1 $[M+H]^+$.

**Example 19**

N-(5-(4-((2-(difluoromethyl)-5-fluoro-3-oxo-3,4-dihydroquinoxalin-6-yl)methyl)piperazin-1-yl)-6-fluoropyridin-2-yl)-N,1-dimethyl-1H-pyrazole-4-carboxamide (compound 19)

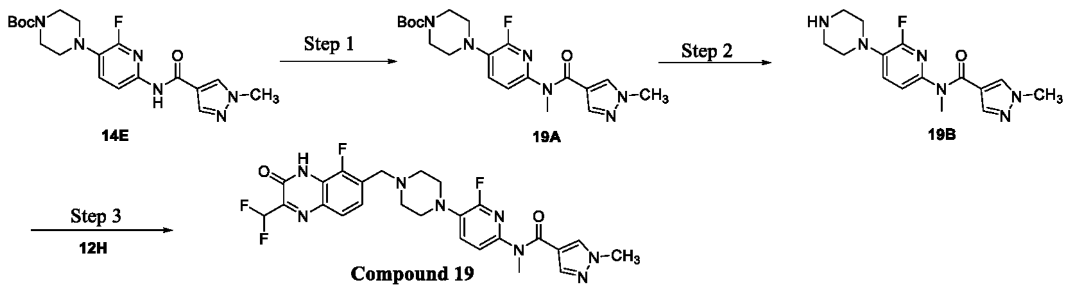


Step 1: Compound 14E (0.505 g, 1.25 mmol) was accurately weighed and added to DMF (10 mL). Under nitrogen protection, the mixture was cooled to about 0°C. NaH (100 mg, 2.5 mmol, 60%) was weighed and added to the reaction liquid, and after the addition was completed, the mixture was stirred at this temperature for 30 min. Iodomethane (0.27 g,

1.9 mmol) was added, and after the addition was completed, the mixture was warmed to room temperature and reacted for 1 h. After the reaction was completed as monitored by TLC spot plate (developing agent: EA), the reaction was destroyed by adding saturated ammonium chloride to the reaction liquid. Then, water (100 mL) was added, and the mixture was extracted with ethyl acetate (150 mL × 2). The organic phases were combined, washed with saturated brine (150 mL × 2), dried over anhydrous sodium sulphate, filtered and concentrated to obtain the target compound 19A (0.44 g, crude, yield: 84.12%), which was directly used in the next reaction.

LC-MS (ESI): m/z = 419.5 $[M+H]^+$.

Step 2: Compound 19A (0.44 g, 1.05 mmol) was weighed and added to dichloromethane (10 mL), and then a solution of hydrogen chloride in dioxane (4 M, 10 mL) was added. The reaction was stirred overnight at room temperature. After the reaction was completed, the reaction liquid was concentrated to obtain compound 19B (0.38 g) as a crude hydrochloride, which was directly used in the next reaction.

LC-MS (ESI): m/z = 319.1 $[M+H]^+$.

Step 3: Compound 19B (0.38 g, crude) and compound 12H (0.29 g, 0.94 mmol) were weighed and dissolved in anhydrous acetonitrile (20 mL), and then diisopropylethylamine (1 mL) was added. The reaction was heated to 75°C and stirred for 3 h. After the reaction was completed as monitored by LCMS, the reaction liquid was cooled to room temperature and then concentrated to remove acetonitrile and DIPEA to obtain a crude, which was purified by Pre-TLC (developing agent: dichloromethane: methanol =15 : 1) to obtain compound 19 (0.276, yield: 53.90%).

LC-MS (ESI): m/z = 545.6$[M+H]^+$.

$^1$H NMR (400 MHz, DMSO-$d_6$) δ 13.00 (s, 1H), 7.73 - 7.71 (m, 1H), 7.66 (s, 1H), 7.52 - 7.48 (m, 1H), 7.44 - 7.40 (m, 1H), 7.21 - 6.93 (m, 2H), 6.86 (s, 1H), 3.74 (s, 5H), 3.26 (s, 3H), 3.10 (s, 4H), 2.61 (s, 4H).

**Example 20**

N-cyclopropyl-5-(4-((2-(difluoromethyl)-5-fluoro-3-oxo-3,4-dihydroquinoxalin-6-yl)methyl)piperazin-1-yl)-6-fluoro-N-methylpicolinamide (compound 20)

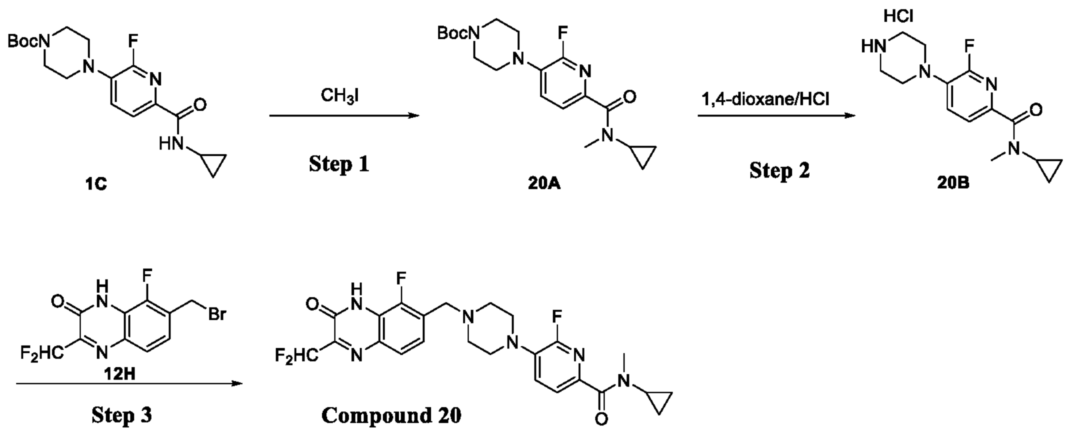


Step 1: Compound 1C (0.455 g, 1.25 mmol) was weighed and added to DMF (10 mL). Under nitrogen protection, the mixture was cooled to about 0°C. NaH (100 mg, 2.5 mmol, 60%) was weighed and added to the reaction liquid, and after the addition was completed, the mixture was stirred at this temperature for 30 min. Iodomethane (0.27 g, 1.9 mmol) was added, and after the addition was completed, the mixture was warmed to room temperature and reacted for 1 h. After the reaction was completed as monitored by TLC (developing agent: EA), the reaction was destroyed by adding saturated ammonium chloride to the reaction liquid. Then, water (100 mL) was added, and the mixture was extracted with ethyl acetate (150 mL × 2). The organic phases were combined, washed with saturated brine (150 mL × 2), dried over anhydrous sodium sulphate, filtered and concentrated to obtain the target compound 20A (0.39 g, 82.6%), which was directly used without further purification.

LC-MS (ESI): m/z = 379.2 $[M+H]^+$.

Step 2: 20A (0.39 g, crude) was dissolved in methanol (10 mL), and an HCl/dioxane (2 mL, 4 M) solution was added. The mixture was reacted at room temperature for 2 h and concentrated to obtain the title compound 20B (283 mg, crude).

LC-MS (ESI): m/z = 279.2 $[M+H]^+$.

Step 3: 12H (200 mg, 0.65 mmol) and 20B (283 mg, crude) were dissolved in anhydrous acetonitrile (20 mL), and

DIPEA (0.5 mL) was added. The mixture was reacted at 60°C for 2 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 20 (172 mg, 52.5%).

$^1$H NMR (400 MHz, DMSO-$d_6$) δ 12.97 (s, 1H), 7.56 - 7.48 (m, 1H), 7.47 - 7.39 (m, 2H), 7.19 - 6.88 (m, 2H), 3.67 (s, 2H), 3.18 - 3.08 (m, 4H), 3.00 - 2.90 (m, 4H), 2.64 - 2.55 (m, 4H), 0.63- 0.19 (m, 4H).

LC-MS (ESI): m/z= 505.2 [M+H]$^+$.

**Example 21**

5-(4-((2-(difluoromethyl)-5-fluoro-3-oxo-3,4-dihydroquinoxalin-6-yl)methyl)piperazin-1-yl)-6-fluoro-N-methyl-N-(1-methyl-1H-pyrazol-4-yl)picolinamide (compound 21)

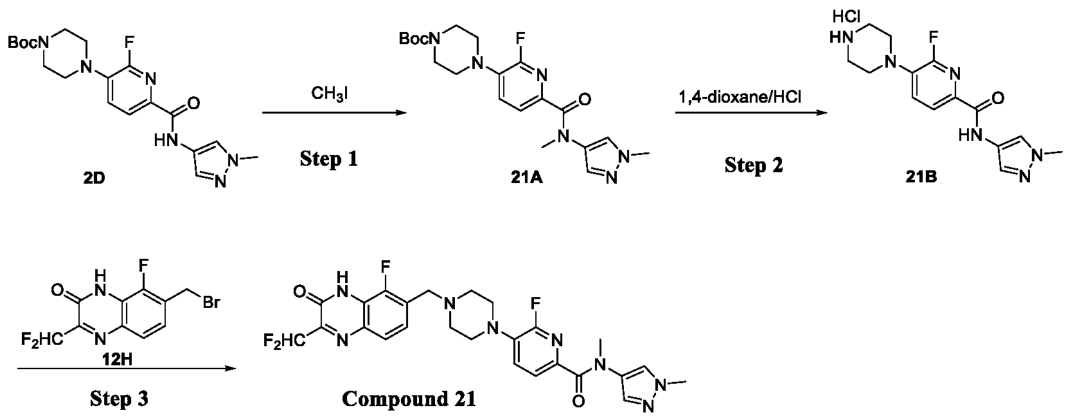


Step 1: Compound 2D (0.404 g, 1 mmol) was weighed and added to DMF (10 mL). Under nitrogen protection, the mixture was cooled to about 0°C. NaH (100 mg, 2.5 mmol, 60%) was weighed and added to the reaction liquid, and after the addition was completed, the mixture was stirred at this temperature for 30 min. Iodomethane (0.27 g, 1.9 mmol) was added, and after the addition was completed, the mixture was warmed to room temperature and reacted for 1 h. After the reaction was completed as monitored by TLC (developing agent: EA), a saturated ammonium chloride solution was added to the reaction liquid, and then water (100 mL) was added. The mixture was extracted with ethyl acetate (150 mL × 2). The organic phases were combined, washed with saturated brine (150 mL × 2), dried over anhydrous sodium sulphate, filtered and concentrated to obtain the target compound 21A (0.37 g, 88.3%), which was directly used without further purification.

LC-MS (ESI): m/z = 419.2 [M+H]$^+$.

Step 2: 21A (0.37 g, crude) was dissolved in methanol (10 mL), and an HCl/dioxane (2 mL, 4 M) solution was added. The mixture was reacted at room temperature for 2 h and concentrated to obtain the title compound 21B (253 mg, crude).

LC-MS (ESI): m/z = 319.2 [M+H]$^+$.

Step 3: 12H (100 mg, 0.33 mmol) and 21B (150 mg, crude) were dissolved in anhydrous acetonitrile (20 mL), and DIPEA (0.5 mL) was added. The mixture was reacted at 60°C for 2 hours. Upon complete depletion of raw materials monitored by LCMS, the system was concentrated. A saturated sodium bicarbonate solution (20 mL) was added, and the mixture was extracted with a mixed solution of DCM : MeOH = 10 : 1 (10 mL × 3). The organic phases were combined, dried over anhydrous sodium sulphate, concentrated and separated by silica gel column chromatography (DCM : MeOH = 1 : 0-10 : 1) to obtain compound 21 (59 mg, 32.85%).

$^1$H NMR (400 MHz, DMSO-$d_6$) δ 13.03 (s, 1H), 7.80 - 7.51 (m, 3H), 7.47 - 7.25 (m, 2H), 7.23 - 6.92 (m, 2H), 3.81 (s, 2H), 3.75 (s, 3H), 3.28 (s, 3H), 3.21 - 3.02 (m, 4H), 2.66 - 2.53 (m, 4H).

LC-MS (ESI): m/z= 545.2 [M+H]$^+$.

**Biological test**

**1. PARP-1 enzyme activity test experiment**

PARP-1 chemical fluorescence detection kit was purchased from BPS Bioscience. The histone solution in the kit

was diluted 5X with 1X PBS, and 25 μL of the diluted histone solution was added to a microwell plate and incubated overnight at 4°C. After the incubation, the plate was washed three times with PBST (0.05% Tween-20). 100 μL of the blocking solution was added to the microwell plate and incubated at 25°C for 90 minutes. After the incubation was completed, the plate was washed three times with PBST. 2.5 μL of compounds at different concentrations diluted in test buffer and 12.5 μL of substrate mixed solution (1.25 μL 10× PARP test buffer; 1.25 μL 10× PARP test mixed solution; 2.5 μL Activated DNA, 7.5 μL double-distilled water) were added to the microwell plate. The PARP-1 enzyme was diluted to 2 ng/μL, 10 μL of the diluent was added to the microwell plate, and the reaction system was incubated at 25°C for 60 minutes.

After the incubation was completed, the plate was washed three times with PBST. Streptavidin-HRP was diluted 50X with a blocking solution, and 25 μL of the diluent was added to the microwell plate and incubated at 25°C for 30 minutes. After the incubation, the plate was washed three times with PBST. ELISA ECL substrate A and substrate B were uniformly mixed at a ratio of 1 : 1 (v/v), 50 μL of the mixture was added to the microwell plate, and the chemiluminescence value was read.

The inhibition rate was calculated according to formula 1, where RLUsample was the readout of the compound well, RLUmax was the readout of the solvent control well, and RLUmin was the readout of the control well without the PARP-1 enzyme. Curve fitting was performed by four parameters (log(inhibitor) vs. response -- Variable slope) using GraphPad Prism software, and the $IC_{50}$ value was calculated.

$$\text{Inhibition\%} = (1-(\text{RLUsample}-\text{RLUmin})/(\text{RLUmax}-\text{RLUmin})) \times 100\% \text{ (formula 1)}$$

Experimental results: the compound of the present invention had a significant inhibitory effect on PARP-1 enzyme activity in vitro, and the $IC_{50}$ value of the example compounds on PARP-1 enzyme activity was less than 100 μM. $IC_{50}$ values are represented by grades A, B, C, and D, where A represents $0 < IC_{50} \leq 5$ nM, B represents 5 nM $< IC_{50} \leq 10$ nM, C represents 10 nM $< IC_{50} \leq 50$ nM, and D represents 50 nM $< IC_{50} \leq 100$ nM. The test results of some examples were shown in Table 1.

Table 1 PARP-1 enzyme activity

| Compound | $IC_{50}$ (nM) |
|---|---|
| 2 | A |
| 4 | A |
| 5 | A |
| 12 | A |
| 14 | A |
| 15 | A |
| 17 | A |
| 18 | A |
| Conclusion: the compounds of the present invention (e.g., example compounds) have significant inhibitory effects on PARP-1 enzyme activity in vitro, especially compound 2, the $IC_{50}$ value of which on PARP-1 is 0.79 nM. | |

**2. MDA-MB-436 cell activity test experiment**

Breast tumour cells MDA-MB-436 were purchased from ATCC, the culture medium was Leibovitz's L-15 +10% FBS, and the cells were cultured in a $CO_2$-free incubator at 37°C. On day 1, the cells in the exponential growth phase were collected, and the cell suspension was adjusted to 4000 cells/135 μL with the culture medium. 135 μL of the cell suspension was added to each well of a 96-well cell culture plate and incubated overnight. On day 2, compounds at different concentrations were added, and the plate was placed in the incubator and incubated for 7 days. After the incubation was completed, according to operation instructions for a CellTiter-Glo kit (Promega, G7573), 75 μL of CTG solution, which was already pre-melted and equilibrated to room temperature, was added to each well, and the mixture was uniformly mixed for 2 min using a microplate shaker. The plate was placed at room temperature for 10 min, and then fluorescence signal values were measured using an Envision2104 plate reader (PerkinElmer). The inhibition rate was calculated using formula (1), where $RLU_{compound}$ was the readout of the drug treated group, $RLU_{control}$ was the average value of the vehicle control group, and $RLU_{blank}$ was the average value of the cell-free well. The $IC_{50}$ value was calculated

using GraphPad Prism software.

$$Inh.\% = (1-(RLU_{compound} - RLU_{blank})/(RLU_{control} - RLU_{blank})) \times 100\% \text{ (formula 1)}$$

Test results: the compounds of the present invention had a significant inhibitory effect on MDA-MB-436 cells, the IC50 value of the compounds on MDA-MB-436 cells was less than 100 nM, and the IC50 value of some excellent compounds on MDA-MB-436 cells was less than 10 nM. The inhibitory rate of the compounds on breast tumour cells MDA-MB-436 was greater than 70%, and the inhibition rate of some excellent compounds was greater than 85%. The results of some specific compounds were as shown in Table 2.

Table 2 MDA-MB-436 cell activity

| Compound | $IC_{50}$ (nM) | Max inh.% 10 μM |
|---|---|---|
| 2 | 3.5 | 86.0 |
| 4 | 32.8 | 80.1 |
| 5 | 28.0 | 79.8 |
| 9 | 1.5 | 84.4 |
| 12 | 10.2 | 74.5 |
| 14 | 24.3 | 73.19 |
| 15 | 6.8 | 74.2 |
| 17 | 19.4 | 72.33 |
| Conclusion: the compounds of the present invention (e.g., example compounds) have good inhibitory activity on breast tumour cells MDA-MB-436. | | |

**3. Pharmacokinetic test in rats**

3.1 Experimental animal: male SD rats, about 220 g, 6-8 weeks old, 6 rats/compound, purchased from Chengdu Ddossy Experimental Animals Co., Ltd.

3.2 Experimental design: on the day of the experiment, 30 SD rats were randomly grouped according to their body weights. The animals were fasted with water available for 12 to 14 h one day before the administration and were fed 4 h after the administration.

Table 3. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 12 | 2.5 | 0.5 | 5 | Plasma | Intravenous administration |
| G2 | 3 | | 10 | 1 | 10 | Plasma | Intragastric administration |
| G3 | 3 | Compound 14 | 2.5 | 0.5 | 5 | Plasma | Intravenous administration |
| G4 | 3 | | 10 | 1 | 10 | Plasma | Intragastric administration |
| G5 | 3 | Compound 15 | 2.5 | 0.5 | 5 | Plasma | Intravenous administration |
| G6 | 3 | | 10 | 1 | 10 | Plasma | Intragastric administration |

(continued)

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G7 | 3 | Compound 18 | 2.5 | 0.5 | 5 | Plasma | Intravenous administration |
| G8 | 3 | | 10 | 1 | 10 | Plasma | Intragastric administration |
| G11 | 3 | Compound 4 | 2.5 | 0.5 | 5 | Plasma | Intravenous administration |
| G12 | 3 | | 10 | 1 | 10 | Plasma | Intragastric administration |
| Note: vehicle for intravenous administration: 10% DMA + 10% Solutol + 80% Saline; vehicle for intragastric administration: 5% DMSO + 30% PEG400 + 65% (20% SBE-CD)<br>(Solutol: polyethylene glycol-15-hydroxystearate; Saline: physiological saline) | | | | | | | |

Before and after the administration, 0.15 ml of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were 0, 5 min, 15 min, 30 min, 1 h, 2 h, 4 h, 6 h, 8 h and 24 h. The brain tissues were collected 24 hours after administration, rinsed with cold physiological saline to remove the residual blood on the surface, drained out and then homogenized. Before analysis and detection, all samples were stored at -80°C. The samples were analysed quantitatively by LC-MS/MS.

Table 4. Pharmacokinetic parameters of test compounds in plasma of rats

| Test compound | Mode of administration | CL (mL/min/kg) | $Vd_{ss}$ (L/kg) | $AUC_{0-t}$ (hr*ng/mL) | F (%) | Brain/Plasma Ratio (24 h) |
|---|---|---|---|---|---|---|
| Compound 12 | i.v. (2.5 mg/kg) | 3.13±1.2 | 0.631±0.12 | 14517±4765 | - | 1.5 |
| | i.g. (10 mg/kg) | - | - | 63275±19621 | ≥98 | |
| Compound 14 | i.v. (2.5 mg/kg) | 0.859±0.090 | 0.148±0.010 | 48705±4795 | - | / |
| | i.g. (10 mg/kg) | - | - | 138052±6646 | 70.9±3.4 | |
| Compound 15 | i.v. (2.5 mg/kg) | 1.63±0.18 | 0.192±0.022 | 25750±2867 | - | 0.916 |
| | i.g. (10 mg/kg) | - | - | 89822±14042 | 87.2±14 | |
| Compound 18 | i.v. (2.5 mg/kg) | 1.90±0.071 | 0.294±0.011 | 21983±838 | - | / |
| | i.g. (10 mg/kg) | - | - | 87649±11698 | ≥98 | |
| Compound 4 | i.v. (2.5 mg/kg) | 5.36±0.068 | 1.13±0.071 | 7386±663 | - | 0.315 |
| | i.g. (10 mg/kg) | - | - | 33396±2667 | ≥98 | |
| -: not applicable.<br>Conclusion: the compounds of the present invention, especially the example compounds, such as compounds 4, 12, 14, 15 and 18, have good pharmacokinetic characteristics in rats. | | | | | | |

**4. Pharmacokinetic test in mice**

4.1 Experimental animal: male Balb/c mice, 20-25 g, 12 mice/compound, purchased from Chengdu Ddossy Experimental Animals Co., Ltd.

4.2 Experimental design: on the day of the test, 60 Balb/c mice were randomly grouped according to their body weights. The animals were fasted with water available for 12 to 14 h one day before the administration and were fed 4 h after the administration.

Table 5. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 3 | Compound 12 | 2.5 | 0.5 | 5 | Plasma | Intravenous administration |
| G2 | 9 | | 10 | 1 | 10 | Plasma | Intragastric administration |
| G3 | 3 | Compound | 2.5 | 0.5 | 5 | Plasma | Intravenous administration |
| G4 | 9 | | 10 | 1 | 10 | Plasma | Intragastric administration |
| G5 | 3 | Compound 18 | 2.5 | 0.5 | 5 | Plasma | Intravenous administration |
| G6 | 9 | | 10 | 1 | 10 | Plasma | Intragastric administration |
| G7 | 3 | Compound 2 | 2.5 | 0.5 | 5 | Plasma | Intravenous administration |
| G8 | 9 | | 10 | 1 | 10 | Plasma | Intragastric administration |
| G11 | 3 | Compound 5 | 2.5 | 0.5 | 5 | Plasma | Intravenous administration |
| G12 | 9 | | 10 | 1 | 10 | Plasma | Intragastric administration |
| Note: vehicle for intravenous administration: 10% DMA + 10% Solutol + 80% Saline; vehicle for intragastric administration: 5% DMSO + 30% PEG400 + 65% (20% SBE-CD) (Solutol: polyethylene glycol-15-hydroxystearate; Saline: physiological saline) | | | | | | | |

Before and after the administration, 0.06 mL of blood was taken from the orbit of the animals under isoflurane anaesthesia and placed in an EDTAK2 centrifuge tube. The blood was centrifuged at 5000 rpm and 4°C for 10 min to collect plasma. The blood collection time points for the intravenous administration group and intragastric administration group were 0, 5 min, 15 min, 30 min, 1 h, 2 h, 4 h, 6 h, 8 h, and 24 h. The brain tissues were collected at 30 min, 2 h and 24 h after administration of compounds 12, 15 and 18, respectively. The brain tissues were collected 24 hours after administration of compounds 2 and 5, rinsed with cold physiological saline to remove the residual blood on the surface, drained out and then homogenized. Before analysis and detection, all samples were stored at - 80°C. The samples were analysed quantitatively by LC-MS/MS.

Table 6. Pharmacokinetic parameters of test compounds in plasma of mice

| Test compound | Mode of administration | CL (mL/min/kg) | $Vd_{ss}$ (L/kg) | $AUC_{0-t}$ (hr*ng/mL) | F (%) | Brain/Plasma Ratio (24 h) |
|---|---|---|---|---|---|---|
| Compound 12 | i.v. (2.5 mg/kg) | 2.16±0.31 | 0.420±0.017 | 18985±3590 | - | / |
| | i.g. (10 mg/kg) | - | - | 80830 | ≥98 | |
| Compound 15 | i.v. (2.5 mg/kg) | 2.57±0.29 | 0.342±0.040 | 16299±1739 | - | / |
| | i.g. (10 mg/kg) | - | - | 67230 | ≥98 | |
| Compound 18 | i.v. (2.5 mg/kg) | 5.14±0.53 | 0.582±0.029 | 8151±811 | - | / |
| | i.g. (10 mg/kg) | - | - | 32332 | ≥98 | |

(continued)

| Test compound | Mode of administration | CL (mL/min/kg) | $Vd_{ss}$ (L/kg) | $AUC_{0-t}$ (hr*ng/mL) | F (%) | Brain/Plasma Ratio (24 h) |
|---|---|---|---|---|---|---|
| Compound 2 | i.v. (2.5 mg/kg) | 21.6±1.3 | 2.09±0.52 | 1867±154 | - | 5.23 |
| | i.g. (10 mg/kg) | - | - | 7273±793 | 97.4 | |
| Compound 5 | i.v. (2.5 mg/kg) | 15.2±1.1 | 2.85±0.23 | 2754±212 | - | 0.911 |
| | i.g. (10 mg/kg) | - | - | 12662±817 | ≥98 | |
| -: not applicable.<br>Conclusion: the compounds of the present invention, especially the example compounds, such as compounds 2, 5, 12, 15 and 18, have good pharmacokinetic characteristics in mice. | | | | | | |

**5. Pharmacokinetic test in beagle dogs**

**5.1 Experimental animal:** male beagle dogs, about 8-11 kg, 6 beagle dogs/compound, purchased from Beijing Marshall Biotechnology Co., Ltd.

**5.2 Test method:** on the day of the experiment, 6 beagle dogs were randomly grouped according to their body weights. The animals were fasted with water available for 12 to 14 h one day before the administration and were fed 4 h after the administration. The administration was performed according to Table 1.

Table 7. Administration information

| Group | Number | Administration information | | | | | |
|---|---|---|---|---|---|---|---|
| | Male | Test compound | Administration dosage (mg/kg) | Administration concentration (mg/mL) | Administrat ion volume (mL/kg) | Collected sample | Mode of administration |
| G1 | 3 | Compound 12 | 0.4 | 0.2 | 2 | Plasma | Intravenous administration |
| G2 | 3 | Compound 12 | 3 | 0.6 | 5 | Plasma | Intragastric administration |
| Note: vehicle for intravenous administration: 5% DMA + 5% Solutol + 90% Saline; vehicle for intragastric administration: 5% DMSO + 5% Solutol + 30% PEG400 + 60% (20% SBE-CD)<br>(DMA: dimethylacetamide; Solutol: polyethylene glycol-15-hydroxystearate; Saline: physiological saline;) | | | | | | | |

Before and after the administration, 1 ml of blood was taken from the jugular veins or limb veins, and placed in an EDTAK2 centrifuge tube. The blood was centrifuged at 5000 rpm and 4°C for 10 min to collect plasma. The blood collection time points for the intravenous administration group and intragastric administration group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

Table 8. Pharmacokinetic parameters of test compounds in plasma of beagle dogs

| Test compound | Mode of | CL | $Vd_{ss}$ (L/kg) | $AUC_{0-t}$ (hr*ng/mL) | F |
|---|---|---|---|---|---|
| | administration | (mL/min/kg) | | | (%) |
| Compound 12 | i.v. (0.2 mg/kg) | 3.47±0.98 | 1.52±0.45 | 1947±474 | - |
| Compound 12 | i.g. (3 mg/kg) | - | - | 18969±2893 | ≥98 |
| -: not applicable.<br>**Conclusion:** the compounds of the present invention, especially the example compounds, such as compound 12, have good pharmacokinetic characteristics in dogs. | | | | | |

**6. Pharmacokinetic test in monkeys**

**6.1 Experimental animal:** male cynomolgus monkeys, 3-5 kg, 3-6 years old, 4 monkeys/compound, purchased from Suzhou Xishan Biotechnology Inc.

**6.2 Test method:** On the day of the experiment, 8 monkeys were randomly grouped according to their body weights. The animals were fasted with water available for 14 to 18 h one day before the administration, and were fed 4 h after the administration.

Table 9. Administration information

| Group | Number | Administration information | | | | | |
|---|---|---|---|---|---|---|---|
| | Male | Test compound | Administration dosage (mg/kg) | Administration concentration (mg/mL) | Administration volume (mL/kg) | Collected sample | Mode of administration |
| G1 | 2 | Compound 12 | 0.5 | 0.25 | 2 | Plasma | Intravenous administration |
| G2 | 2 | Compound 12 | 3 | 0.6 | 5 | Plasma | Intragastric administration |
| G3 | 2 | Compound 14 | 1 | 1 | 1 | Plasma | Intravenous administration |
| G4 | 2 | Compound 14 | 3 | 0.6 | 5 | Plasma | Intragastric administration |
| Note: vehicle for intravenous administration: 5% DMA + 5% Solutol + 90% Saline; vehicle for intragastric administration: 5% DMSO + 5% Solutol + 30% PEG400 + 60% (20% SBE-CD)<br>*Dosage is calculated based on free base. | | | | | | | |

Before and after the administration, 1.0 mL of blood was taken from the limb veins and placed in an EDTAK2 centrifuge tube. The blood was centrifuged at 5000 rpm and 4°C for 10 min to collect plasma. The blood collection time points for the intravenous administration group were 0, 5 min, 15 min, 30 min, 1 h, 2 h, 4 h, 6 h, 8 h, 10 h, 12 h, 24 h and 48 h. Before analysis and detection, all samples were stored at -80°C. The samples were analysed quantitatively by LC-MS/MS.

Table 10. Pharmacokinetic parameters of test compounds in plasma of monkeys

| Test compound | Mode of administration | CL (mL/min/kg) | $Vd_{ss}$ (L/kg) | $AUC_{0-t}$ (hr*ng/mL) | F (%) |
|---|---|---|---|---|---|
| Compound 12 | i.v. (0.5 mg/kg) | 1.50±0.13 | 0.419±0.020 | 5467±422 | - |
| Compound 12 | i.g. (3 mg/kg) | - | - | 20716±1986 | 63.2±6.1 |
| Compound 14 | i.v. (1 mg/kg) | 0.348±0.057 | 0.151±0.022 | 47058±6264 | - |
| Compound 14 | i.g. (3 mg/kg) | - | - | 80131±6963 | 56.8±4.9 |
| Conclusion: the compounds of the present invention, especially the example compounds, such as compounds 12 and 14, have good pharmacokinetic characteristics in monkeys. | | | | | |

## Claims

1. A compound represented by formula (II), or a stereoisomer, solvate, deuterated form or pharmaceutically acceptable salt thereof,

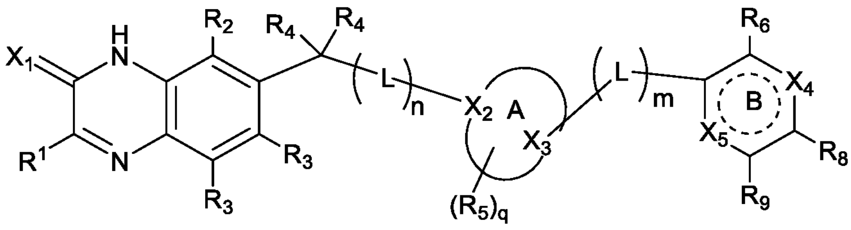

(II)

**characterized in that** $X_1$ is O or S;
$X_2$ is N, C or $CR_{5'}$;
$X_3$ is N, C or $CR_{5''}$;
ring A is 4- to 12-membered heterocycloalkyl containing 1-3 N atoms and 0-2 heteroatoms selected from O and S;
$R_1$, $R_2$ and $R_3$ are independently H, D, halogen, CN, $NH_2$, $-SF_5$, $C_{1-4}$ alkyl, $C_{2-6}$ alkenyl, $C_{2-6}$ alkynyl, $C_{1-4}$ alkoxy, $C_{3-6}$ cycloalkyl, $-O-C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, and the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
each $R_4$ is independently selected from H, D, halogen, $C_{1-4}$ alkyl, $C_{3-6}$ cycloalkyl, OH, CN, $NH_2$, $C_{1-4}$ alkoxy, $C_{2-6}$ alkenyl or $C_{2-6}$ alkynyl, wherein the alkyl, alkoxy, cycloalkyl, alkenyl and alkynyl are optionally substituted with 1-5 groups selected from D, halogen, $C_{1-4}$ alkyl, $C_{1-4}$ alkoxy, OH, CN, $NH_2$ and $C_{1-4}$ haloalkyl;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each L is independently -NH-, $-CH_2-$, -O-, -S-, -S(=O)-, $-S(=O)_2-$ or -C(=O)-; n and m are independently 0, 1, 2 or 3;
$R_5$, $R_{5'}$ and $R_{5''}$ are each independently H, D, =O, halogen, $C_{1-4}$ alkyl, $C_{1-4}$ alkoxy, CN or $C_{3-6}$ cycloalkyl, wherein the alkyl, alkoxy and cycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
optionally, one $R_4$ forms a bond with $R_{5'}$;
q is 0, 1, 2, 3 or 4;
$X_4$ is N, $NR_7$ or $CR_7$;
$X_5$ is N, $NR_{10}$ or $CR_{10}$;
ring B is six-membered heteroaryl;
$R_6$, $R_7$, $R_9$ and $R_{10}$ are independently H, D, CN, OH, halogen, $C_{1-4}$ alkyl, $C_{1-4}$ alkoxy, $C_{3-6}$ cycloalkyl or 4- to 7-membered heterocycloalkyl containing 1-3 heteroatoms selected from N, S and O, wherein the alkyl, alkoxy, cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
$R_8$ is $C_{1-4}$ alkyl, $C_{1-4}$ alkoxy, $-NHC_{1-4}$ alkyl, $-CONHC_{1-4}$ alkyl, $-CONHC_{1-4}$ alkyleneO$C_{1-4}$ alkyl, $-NHCOC_{1-4}$ alkyl, $-NHCOOC_{1-4}$ alkyl, $-OCONHC_{1-4}$ alkyl, $C_{2-6}$ alkenyl, $C_{2-6}$ alkynyl, $R_{11}$, $-NHR_{11}$, $-(CH_2)_rR_{11}$, $-OR_{11}$, $-COR_{11}$, $-(CH_2)_rNHR_{11}$, $-NH(CH_2)_rR_{11}$, $-(CH_2)_rOR_{11}$, $-O(CH_2)_rR_{11}$, $-CONHR_{11}$, $-NHCOR_{11}$, $-NHCONHR_{11}$, $-CONH(CH_2)_rR_{11}$, $-CONH(CH_2)_rOR_{11}$, $-(CH_2)_rCONHR_{11}$, $-OCONHR_{11}$, $-COOR_{11}$, or $-CO(CH_2)_rNHR_{11}$, wherein the alkyl, alkylene, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each $R_{11}$ is independently $C_{3-6}$ cycloalkyl, 4- to 7-membered heterocycloalkyl containing 1-3 heteroatoms selected from N, S and O, 5- to 6-membered heteroaryl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, $-NHC_{1-4}$ alkyl, $-$ $CONHC_{1-4}$ alkyl, $-NHCOC_{1-4}$ alkyl, OH, CN, $NH_2$ and $C_{1-4}$ haloalkyl;
each r is independently 1, 2 or 3;
provided that when

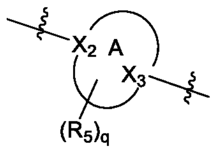

is

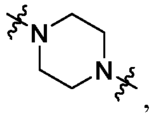

n and m are 0,

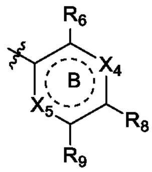

is

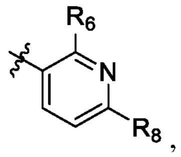

and $R_6$ is H, halogen, $C_{1-4}$ alkyl or halo$C_{1-4}$ alkyl, $R_8$ is not - $CONHC_{1-4}$ alkyl.

2. The compound, or the stereoisomer, solvate, deuterated form or pharmaceutically acceptable salt thereof according to claim 1,

**characterized in that** $X_1$ is O;
$X_2$ is N or $CR_{5'}$;
$X_3$ is N, C or $CR_{5''}$;
ring A is 4- to 9-membered monocyclic heterocycloalkyl, 6- to 12-membered spiroheterocycloalkyl, 6- to 12-membered fused heterocycloalkyl or 6- to 12-membered bridged heterocycloalkyl containing 1-3 N atoms and 0-2 heteroatoms selected from O and S;
$R_1$ is H, D, halogen, CN, $C_{1-4}$ alkyl, $C_{2-4}$ alkenyl, $C_{2-4}$ alkynyl, $C_{1-4}$ alkoxy, $C_{3-6}$ cycloalkyl, -O-$C_{3-6}$ cycloalkyl, $-(CH_2)_r-C_{3-6}$ cycloalkyl, heterocycloalkyl, -O-heterocycloalkyl or $-(CH_2)_r$-heterocycloalkyl, wherein the heterocycloalkyl is 4- to 7-membered heterocycle containing 1-3 heteroatoms selected from N, S and O, wherein the alkyl, alkenyl, alkynyl, alkoxy, cycloalkyl and heterocycloalkyl are optionally substituted with 1-5 groups selected from D, halogen, $C_{1-4}$ alkyl, $C_{1-4}$ alkoxy and $C_{1-4}$ haloalkyl;
$R_2$ and $R_3$ are independently H, D, halogen or $C_{1-4}$ alkyl, wherein the alkyl is optionally substituted with 1-5 groups selected from D, halogen, OH, CN and $NH_2$;
each $R_4$ is independently selected from H, D, halogen, $C_{1-4}$ alkyl, $C_{3-6}$ cycloalkyl, OH, CN or $NH_2$, wherein the alkyl and cycloalkyl are optionally substituted with 1-5 groups selected from D, halogen, $C_{1-4}$ alkyl, $C_{1-4}$ alkoxy, OH, CN, $NH_2$ and $C_{1-4}$ haloalkyl;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
each L is independently -NH-, $-CH_2-$, -O- or -C(=O)-;
n and m are independently 0, 1, 2 or 3;
$R_5$, $R_{5'}$ and $R_{5''}$ are each independently H, D, =O, halogen, $C_{1-4}$ alkyl or $C_{1-4}$ alkoxy, wherein the alkyl and alkoxy are optionally substituted with 1-3 groups selected from D, halogen, OH, CN and $NH_2$;

optionally, one $R_4$ forms a bond with $R_{5'}$;
q is 0, 1 or 2;
$R_6$, $R_7$, $R_9$ and $R_{10}$ are independently H, D, CN, OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl.

**3.** The compound, or the stereoisomer, solvate, deuterated form or pharmaceutically acceptable salt thereof according to claim 2,

**characterized in that** ring A is substituted or unsubstituted 6- to 10-membered fused heterocycloalkyl, substituted or unsubstituted 6- to 11-membered spiroheterocycloalkyl, substituted or unsubstituted 6- to 10-membered bridged heterocycloalkyl, substituted or unsubstituted 4- to 5-membered monocyclic heterocycloalkyl, substituted or unsubstituted 7- to 8-membered monocyclic heterocycloalkyl, substituted or unsubstituted

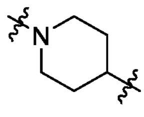
,

or substituted or unsubstituted

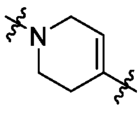
;

or

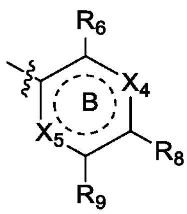

is selected from substituted or unsubstituted

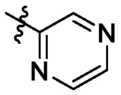 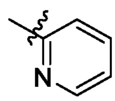
;

or
$R_8$ is $C_{1-4}$ alkyl, $C_{1-4}$ alkoxy, -NH$C_{1-4}$ alkyl, -NHCO$C_{1-4}$ alkyl, -NHCOO$C_{1-4}$ alkyl, -OCONH$C_{1-4}$ alkyl, -CONH$C_{1-4}$ alkyleneO$C_{1-4}$ alkyl, $C_{2-6}$ alkenyl, $C_{2-6}$ alkynyl, $R_{11}$, -NH$R_{11}$, -$(CH_2)_r R_{11}$, -O$R_{11}$, -CO$R_{11}$, -$(CH_2)_r$NH$R_{11}$, -NH$(CH_2)_r R_{11}$, - $(CH_2)_r$O$R_{11}$, -O$(CH_2)_r R_{11}$, -CONH$R_{11}$, -NHCO$R_{11}$, -NHCONH$R_{11}$, - CONH$(CH_2)_r R_{11}$, -CONH$(CH_2)_r$O$R_{11}$, -COO$R_{11}$, -$(CH_2)_r$CONH$R_{11}$, -OCONH$R_{11}$, or -CO$(CH_2)_r$NH$R_{11}$, wherein the alkyl, alkoxy, alkenyl and alkynyl are optionally substituted with 1-3 groups selected from D, halogen, $C_{1-4}$ alkyl, $C_{1-4}$ alkoxy, OH, CN, $NH_2$ and $C_{1-4}$ haloalkyl.

**4.** The compound, or the stereoisomer, solvate, deuterated form or pharmaceutically acceptable salt thereof according to claim 2,

**characterized in that**
ring A is selected from

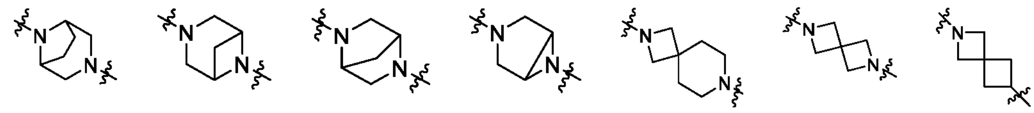

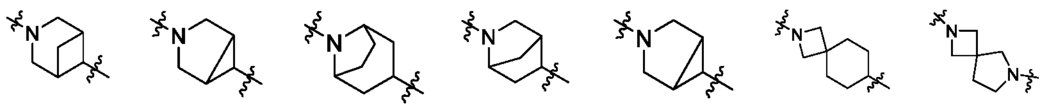

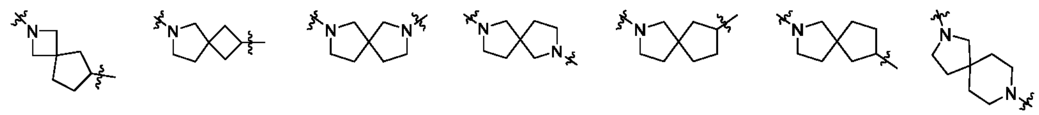

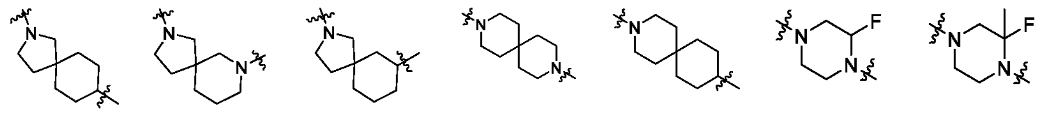

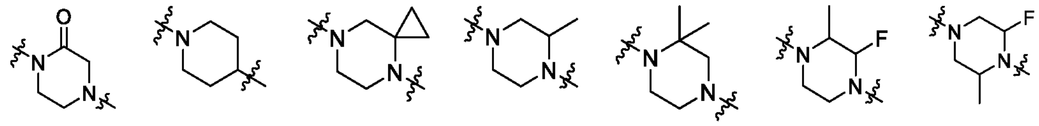

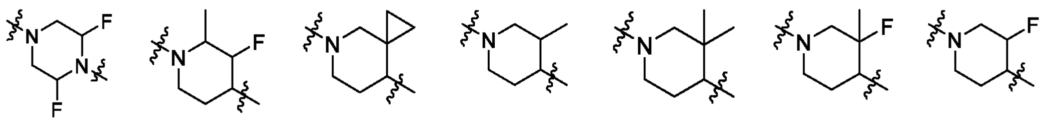

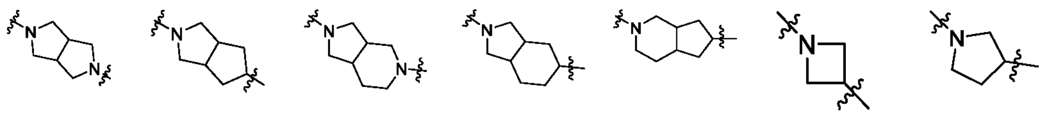

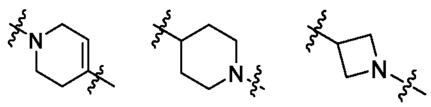

.

5. The compound, or the stereoisomer, solvate, deuterated form or pharmaceutically acceptable salt thereof according to claim 3 or 4, **characterized in that**

$R_1$ is H, D, F, Cl, Br, I, $C_{1-4}$ alkyl or $C_{3-6}$ cycloalkyl, wherein the alkyl and cycloalkyl are optionally substituted with 1-3 groups selected from D, halogen, $C_{1-4}$ alkyl, $C_{1-4}$ alkoxy and $C_{1-4}$ haloalkyl;
$R_2$ is H, D, F, Cl, Br, I or $C_{1-4}$ alkyl, wherein the alkyl is optionally substituted with 1-5 groups selected from D, halogen, OH, CN and $NH_2$;
$R_3$ is H, D, F, Cl, Br or I;
each $R_4$ is independently selected from H, D, F, Cl, Br or I;
optionally, two $R_4$ together with the carbon atom to which they are attached form $C_{3-6}$ cycloalkyl or 4- to 7-membered heterocycloalkyl containing 1-3 heteroatoms selected from N, S and O, wherein the cycloalkyl and heterocycloalkyl are optionally substituted with 1-3 groups selected from D, halogen, $C_{1-4}$ alkyl, $C_{1-4}$ alkoxy, OH, CN, $NH_2$ and $C_{1-4}$ haloalkyl;
n and m are 0;
$R_5$, $R_{5'}$ and $R_{5''}$ are each independently H, D, F, Cl, Br, I, $C_{1-4}$ alkyl or $C_{1-4}$ alkoxy, wherein the alkyl and alkoxy are optionally substituted with 1-3 groups selected from D, halogen, OH, CN and $NH_2$;
optionally, one $R_4$ forms a bond with $R_{5'}$;
q is 0, 1 or 2;
ring B is six-membered heteroaryl containing 1-2 N atoms;

$R_6$, $R_7$, $R_9$ and $R_{10}$ are independently H, D, CN, OH, halogen, $C_{1-4}$ alkyl or $C_{1-4}$ alkoxy, wherein the alkyl and alkoxy are optionally substituted with 1-3 groups selected from D, halogen, $C_{1-4}$ alkyl, $C_{1-4}$ alkoxy, OH, CN, $NH_2$ and $C_{1-4}$ haloalkyl.

**6.** The compound, or the stereoisomer, solvate, deuterated form or pharmaceutically acceptable salt thereof according to claim 1, **characterized in that** the compound has a structure of formula (IV) or (V):

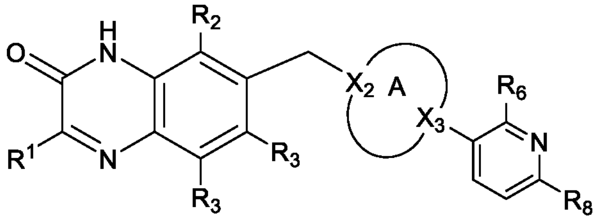
(IV)

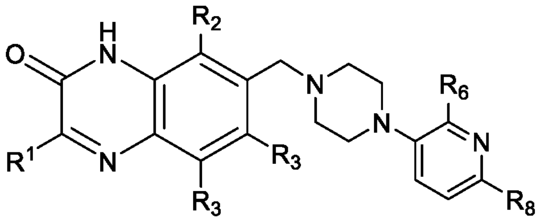
(V)

wherein

ring A is selected from

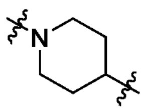, 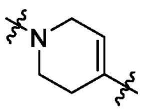, or 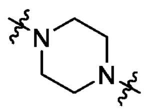;

$R_1$ is $C_{1-4}$ alkyl and $C_{3-6}$ cycloalkyl, wherein the alkyl and cycloalkyl are optionally substituted with 1-3 groups selected from D, halogen, OH, CN and $NH_2$;
$R_3$ is H or D;
each $R_2$ is independently F, Cl, CN, $NH_2$, $C_{1-2}$ alkyl, $C_{2-4}$ alkenyl or $C_{2-4}$ alkynyl, wherein the alkyl is optionally further substituted with 1-3 groups selected from D, F, Cl, OH, CN and $NH_2$;
$R_6$ is D, CN, OH, F, Cl, $C_{1-2}$ alkyl, $C_{1-2}$ alkoxy, $C_{3-4}$ cycloalkyl or 4- to 5-membered heterocycloalkyl containing 1-3 heteroatoms selected from N, S and O, wherein the alkyl, alkoxy, cycloalkyl and heterocycloalkyl are optionally substituted with 1-3 groups selected from D, F, Cl, OH, CN and $NH_2$;
$R_8$ is $-CONHR_{11}$ or $-NHCOR_{11}$;
each $R_{11}$ is independently $C_{3-6}$ cycloalkyl, 4- to 7-membered heterocycloalkyl containing 1-3 heteroatoms selected from N, S and O, or 5- to 6-membered heteroaryl containing 1-3 heteroatoms selected from N, S and O, wherein the cycloalkyl, heterocycloalkyl and heteroaryl are optionally substituted with 1-3 groups selected from D, =O, halogen, $C_{1-4}$ alkyl, $C_{1-4}$ alkoxy, $-NHC_{1-4}$ alkyl, - $CONHC_{1-4}$ alkyl, $-NHCOC_{1-4}$ alkyl, OH, CN, $NH_2$ and $C_{1-4}$ haloalkyl.

**7.** The compound, or the stereoisomer, solvate, deuterated form or pharmaceutically acceptable salt thereof according to claim 6, **characterized in that**

$R_1$ is $C_{1-2}$ alkyl, wherein the alkyl is optionally substituted with 1-3 groups selected from D, F and Cl;
$R_3$ is H;
$R_2$ is F, Cl or $C_{1-2}$ alkyl, wherein the alkyl is optionally further substituted with 1-3 groups selected from D, F and Cl;
$R_6$ is D, F, Cl and $C_{1-2}$ alkyl, wherein the alkyl is optionally substituted with 1-3 groups selected from D, F and Cl;
$R_8$ is $-CONHR_{11}$ or $-NHCOR_{11}$;
$R_{11}$ is $C_{3-6}$ cycloalkyl or 5- to 6-membered heteroaryl containing 1-3 heteroatoms selected from N, S and O, wherein the cycloalkyl and heteroaryl are optionally substituted with 1-3 groups selected from D, F, Cl, $C_{1-2}$ alkyl

and $C_{1-2}$ haloalkyl.

**8.** A compound represented by formula (II), formula (IV) or formula (V), or a stereoisomer, solvate, deuterated form or pharmaceutically acceptable salt thereof, **characterized in that** the compound has a structure selected from one of

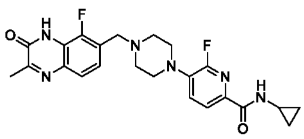
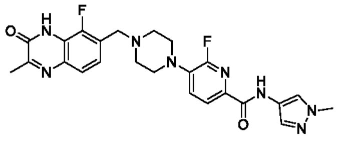
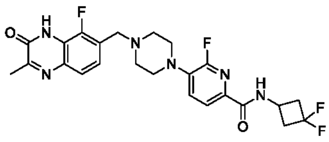
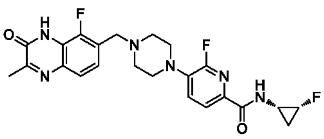
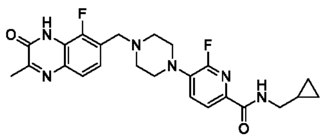
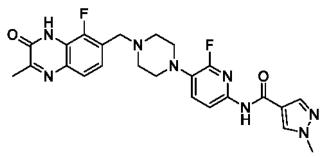
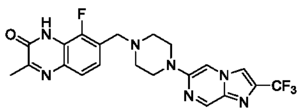
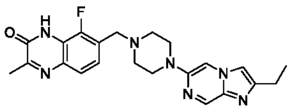
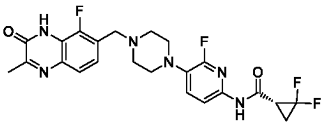
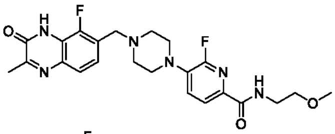
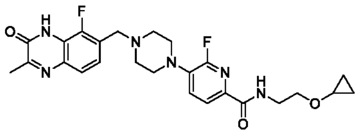
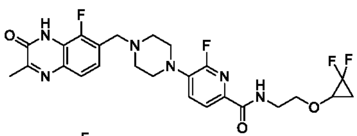
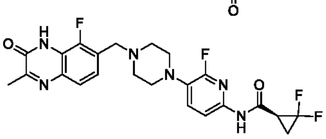
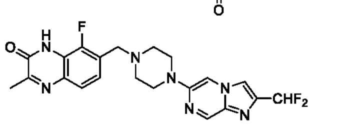
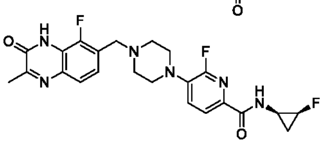
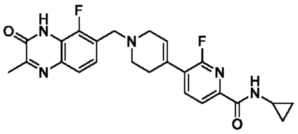
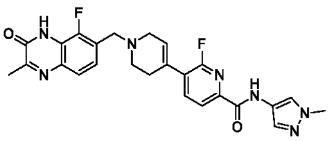
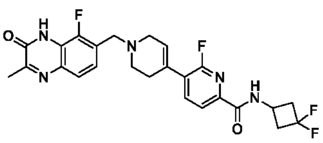
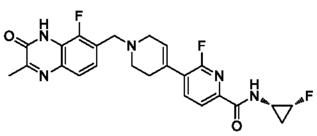
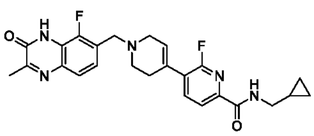
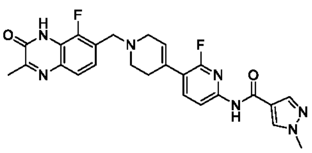
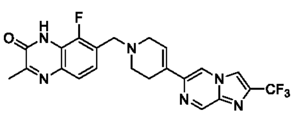
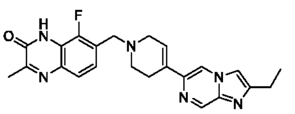
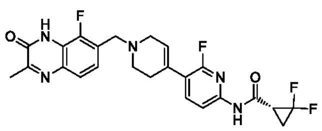

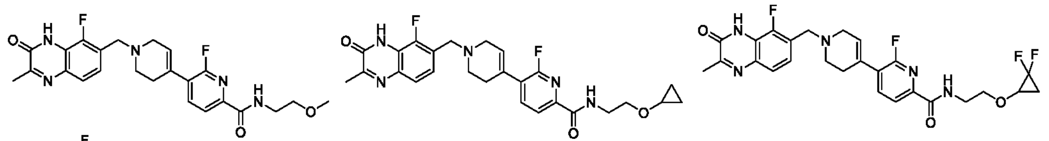

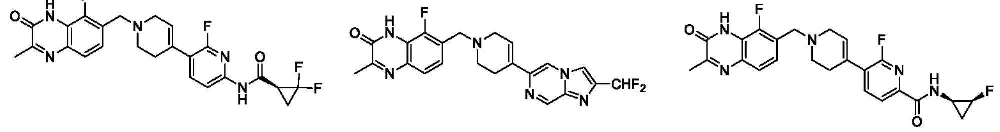

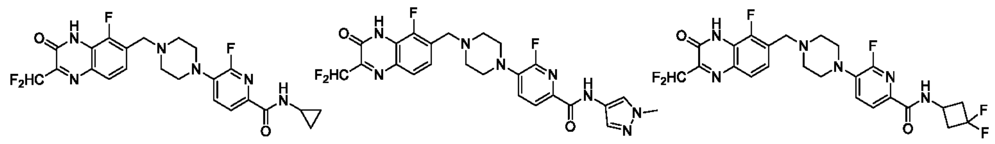

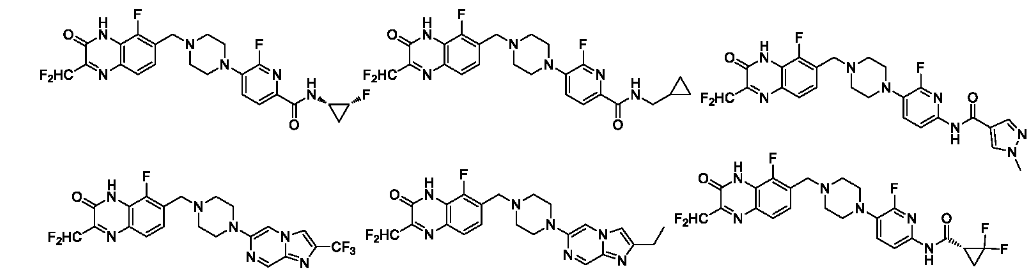

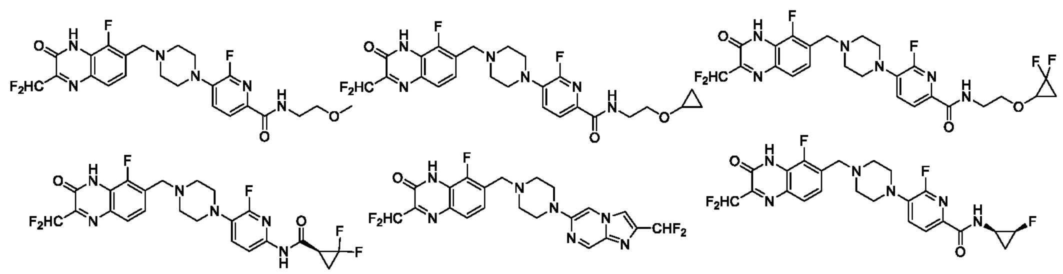

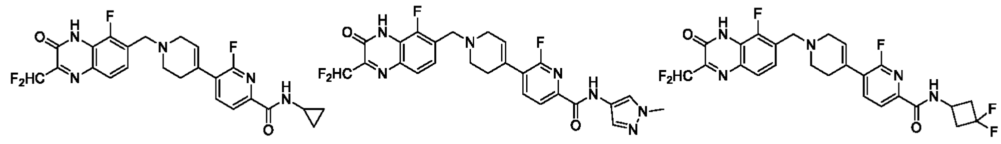

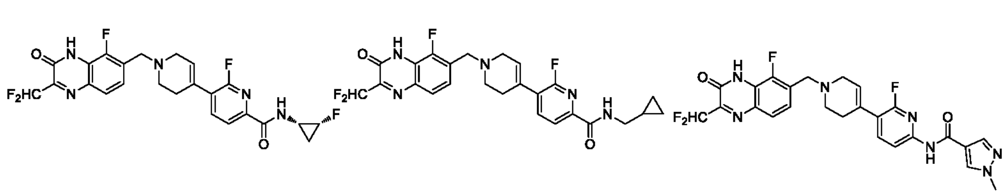

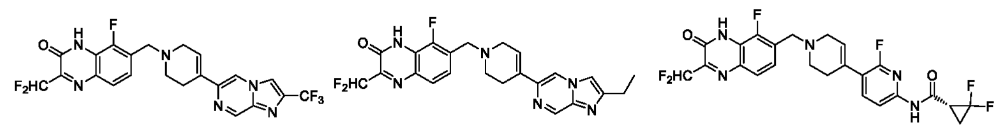

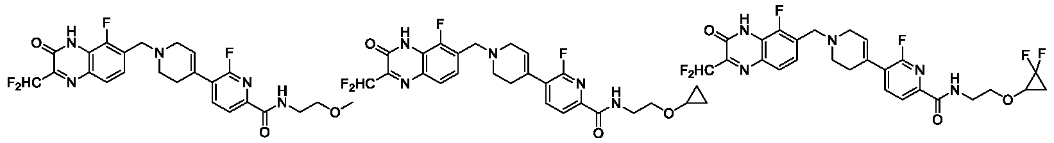

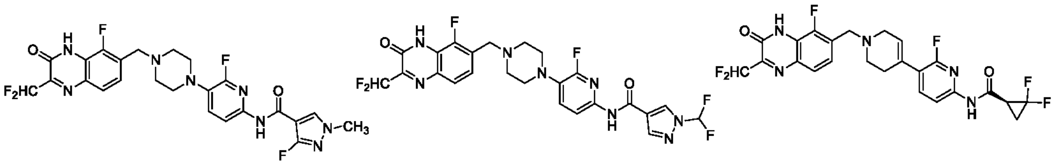

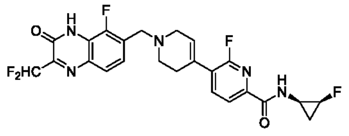

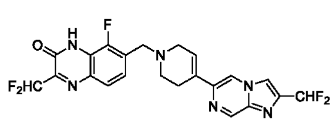

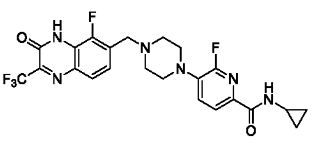

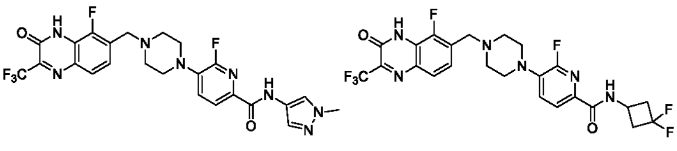

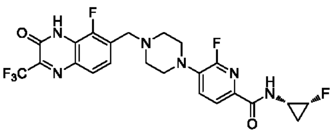

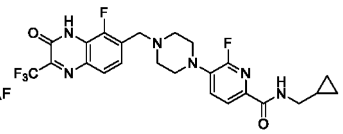

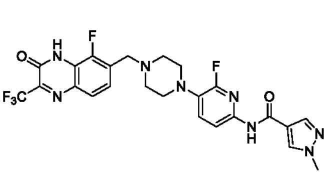

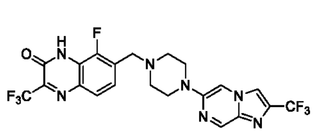

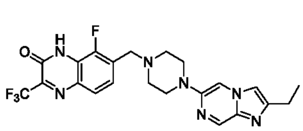

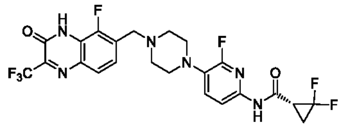

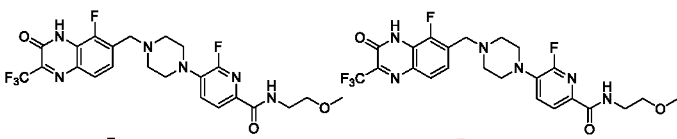

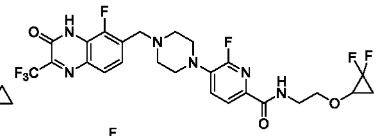

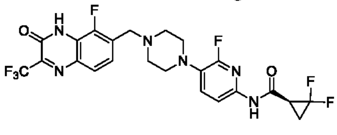

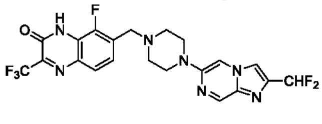

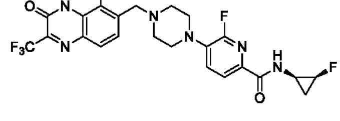

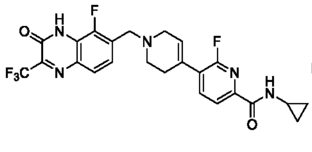

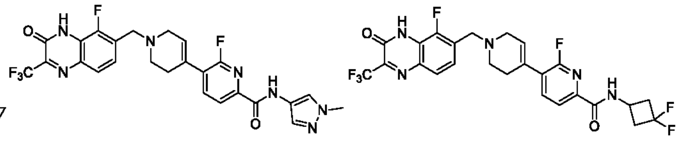

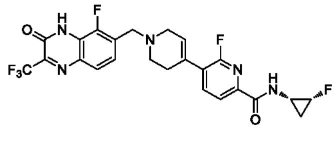

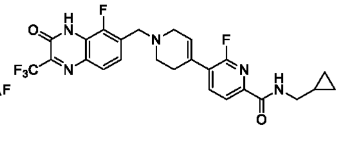

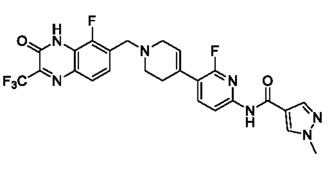

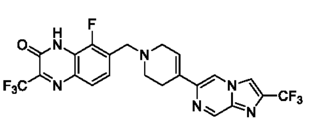
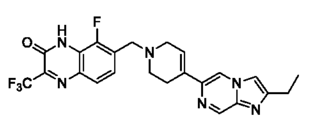
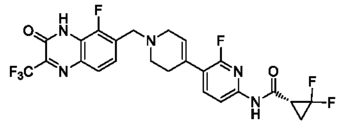
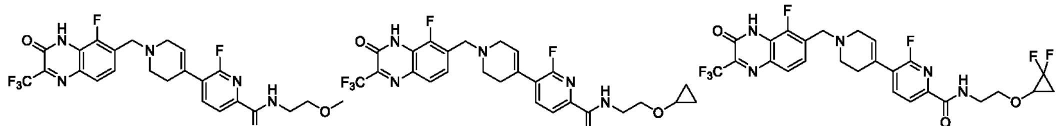
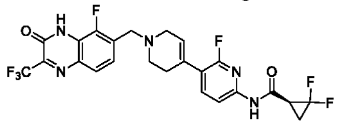
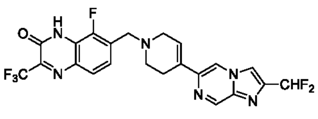
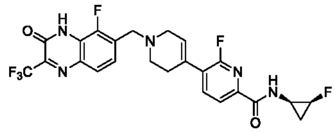
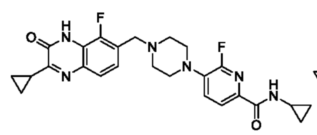
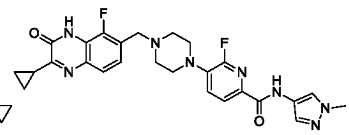
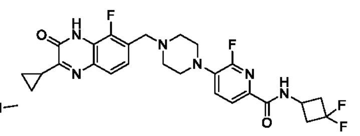
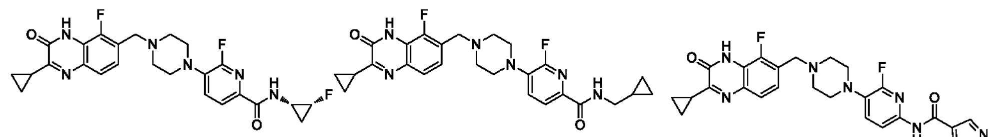
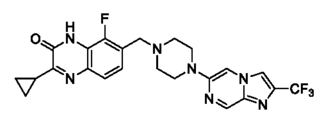
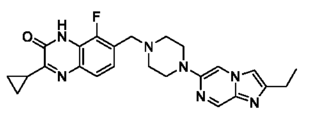
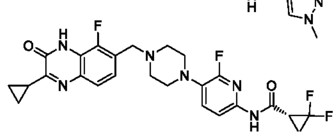
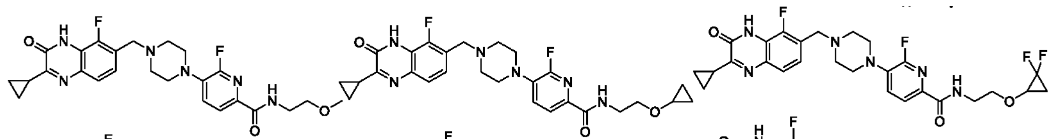
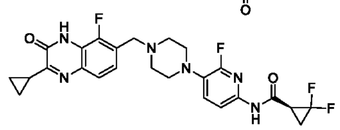
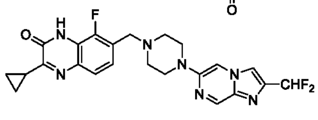
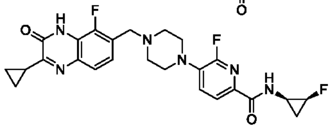
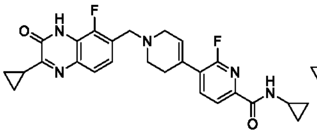
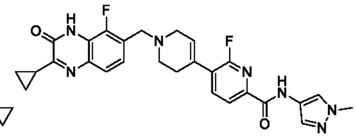
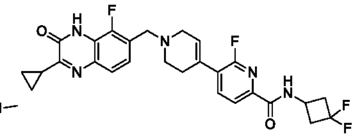
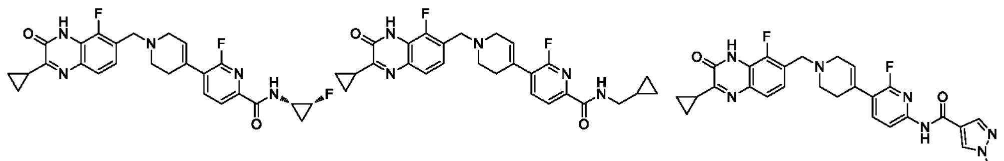
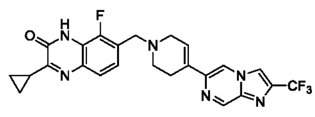
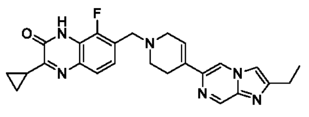

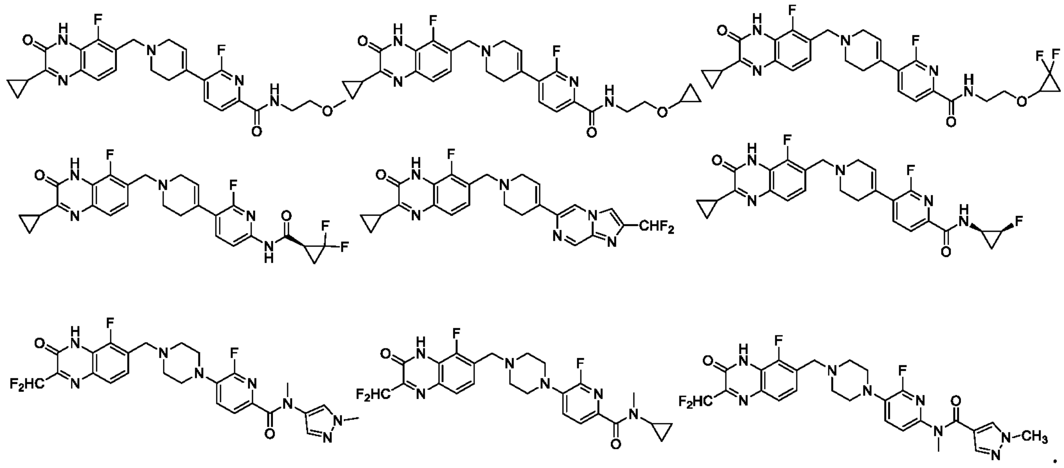

9. A pharmaceutical composition, **characterized in that** the pharmaceutical composition comprises the compound, or the stereoisomer, solvate, deuterated form or pharmaceutically acceptable salt thereof according to any one of claims 1-8, and a pharmaceutically acceptable carrier and/or excipient.

10. Use of the compound, or the stereoisomer, solvate, deuterated form or pharmaceutically acceptable salt thereof according to any one of claims 1-8, or the composition according to claim 9 in the preparation of a drug for treating/preventing a PARP1-mediated disease.

11. The use according to claim 10, **characterized in that** the PARP1-mediated disease is selected from breast cancer, uterine cancer, cervical cancer, ovarian cancer and prostate cancer.

12. A pharmaceutical composition or pharmaceutical preparation, **characterized in that** the pharmaceutical composition or pharmaceutical preparation comprises 1-1440 mg of the compound, or the stereoisomer, solvate, deuterated form or pharmaceutically acceptable salt thereof according to any one of claims 1-8, and a pharmaceutically acceptable carrier and/or excipient.

13. A method for treating a disease in a mammal, **characterized in that** the method comprises administering to a subject a therapeutically effective amount of the compound, or the stereoisomer, solvate, deuterated form or pharmaceutically acceptable salt thereof according to any one of claims 1-8, wherein the therapeutically effective amount is preferably 1-1440 mg, and the disease is preferably breast cancer, uterine cancer, cervical cancer, ovarian cancer and prostate cancer.

**INTERNATIONAL SEARCH REPORT**

International application No. **PCT/CN2023/096215**

**A. CLASSIFICATION OF SUBJECT MATTER**

C07D471/04(2006.01)i； C07D401/12(2006.01)i； C07D401/14(2006.01)i； C07D519/00(2006.01)i； A61K31/498(2006.01)i； A61K31/4709(2006.01)i； A61P35/00(2006.01)i； A61K31/496(2006.01)i

According to International Patent Classification (IPC) or to both national classification and IPC

**B. FIELDS SEARCHED**

Minimum documentation searched (classification system followed by classification symbols)

C07D,A61K,A61P

Documentation searched other than minimum documentation to the extent that such documents are included in the fields searched

Electronic data base consulted during the international search (name of data base and, where practicable, search terms used)

CNTXT, VCN, VEN, ENTXT, ENTXTC, STN(REG, CAPLUS): PARP, 抑制剂, cancer, tumor, ihibitor, 结构式, structural formula

**C. DOCUMENTS CONSIDERED TO BE RELEVANT**

| Category* | Citation of document, with indication, where appropriate, of the relevant passages | Relevant to claim No. |
|---|---|---|
| X | CN 114144413 A (ASTRAZENECA AB) 04 March 2022 (2022-03-04) claims 1 and 16-37, description, paragraphs 65-66, and examples 8-28 | 1-12 |
| X | WO 2021260092 A1 (ASTRAZENECA AB) 30 December 2021 (2021-12-30) examples 1-60 | 1-12 |
| X | WO 2022074617 A1 (ASTRAZENECA UK LTD. et al.) 14 April 2022 (2022-04-14) synthesis examples 8-28 | 1-12 |
| PX | WO 2022222921 A1 (WIGEN BIOMEDICINE TECHNOLOGY (SHANGHAI) CO., LTD.) 27 October 2022 (2022-10-27) claims 1-12 | 1-12 |
| PX | WO 2022228387 A1 (FOCHON BIOSCIENCES LTD.) 03 November 2022 (2022-11-03) claims 1-23 | 1-12 |
| PX | WO 2022261777 A1 (REPARE THERAPEUTICS INC.) 22 December 2022 (2022-12-22) claims 1-23 | 1-12 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 September 2023** | **16 September 2023** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/096215**

**C. DOCUMENTS CONSIDERED TO BE RELEVANT**

| Category* | Citation of document, with indication, where appropriate, of the relevant passages | Relevant to claim No. |
|---|---|---|
| PX | WO 2023046034 A1 (MINGHUI PHARMACEUTICAL (HANGZHOU) LTD. et al.) 30 March 2023 (2023-03-30)<br>claims 1-16 | 1-12 |
| PX | WO 2023046158 A1 (ZHANG, Wenyan) 30 March 2023 (2023-03-30)<br>claims 1-14 | 1-12 |
| PX | WO 2023056039 A1 (XINTHERA, INC.) 06 April 2023 (2023-04-06)<br>claims 1-85 | 1-12 |
| E | WO 2023088408 A1 (CHENGDU BAIYU PHARMACEUTICAL CO., LTD.) 25 May 2023 (2023-05-25)<br>claims 8-10 | 1-3, 5, 9-12 |
| E | WO 2023118085 A1 (ASTRAZENECA AB) 29 June 2023 (2023-06-29)<br>claims 25-32 | 1-3, 5, 9-12 |
| E | WO 2023134647 A1 (ACERAND THERAPEUTICS (HONG KONG) LTD.) 20 July 2023 (2023-07-20)<br>claims 15, 17-19 | 1-3, 5, 9-12 |
| E | WO 2023138541 A1 (WIGEN BIOMEDICINE TECHNOLOGY (SHANGHAI) CO., LTD.) 27 July 2023 (2023-07-27)<br>claims 12, 14-17 | 1-3, 5-7, 9-12 |
| E | WO 2023146957 A1 (XINTHERA, INC.) 03 August 2023 (2023-08-03)<br>embodiments 28, 33-35, 42-43, 48, and some of the compounds of claims 37-46 | 1-3, 5-7, 9-12 |
| A | US 2010222348 A1 (ANGIBAUD PATRICK RENE et al.) 02 September 2010 (2010-09-02)<br>entire document | 1-12 |
| X | Johannes et al. "Discovery of 5-{4-[(7-Ethyl-6-oxo-5,6-dihydro-1,5-naphthyridin-3-yl)methyl]piperazin-1-yl}-N-methylpyridine-2-carboxamide (AZD5305): A PARP1-DNA Trapper with High Selectivity for PARP1 over PARP2 and Other PARPs"<br>*Journal of Medicinal Chemistry,* Vol. 64, No. (19), 27 September 2021 (2021-09-27),<br>pp. 14498-14512 | 1-12 |

Form PCT/ISA/210 (second sheet) (July 2022)

**INTERNATIONAL SEARCH REPORT**

International application No.
**PCT/CN2023/096215**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13**
because they relate to subject matter not required to be searched by this Authority, namely:
Claim 13 relates to a treatment method implemented on the human/animal body (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/096215**

| Patent document cited in search report | Publication date (day/month/year) | Patent family member(s) | Publication date (day/month/year) |
|---|---|---|---|
| CN 114144413 A | 04 March 2022 | AU 2020318599 A1 | 10 March 2022 |
| | | US 2021040084 A1 | 11 February 2021 |
| | | US 11325906 B2 | 10 May 2022 |
| | | CR 20220070 A | 21 March 2022 |
| | | UY 38793 A | 26 February 2021 |
| | | ECSP 22012826 A | 31 March 2022 |
| | | BR 112022000534 A2 | 10 May 2022 |
| | | MX 2022000711 A | 23 February 2022 |
| | | AR 119424 A1 | 15 December 2021 |
| | | IL 289534 A | 01 March 2022 |
| | | KR 20220035941 A | 22 March 2022 |
| | | JP 2022541483 A | 26 September 2022 |
| | | CL 2022000110 A1 | 20 September 2022 |
| | | JOP 20220008 A1 | 30 January 2023 |
| | | DOP 2022000006 A | 15 March 2022 |
| | | US 2022227768 A1 | 21 July 2022 |
| | | PE 20221339 A1 | 13 September 2022 |
| | | CO 2022001590 A2 | 18 March 2022 |
| | | TW 202116750 A | 01 May 2021 |
| | | WO 2021013735 A1 | 28 January 2021 |
| | | EP 3999506 A1 | 25 May 2022 |
| | | CA 3145644 A1 | 28 January 2021 |
| WO 2021260092 A1 | 30 December 2021 | US 2022009901 A1 | 13 January 2022 |
| | | EP 4172152 A1 | 03 May 2023 |
| | | JP 2023532257 A | 27 July 2023 |
| | | KR 20230026450 A | 24 February 2023 |
| | | CN 115768760 A | 07 March 2023 |
| WO 2022074617 A1 | 14 April 2022 | CA 3195117 A1 | 14 April 2022 |
| | | BR 112023006337 A2 | 09 May 2023 |
| | | AU 2021356762 A1 | 25 May 2023 |
| | | KR 2023084163 A | 12 June 2023 |
| | | CN 116348115 A | 27 June 2023 |
| | | EP 4225314 A1 | 06 August 2023 |
| WO 2022222921 A1 | 27 October 2022 | None | |
| WO 2022228387 A1 | 03 November 2022 | TW 202309027 A | 01 March 2023 |
| WO 2022261777 A1 | 22 December 2022 | None | |
| WO 2023046034 A1 | 30 March 2023 | None | |
| WO 2023046158 A1 | 30 March 2023 | None | |
| WO 2023056039 A1 | 06 April 2023 | US 20230159525 A1 | 25 May 2023 |
| | | US 20230203033 A1 | 25 May 2023 |
| WO 2023088408 A1 | 25 May 2023 | None | |
| WO 2023118085 A1 | 29 June 2023 | None | |
| WO 2023134647 A1 | 20 July 2023 | CN 116425744 A | 14 July 2023 |
| WO 2023138541 A1 | 27 July 2023 | CN 116425744 A | 14 July 2023 |
| WO 2023146957 A1 | 03 August 2023 | None | |
| US 2010222348 A1 | 02 September 2010 | PL 2215075 T3 | 30 April 2014 |
| | | WO 2009053373 A1 | 30 April 2009 |
| | | US 8404713 B2 | 26 March 2013 |

Form PCT/ISA/210 (patent family annex) (July 2022)

**INTERNATIONAL SEARCH REPORT**
**Information on patent family members**

International application No.
**PCT/CN2023/096215**

| Patent document cited in search report | Publication date (day/month/year) | Patent family member(s) | | | Publication date (day/month/year) |
|---|---|---|---|---|---|
| | | ES | 2448870 | T3 | 17 March 2014 |
| | | EP | 2215075 | A1 | 11 August 2010 |
| | | EP | 2215075 | B1 | 11 December 2013 |
| | | JP | 2011500758 | A | 06 January 2011 |
| | | JP | 5525447 | B2 | 18 June 2014 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021013735 A1
- US 20220009901 A1
- WO 2016051193 A1